(19) Europäisches Patentamt
European Patent Office
Office européen des brevets

(11) Numéro de publication : **0 436 435 B1**

(12) **FASCICULE DE BREVET EUROPEEN**

(45) Date de publication du fascicule du brevet : **23.03.94 Bulletin 94/12**

(21) Numéro de dépôt : **90403762.9**

(22) Date de dépôt : **26.12.90**

(51) Int. Cl.$^5$ : **C07C 217/58,** C07C 215/50, C07C 215/30, C07C 235/40, C07C 235/34, C07C 62/30, C07C 62/32, C07C 62/34, C07C 61/22, A61K 31/19, A61K 31/22

(54) **Phényléthanolaminométhyltétralines, procédé pour leur préparation et compositions pharmaceutiques les contenant.**

(30) Priorité : **29.12.89 FR 8917465**
**26.11.90 EP 90403342**

(43) Date de publication de la demande : **10.07.91 Bulletin 91/28**

(45) Mention de la délivrance du brevet : **23.03.94 Bulletin 94/12**

(84) Etats contractants désignés : **AT BE CH DE DK ES FR GB GR IT LI LU NL SE**

(56) Documents cités :
**EP-A- 0 211 721**
**EP-A- 0 213 080**
**EP-A- 0 303 545**
**EP-A- 0 325 963**
**JOURNAL OF MEDICINAL CHEMISTRY, vol. 26, mars 1983, pages 328-334; T.K. SCHAAF et al.: "Structure-activity studies of configurationally rigid arylprostaglandins"**

(73) Titulaire : **ELF SANOFI**
**32-34, rue Marbeuf**
**F-75008 Paris (FR)**
(84) **BE CH DE DK ES FR GB GR LI LU NL SE AT**
Titulaire : **MIDY S.p.A.**
**Via Piranesi 38**
**I-20137 Milano (IT)**
(84) **IT**

(72) Inventeur : **Cecchi, Roberto**
**Via Dei Tigli 1**
**I-20075 Lodi (Milan) (IT)**
Inventeur : **Guzzi, Umberto**
**Via Don Gnocchi 28**
**I-20010 Milano (IT)**

(74) Mandataire : **Gillard, Marie-Louise et al**
**Cabinet Beau de Loménie 158, rue de l'Université**
**F-75340 Paris Cédex 07 (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



Jouve, 18, rue Saint-Denis, 75001 PARIS

## Description

La présente invention concerne des nouvelles phényléthanolaminométhyltétralines, un procédé pour la préparation de ces composés, les intermédiaires dans ce procédé et les compositions pharmaceutiques contenant lesdites phényléthanolaminométhyltétralines en tant que principes actifs.

Le brevet européen 211721 décrit des phényléthanolaminotétralines substituées sur le cycle aromatique de la tétraline, de formule (A) suivante

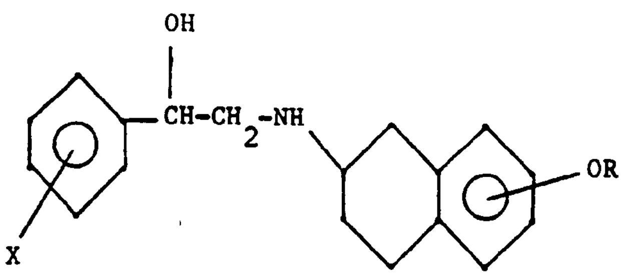

(A)

dans laquelle X représente l'hydrogène, un halogène, un groupe trifluorométhyle, ou un groupe alkyle inférieur et R représente l'hydrogène ou un groupe méthyle non substitué ou substitué par un groupe carboxy ou alcoxycarbonyle, ayant des propriétés pharmacologiques très intéressantes. Entre autres, les composés (A) sont indiqués comme modulateurs de la motricité intestinale et utérine.

On a maintenant trouvé que des composés, qui diffèrent des composés connus essentiellement par la présence d'un groupe méthylène ($-CH_2-$) entre le noyau tétralinique et le groupe amino, ont une activité sur la motricité intestinale qui est supérieure ou au moins égale à celle des phényléthanolaminotétralines correspondantes, associée à une plus grande sélectivité vers l'intestin.

Ainsi, la présente invention concerne, selon un de ses aspects, les phényléthanolaminométhyltétralines ayant la formule (I) suivante

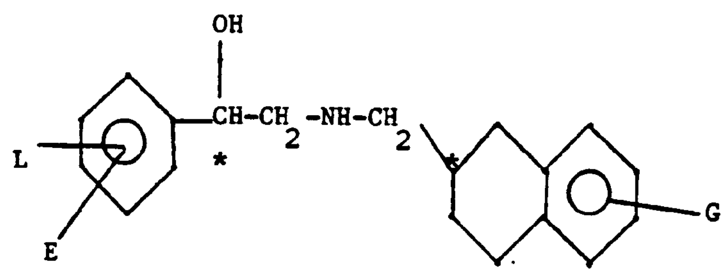

(I)

dans laquelle

- E représente l'hydrogène, un groupe ($C_1$-$C_4$)alkyle, un groupe ($C_1$-$C_4$)alcoxy, un groupe phényle, un groupe nitro, un atome d'halogène, ou un groupe trifluorométhyle,
- L représente l'hydrogène, un groupe ($C_1$-$C_4$)alkyle, un groupe phényle, un groupe ($C_1$-$C_4$) alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe -OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

et leurs sels.

Dans la présente description:

- le terme "($C_1$-$C_4$)alkyle", désigne un radical monovalent d'un hydrocarbure saturé à chaîne droite ou ramifiée, qui peut contenir de 1 à 4 atomes de carbone, tel que méthyle, éthyle, propyle, isopropyle, n-butyle, sec-butyle ou tert-butyle;
- le terme "($C_1$-$C_4$)alcoxy" désigne un radical alcoxy à chaîne droite ou ramifiée contenant de 1 à 4 atomes de carbone, tel que méthoxy, éthoxy, propoxy, isopropoxy, n-butoxy, sec-butoxy ou tert-butoxy;
- le terme "($C_3$-$C_7$)cycloalkyle" désigne un radical monovalent d'un hydrocarbure cyclique saturé renfermant 3 à 7 atomes de carbone, comme les radicaux cyclopropyle, cyclobutyle, cyclopentyle, cyclopentyle substitué par un ou deux groupes méthyle ou par un groupe éthyle, ou les radicaux cyclohexyle, méthylcyclohexyle ou cycloheptyle;

- le terme "($C_2$-$C_4$)alcanoyle" identifie un radical acylique d'un acide carboxylique aliphatique saturé contenant 2 à 4 atomes de carbone, à savoir les radicaux acétyle, propionyle, 2-méthylpropionyle et butyrroyle;
- le terme "halogène" comprend les quatre halogènes: fluor, chlore, brome, iode, les trois premiers étant particulièrement préférés;
- les termes "tétraline", "tétralinique" et "tétralone" se réfèrent au noyau 1, 2, 3, 4-tétrahydronaphtalène.

Les sels des composés de formule (I) selon la présente invention comprennent aussi bien les sels d'addition avec des acides minéraux ou organiques pharmaceutiquement acceptables tels que le chlorhydrate, le bromhydrate, le sulfate, l'hydrogénosulfate, le dihydrogénophosphate, le citrate, le maléate, le tartrate, le fumarate, le gluconate, le méthanesulfonate, le 2-naphtalènesulfonate, etc., que les sels qui permettent une séparation ou une cristallisation convenable des composés de formule (I), tels que le picrate, l'oxalate ou les sels d'addition avec des acides optiquement actifs, par exemple les acides camphorsulfoniques et les acides mandéliques ou mandéliques substitués.

De plus, lorsque le composé de formule (I) possède un groupe carboxy libre, les sels comprennent aussi les sels avec des bases minérales, de préférence celles avec des métaux alcalins tels que le sodium ou le potassium, ou avec des bases organiques, comme le trométamol.

Dans la formule (I) ci-dessus les deux atomes de carbone asymétriques sont marqués par un astérisque. Tous les composés de formule (I) peuvent donc exister sous forme d'au moins quatre isomères stériques différents, (R,R), (R,S), (S,S) et (S,R). Les stéréoisomères optiquement purs, ainsi que les mélanges des deux, trois ou tous les quatre isomères, dans une proportion quelconque, font partie de la présente invention. D'autres centres d'asymétrie pourraient être présents dans les radicaux E, L et G. De même, les stéréoisomères provenant de la présence de ces centres chiraux et leurs mélanges font partie de l'invention.

Pour l'expression de l'activité pharmacologique la configuration préférée de l'atome de carbone chiral de la chaîne éthanolaminique est de toute façon la configuration absolue R. Les composés de formule (I) où E, L, et G sont tels que définis ci-dessus et l'atome de carbone chiral de la chaîne éthanolaminique a la configuration absolue R, réprésentent donc un groupe préféré de composés selon l'invention.

Des composés préférés de la présente invention comprennent les composés de formule (I) dans laquelle E et L sont tels que définis ci-dessus et G représente l'hydrogène, un groupe hydroxy ou un groupe -OG' où G' représente un groupe ($C_1$-$C_4$)alkyle non-substitué ou substitué par hydroxyle, ($C_1$-$C_4$)alcoxy, carboxyle, ($C_1$-$C_4$)alcoxycarbonyle ou ($C_3$-$C_7$)cycloalkyle, et leurs sels.

Des composés de la présente invention particulièrement avantageux comprennent les composés de formule (I) dans laquelle E est l'hydrogène, un groupe ($C_1$-$C_4$)-alkyle, ou un atome d'halogène, L est l'hydrogène et G représente l'hydrogène, un groupe hydroxy ou un groupe -OG' où G' représente un groupe ($C_1$-$C_4$)alkyle non-substitué ou substitué par carboxyle ou ($C_1$-$C_4$)alcoxycarbonyle, et leurs sels.

Les composés de formule (I) peuvent être préparés en traitant un composé de formule (II)

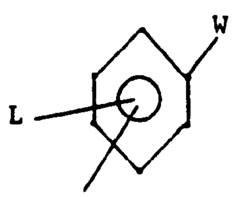

(II)

dans laquelle E et L ont les significations données ci-dessus et le radical -W représente un des groupes suivants

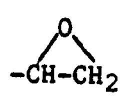 ,

(a)

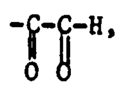

(b)

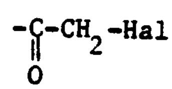

(c)

ou

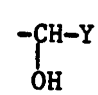

(d)

où Hal représente le chlore, le brome ou l'iode et Y représente un groupe -COOH ou un dérivé fonctionnel de

celui-ci; avec un composé de formule (III)

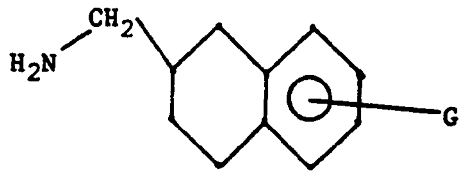

(III)

dans laquelle G a la signification donnée ci-dessus, et, quand -W est autre que

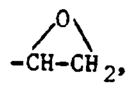

en traitant le produit ainsi obtenu avec un agent réducteur approprié.

Plus particulièrement, la réaction entre les composés de formule (II) et le dérivé 2-aminométhyltétralinique (III) est réalisée selon des modes opératoires et dans des conditions différentes qui sont essentiellement fonction de la nature du produit de départ de formule (II) utilisé et qui dépendent notamment de la signification du groupe -W.

Ces modes opératoires sont traités en détails ci-dessous et ont été désignés Méthodes (a) à (d).

Méthode (a)

Selon ce mode opératoire, l'ouverture de l'époxyde de formule (IIa)

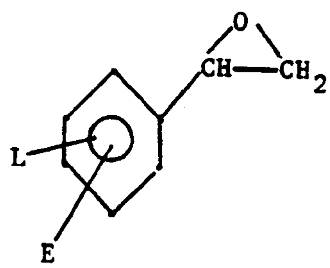

(IIa)

par l'amine (III) est conduite dans un solvant organique tel qu'un alcanol inférieur comme le méthanol, l'éthanol et l'isopropanol, le diméthylsulfoxyde, un éther linéaire ou cyclique, ou un amide comme le diméthylformamide ou le diméthylacétamide, en utilisant des quantités au moins équimoléculaires des réactifs, mais de préférence un excès de l'amine (III). La température de la réaction est comprise entre la température ambiante et la température de reflux du solvant choisi. Un agent basique, tel que la triéthylamine, l'hydroxyde de sodium ou l'acétate de sodium, peut être convenablement utilisé.

Méthode (b)

Dans la réaction qui comprend la condensation du phénylglyoxal de formule (IIb)

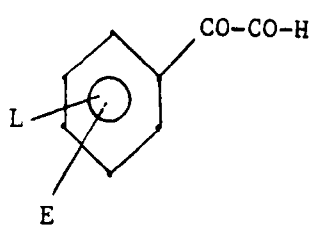

(IIb)

avec l'amine (III) et la réduction du produit obtenu, le mode opératoire préféré prévoit que les deux réactions

soient conduites simultanément en faisant réagir les composés (IIb) et (III) en présence d'un agent réducteur approprié. Si l'amine de formule (III) et le phénylglyoxal de formule (IIb) ne contiennent pas de groupe sensible à la réduction, la réaction est aisement réalisée par hydrogénation catalytique en présence, par exemple, de bioxyde de platine ou de nickel de Raney et dans un solvant alcoolique, tel que le méthanol ou l'éthanol, à la pression atmosphérique ou sous pression. Selon un autre mode opératoire pour cette réaction on utilise un hydrure d'un métal alcalin, par exemple borohydrure de sodium, dans un solvant alcoolique tel que l'éthanol, de préférence à basse température.

Méthode (c)

Selon un autre mode opératoire les composés de formule (I) sont obtenus par réaction entre l'amine de formule (III) et une alphahaloacétophénone de formule (IIc)

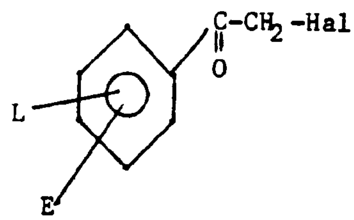

(IIc)

dans un solvant inerte tel qu'un éther lineaire ou cyclique, un alcool inférieur comme le méthanol, l'éthanol ou l'isopropanol, un hydrocarbure aromatique comme le toluène ou le benzène, un hydrocarbure aliphatique halogéné comme le chloroforme ou un nitrile comme l'acétonitrile. Cette réaction de substitution nucléophile est conduite avantageusement à la température ambiante ou à froid. La réduction du produit ainsi obtenu peut être effectuée selon les techniques connues, par exemple par hydrogénation en présence d'un catalyseur tel que le palladium sur charbon, le nickel de Raney, ou le dioxyde de platine, en présence d'un solvant alcoolique, tel que le méthanol ou l'éthanol, de préférence à basse température; ou par action de l'hydrure de lithium et d'aluminium dans l'éther éthylique ou le tétrahydrofuranne ou, encore, par action d'un alcoxyde d'aluminium, tel que l'isopropoxyde d'aluminium, dans un solvant tel que l'isopropanol, de préférence au reflux, ou enfin par action du $NaCNBH_3$ à pH~5.

Méthode (d)

Selon un mode opératoire préféré, qui représente un autre aspect de la présente invention, on fait réagir l'amine (III) avec un composé de formule (IId)

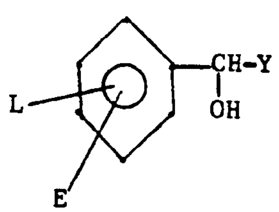

(IId)

dans laquelle E, L et Y sont tels que définis ci-dessus. Comme dérivé fonctionnel du groupe -COOH on peut utiliser le chlorure, l'anhydride, les anhydrides mixtes, les ester actifs ou l'acide libre convenablement activé, par exemple avec le dicyclohexylcarbodiimide (DCCI) ou l'hexafluorophosphate de benzotriazolyl-N-oxytris-(diméthylamino)phosphonium (BOP). La réaction entre le composé de formule (IId) ci-dessus et l'aminométhyltétraline (III) est conduite dans un solvant organique, aprotique, apolaire ou de préférence polaire, tel que le diméthylformamide, le diméthylsulfoxyde, le chlorure de méthylène, le benzène, le toluène, éventuellement en présence d'un accepteur de protons, tel que les amines tertiaires aliphatiques, notamment la triéthylamine. Le mandélamide de formule (IV)

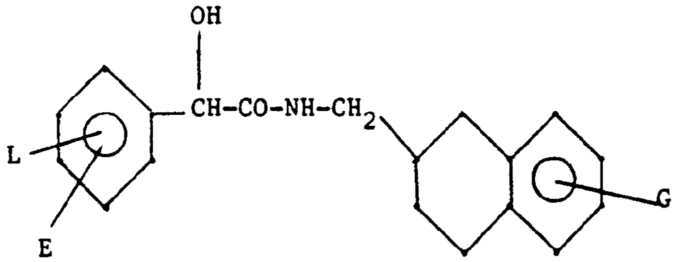

(IV)

ainsi obtenu peut être directement soumis à une réduction du groupe amido en groupe méthylèneamino.

L'étape de réduction est effectuée par exemple, par action d'un hydrure tel que l'hydrure de lithium et d'aluminium, ou du diborane, notamment d'un reactif générant le diborane tel que le complexe entre le borane et le diméthylsulfure, ci-après désigné "borane-méthylsulfure". La réaction est conduite dans un solvant organique tel que le tétrahydrofuranne et le composé de formule (I) est isolé selon les techniques connues. Dans le cas de la réduction d'un mandélamide de formule (IV) dans laquelle G est un groupe -OG' où G' représente un groupe alkyle substitué par un groupe carboxy éventuellement salifié ou un groupe ($C_1$-$C_4$)alcoxycarbonyle, en utilisant le boraneméthylsulfure et en opérant à basse température (10-25°C) on peut obtenir la réduction sélective du groupe amido.

En général, lorsque le produit désiré de formule (I) ci-dessus contient un ou plusieurs groupes sensibles à la réduction, il est convenable d'utiliser comme produit de départ (II) un composé de formule (IIa) ou de choisir des agents réducteurs ou des conditions de réaction particulières connues en littérature qui permettent d'obtenir, au moins de préférence, la réduction sélective de la chaîne entre le groupe amino et le cycle benzénique, avec formation de la chaîne désirée -CH(OH)-$CH_2$-NH- sans altération des autres groupes.

Selon un autre mode opératoire général pour la préparation des composés de formule (I) dans laquelle G est un groupe -OG', on prépare le composé correspondant (I) où G est un groupe hydroxyle, par la méthode générale décrite ci-dessus, et, après, on le transforme dans le produit voulu par O-alkylation ou O-acylation selon des procédures conventionnelles qui prévoient la réaction du composé de formule (I) dans laquelle G est un groupe hydroxy avec un agent d'alkylation ou d'acylation de formule G'-D dans laquelle G' est comme défini ci-dessus et D est un groupe facilement éliminable. Ce mode opératoire est même préférable lorsque G est un groupe OG' où G' représente un groupe ($C_1$-$C_4$)alkyle substitué par un groupe carboxy ou ($C_1$-$C_4$)alcoxycarbonyle; ou un groupe ($C_2$-$C_4$)alcanoyle.

L'O-alkylation par exemple, peut être conduite en utilisant un halogénure de ($C_1$-$C_4$)alkyle éventuellement substitué, tel que le chlorure, le iodure ou, de préférence, le bromure, en présence d'un agent de condensation basique.

L'O-alkylation est conduite dans un solvant organique polaire aprotique tel que l'acétone, les esters comme l'acétate d'éthyle, ou les éthers, de préférence cycliques, tels que le tétrahydrofuranne ou le dioxanne. Comme agent de condensation basique on peut utiliser les carbonates alcalins ou alcalino-terreux, tels que les carbonates de sodium, de potassium ou de calcium ou les amines tertiaires aliphatiques, tel que la triéthylamine.

La réaction d'O-acylation par action des halogénures d'acides ($C_2$-$C_4$)alcanoiques, peut être effectuée dans un milieu réactionnel aqueux ou non aqueux, par exemple dans des cétones aqueuses comme l'acétone aqueuse, dans des esters comme l'acétate d'éthyle, dans des hydrocarbures halogénés comme le chlorure de méthylène, dans des amides comme le diméthylformamide, dans des nitriles comme l'acétonitrile ou dans des mélanges de deux ou plusieurs de tels solvants. La température est comprise entre -50 et + 50°C, de préférence de -20 à +30°C, et de préférence on opère en présence d'un accepteur de protons fixant l'acide halogenhydrique qui se forme de la réaction. Comme accepteurs de protons, on peut citer les amines tertiaires, comme par exemple la triéthylamine, la diméthylaniline ou la 4-diméthylaminopyridine, et des bases inorganiques, comme par exemple le carbonate de sodium ou de potassium ou le carbonate de calcium.

L'acylation peut être également conduite en utilisant les acides carboxyliques eux-mêmes à titre d'agents acylants.

Dans ce cas, on opère avantageusement en présence d'un agent de condensation, comme par exemple un carbodiimide, tel que le DCCI, un composé carbonylé, tel que le carbonyldiimidazole, ou un sel d'isoxazolium tel que le perchlorate de N-éthyle-5-phénylisoxazolium.

On peut également effectuer l'O-acylation avec d'autres dérivés comme, par exemple, un ester activé, un anhydride symétrique ou un anhydride mixte. Les réactions d'acylation, dans lesquelles interviennent les acides libres ou leurs dérivés susmentionnés, sont avantageusement réalisées dans un milieu réactionnel anhydre, par exemple le chlorure de méthylène, le tétrahydrofuranne, le diméthylformamide ou l'acétonitrile.

Dans des cas particuliers, on peut facilement envisager des méthodes alternatives pour l'introduction des

groupes G', ces méthodes faisant partie de la chimie conventionnelle. Les composés (I), dans lesquelles G' est un groupe alkyle substitué par un carboxyle, peuvent être aisement préparés, par exemple, par saponification des esters correspondants.

Les composés de formule (I) dans laquelle G est un groupe OG' où G' est un groupe 1-méthyl-1-($C_1$-$C_4$)alcoxycarbonyléthyle ou 1-éthyl-1-($C_1$-$C_4$)alcoxycarbonyléthyle peuvent être préparés par réaction des composés (I) correspondants, où -G est hydroxy, avec un composé de formule

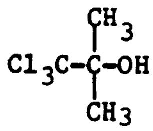 ou 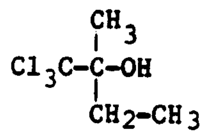

respectivement, en présence d'une base, suivie d'une réaction avec du chlorure de thionyle dans le ($C_1$-$C_4$)alcanol dont on veut l'ester (J. Am. Chem. Soc., 1948, 70, 1153).

Les réactions d'O-alkylation ou d'O-acylation peuvent être conduites directement sur les composés de formule (I) ayant un groupe hydroxy dans le cycle aromatique tétralinique, mais, pour éviter la formation de produits de N-alkylation ou de N-acylation on protège de préférence le groupe amino avec un groupe N-protecteur temporaire R' avant de soumettre les composés (I) aux dits procédés. Les groupes N-protecteurs R' souhaitables sont tous les groupes conventionels qui sont susceptibles d'être éliminés par hydrogénation catalytique ou par hydrolyse acide douce, tels que le groupe benzyloxycarbonyle, le groupe benzyloxycarbonyle substitué sur le noyau aromatique, par exemple par un groupe méthoxy ou nitro, le groupe t-alcoxycarbonyle par exemples le tert-butoxycarbonyle (Boc), ou le tert-amyloxycarbonyle (Aoc); de préférence le groupe Boc est particulièrement préféré.

La N-protection par le groupe R' est effectuée en faisant réagir les composés de formule (I) dans laquelle G est l'hydroxyle avec le réactif approprié pour la protection des groupes amino comme décrit, par exemple, par M. Bodanszky et al., Peptide Synthesis, 2nd Edition, John Wiley & amp;Sons, 1976, pages 18 à 49, chapitres 3 à 6.

Les groupes Boc et Aoc, par exemple, peuvent être introduits par réaction avec le di-tert-butyl- et, respectivement, le di-tert-amyldicarbonate en milieu basique et dans un solvant organique tel que le dioxanne, le tétrahydrofuranne ou le diméthylformamide. Les groupes benzyloxycarbonyle et benzyloxycarbonyle substitué peuvent être introduits selon la procédure générale décrite par E.C. Horning, Organic Synthesis, vol. III, Wiley, New York, 1955, page 167.

Les composés ansi obtenus de formule (I')

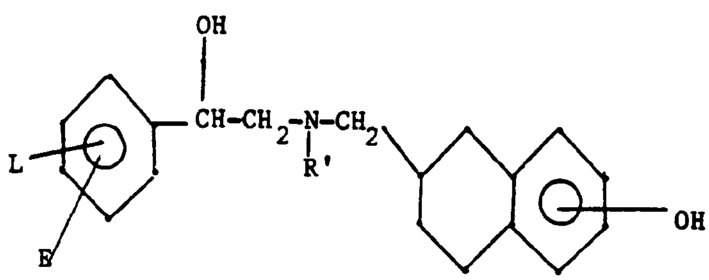

(I')

dans laquelle E, L et R' sont tels que définis ci-dessus, sont soumis à l'O-alkylation ou à l'O-acylation selon les procédés conventionels décrits ci-dessus et, ensuite, le groupe N-protecteur R' du composé ainsi obtenu de formule (I")

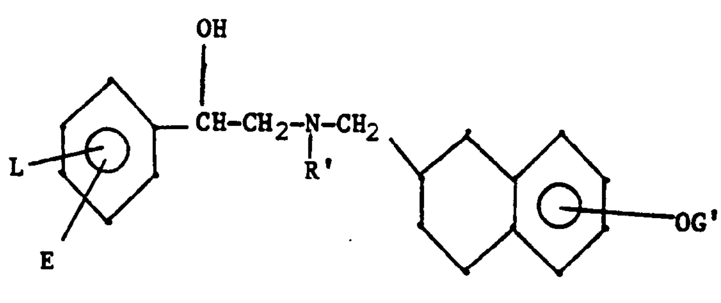

(I")

où E, L, R' et G' sont tels que définis ci-dessus, est éliminé.

L'élimination des groupes N-protecteurs est effectuée par hydrogénation catalytique ou par hydrolyse acide douce selon des méthodes bien connues en littérature. Notamment, les groupe Boc et Aoc sont éliminés en conditions acides, par action de l'acide trifluoroacétique. Les groupes benzyloxycarbonyle et benzyloxycarbonyle substitué sont éliminés par hydrogénation catalytique, de préférence en utilisant le palladium sur charbon comme catalyseur.

Lorsqu'on obtient un composé de formule (I") dans laquelle -G' représente un groupe alkyle substitué par ($C_1$-$C_4$)alcoxycarbonyle, il peut être soumis à la saponification en milieu basique avant ou après la déprotection du groupe amino.

Les produits (I) sont isolés selon les méthodes conventionnelles, de préférence sous forme d'un de leurs sels d'addition avec les acides minéraux ou organiques qui permettent une séparation ou une cristallisation convenable des composés comme indiqué plus haut, tels que l'acide picrique, l'acide oxalique ou un acide optiquement actif, par exemple un acide mandélique ou mandélique substitué, ou un acide camphorsulphonique, ou avec les acides mineraux ou organiques qui forment des sels pharmaceutiquement acceptables tels que l'acide chlorhydrique, l'acide bromhydrique, l'acide sulfurique, l'acide phosphorique, l'acide méthanesulfonique, l'acide méthylsulfurique, l'acide maleique, fumarique, naphtalènesulfonique.

La base libre peut être libérée par neutralisation et transformée dans un autre sel d'addition acide ou, lorsque G est un groupe OG' où G' est un groupe alkyle substitué par un carboxyle, dans un sel avec un métal, notamment alcalin, ou alcalino-térreux tel que le sel de sodium ou de calcium.

Les composés de formule (I) qui présentent les deux seuls atomes de carbone asymétrique marqués par l'astérisque peuvent exister sous quatre formes isomériques différentes. Le procédé de la présente invention permet d'opérer tant sur des mélanges racémiques que sur des isomères optiquement purs. En particulier, les réactions qui forment l'ensemble de ce procédé, ne modifient pas la stéréochimie des composés concernés. Ainsi, en partant d'un composé de formule (IIb) ou (IIc), qui n'a pas de centre chiral, ou d'un composé de formule (IIa) ou (IId) sous forme racémique, et d'un composé de formule (III) sous forme racémique, on obtient un mélange d'isomères, notamment les isomères (R,R), (S,S), (R,S) et (S,R).

De même, en utilisant un composé de formule (III) sous forme optiquement pure, par exemple ayant la configuration absolue R, on obtient un mélange de deux seuls isomères, à savoir le (R,R) et le (S,R). Dans ce dernier cas, en utilisant aussi un composé de départ de formule (IIa) ou (IId) sous forme optiquement pure, on peut obtenir les isomères purs.

Lorsqu'un mélange de quatre isomères est obtenu, il peut être séparé en deux couples d'enantiomères (R,R)+(S,S) et (R,S)+(S,R), entre eux diastéréoisomériques, par des technique convenables comme la cristallisation fractionnée dans un solvant approprié, de préférence un alcanol inférieur, tel que l'éthanol, l'isopropanol ou leur mélanges. Chaque couple d'énantiomères peut être ensuite séparé en isomères purs par exemple par formation de sels diastéréoisomériques, ou par chromatographie sur des colonnes chirales, ou par d'autres techniques convenables.

Lorsqu'un des composés de départ est sous forme optiquement active, le mélange des deux diastéréoisomères ainsi obtenu est séparé en isomères purs selon les techniques citées précédemment.

Les composés de départ de formule (II) sont des produits connus ou de toute façon ils peuvent être préparés selon des méthodes conventionnelles décrites dans la littérature chimique. Par exemple, les composés de formule (IIa) peuvent être obtenus par époxydation des dérivés styréniques correspondants par l'oxygène en présence d'un catalyseur à l'argent, ou par action du méthylide de diméthylsulfonium ou diméthylsulfoxonium sur le benzaldéhyde substitué correspondant selon la méthode décrite par E. J. Corey dans J. Am. Chem. Soc., 1956, 87, 1353.

Selon une méthode de préparation préférée, on peut obtenir un composé de formule (IIa) sous forme optiquement active par réduction de l'acide mandélique substitué ayant la configuration absolue voulue à l'atome de carbone en position alpha, dans le dérivé glycolique correspondant, par estérification du groupe alcoolique primaire avec un dérivé fonctionnel d'un acide sulfonique tel que le chlorure de tosyle ou de mésyle et, après, par cyclisation du composé ainsi obtenu par traitement avec une base forte tel qu'un hydroxyde alcalin dans les conditions de réaction conventionellement utilisées pour les substitutions nucléophiles intramoléculaires.

Les composés de formule (IIb) sont aisement préparés par action d'un agent oxydant, tel que le dioxyde de selenium, sur les acétophénones correspondantes, dans l'eau ou dans un solvant organique tel qu'un éther cyclique, notamment le dioxanne ou le tétrahydrofuranne.

Selon un autre mode opératoire, lesdits composés de formule (IIb) sont préparés par action du diméthylsulfoxyde sur les haloacétophénones de formule (IIc) ayant la même substitution sur le noyau benzénique selon la méthode décrite par N. Kornblum dans J. Am. Chem. Soc., 1957, 79, 6562, ou encore, à partir des alphadihaloacétophénones correspondantes par la réaction décrite par F. Venier dans le C.R. Acad. Sci., 1968, 266, 1650.

Les composés de départ de formule (IIc) sont aisement obtenus par halogénation des cétones correspondantes ou, dans certains cas, par une réaction de Friedel-Crafts en utilisant les dérivés benzéniques substitués correspondants et un halogénure d'un acide haloacétique. Enfin, les dérivés fonctionnels des acides mandéliques ou mandéliques substitués de formule (IId) sont préparés à partir des acides correspondants qui, à leur tour, peuvent être obtenus par hydrolyse de mandélonitriles. Ces derniers composés peuvent être préparés à partir soit du benzaldehyde, substitué ou non, et de l'acide cyanhydrique, soit du benzaldehyde, substitué ou non, du cyanure de sodium et du bisulfite de sodium selon des méthodes bien connues dans la littérature chimique. Les acide mandéliques de formule (IId) obtenus sous forme racémique peuvent être aisement séparés en leurs isomères optiquement purs par formation de sels diastéréoisomériques avec des bases organiques optiquement actives appropriées selon les méthodes et les techniques conventionnelles.

Les composés de formule (III) dans laquelle G représente un atome de chlore, un groupe hydroxy ou un groupe OG', où G' a la signification donnée ci-dessus, sauf les composés de formule (III) dans laquelle G est un groupe méthoxy en position 7 ou 8, ainsi que les stéréoisomères optiquement purs des composés de formule (III) dans laquelle G représente l'hydrogène, un atome de chlore, un groupe hydroxy ou un groupe OG' où G' a la signification donnée ci-dessus, et leurs sels éventuels, sont des produits nouveaux et représentent les intermédiaires clé dans la préparation des composés (I). Lesdits produits de formule (III) représentent, par conséquent, un autre objet de la présente invention.

Des composés de formule (III) préférés comprennent les composés de formule (III) dans laquelle G représente un groupe hydroxy ou un groupe OG' où G' représente un groupe ($C_1$-$C_4$)alkyle substitué par un carboxyle ou un ($C_1$-$C_4$)alcoxycarbonyle.

Les composés de formule (III) peuvent être préparés en partant d'une 1-tétralone de formule (V)

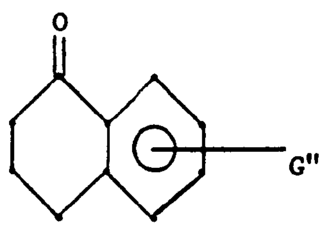

(V)

dans laquelle G" représente l'hydrogène, un atome de chlore, un groupe hydroxy ou un groupe méthoxy, selon une méthode générale qui est illustrée dans le Schéma I suivant

Schéma I

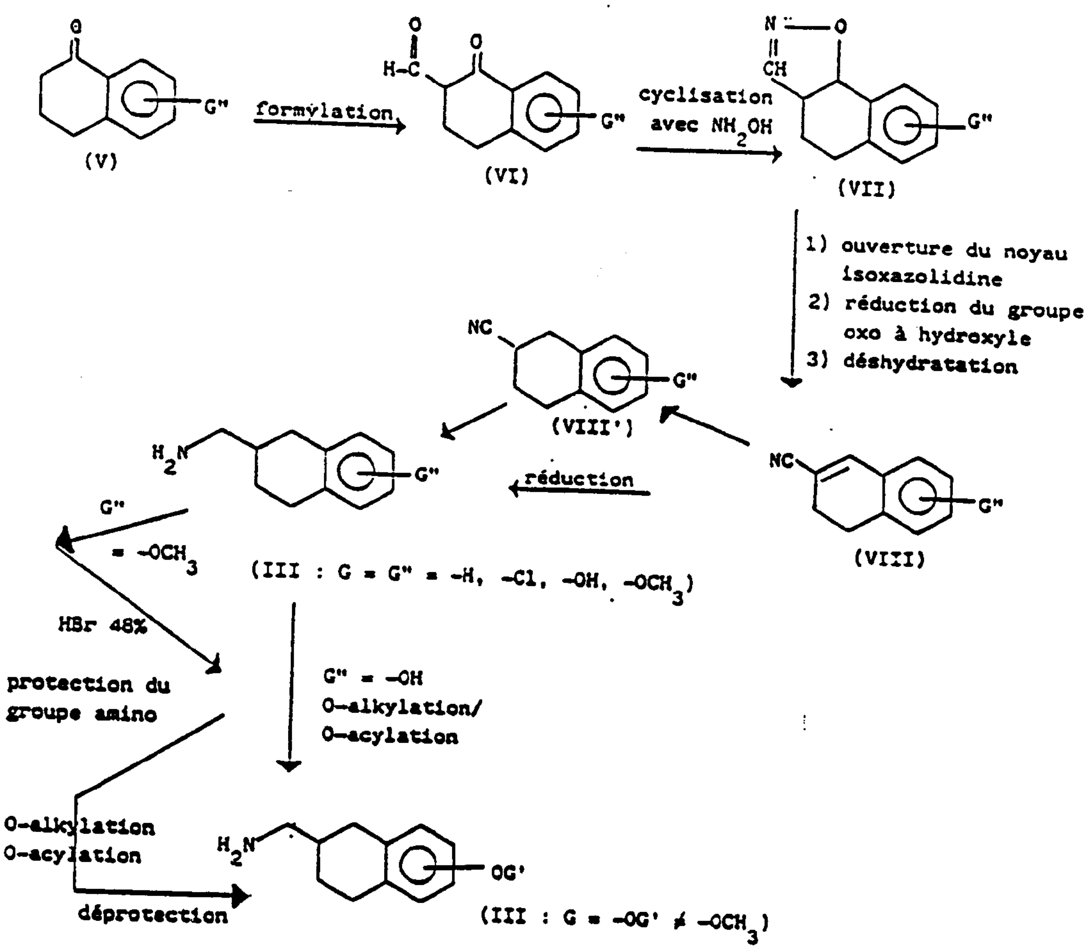


La méthode générale illustrée dans le Schéma I prévoit:

(i) une réaction de Claisen pour l'introduction d'un groupe formyle en position 2 de la 1-tétralone (V), par réaction avec un formiate d'alkyle en présence de sodium,

(ii) la réaction du composé (VI) ainsi obtenu avec de l'hydroxylamine en chauffant dans un milieu acide,

(iii) l'ouverture du cycle isoxazolidinique du composé (VII) et la réduction du groupe 1-oxo en 1-hydroxy (par exemple, selon la méthode décrite dans Synthesis, 1981, 449),

(iv) la déshydratation du composé intermédiaire ainsi obtenu, avec un système déshydratant tel que par exemple le $POCl_3$/pyridine, en obtenant le composé (VIII), et

(v) le traitement du composé (VIII) avec un agent réducteur approprié donnant lieu au composé correspondant de formule III où G = G" et représente l'hydrogène, un atome de chlore, un groupe hydroxyle ou un groupe méthoxyle.

La réduction du composé de formule (VIII) peut être effectuée soit en deux étapes, par exemple d'abord avec du borohydrure de sodium et après avec de l'hydrure de lithium et d'aluminium ou de l'hydrure d'isobutylaluminium (DIBAL), soit en une seule étape, par exemple directement avec l'hydrure de lithium et d'aluminium ou avec le DIBAL. Dans le premier cas on peut isoler la 2-cyanotétraline éventuellement substituée par le groupe G".

Les composés de formule (III) dans laquelle G est un groupe OG' autre que méthoxy sont donc préparés par O-alkylation ou O-acylation du composé (III) où G = G" = OH selon les méthodes conventionnelles décrites

plus haut pour l'O-alkylation ou l'O-acylation des composés de formule (I), dans laquelle G est hydroxy. Dans ce cas aussi, l'O-acylation ou l'O-alkylation éventuelle du composé de formule (III) où G = G" = OH peuvent être conduites de préférence avec protection préalable du groupe amino. Pour cette protection préalable, on peut utiliser non seulement les groupes N-protecteurs R' indiqués plus haut pour la protection du groupement -NH- des composés (I), mais également les groupes benzyle, benzhydryle ou trityle non substitués ou substitués sur le cycle benzénique ou un des cycles benzéniques par un groupe méthoxy ou nitro et le groupe 2, 2, 2-trichloroéthyle ou de former des dérivés phtalimido. La déprotection de ces groupes N-protecteurs est effectuée selon les techniques conventionnelles, notamment par hydrogénation catalytique avec palladium ou hydroxyde de palladium sur charbon, dans le cas des groupes benzyle, benzhydryle et trityle éventuellement substitués ou du groupe 2, 2, 2-trichloro- éthyle et par traitement avec l'hydrazine dans le cas du groupe phtalimido. Les groupes trityle et méthoxytrytyle peuvent être aussi éliminés par hydrolyse douce, par exemple dans l'acide formique à 50%.

Les composés de formule (III), dans laquelle G est un groupe OH, peuvent également être préparés en partant d'un composé de formule (V) où G" est un groupe méthoxy dans la même position et en soumettant les composés (III) obtenus selon la méthode générale du Schéma I à une réaction de déméthylation avec de l'acide bromhydrique.

De plus, les composés de formule(III), dans laquelle G est un groupe OG' où G' est éthyle substitué par un groupe carboxy ou ($C_1$-$C_4$)alcoxycarbonyle, peuvent être préparés à partir des composés correspondants de formule (III) dans laquelle G est un groupe OG', où G' est méthyle substitué par un groupe carboxy, par protection du groupe amino avec un groupe Boc ou Aoc suivie par la réaction de Arndt-Eistert (Ber., 1935, 68, 200) qui prévoit la conversion de l'acide dans le chlorure d'acide correspondant suivie par la réaction de ce dernier produit avec le diazométhane et par l'hydrolyse du produit ainsi obtenu en présence de $Ag_2O$.

Les dérivés 2-cyano-3,4-dihydronaphtalènes de formule (VIII) peuvent aussi être préparés à partir des 2-tétralones correspondantes de formule (IX)

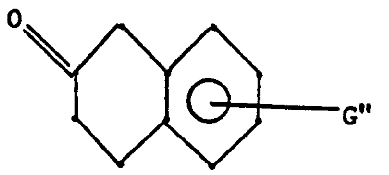

(IX)

par réaction avec une quantité au moins équimoléculaire d'un cyanure alcalin, notamment de cyanure de sodium, dans un solvant organique aprotique, de préférence polaire, par exemple le diméthylsulfoxyde ou le diméthylformamide. Cette réaction, qui peut être conduite à une température comprise entre la température ambiante et la température de reflux du mélange réactionnel, conduit directement au composé (VIII) qui est ensuite traité comme décrit dans le Schéma I.

Si on le desire, les mélanges racémiques des composés (III) ainsi obtenus sont séparés en isomères purs par formation des sels diastéréoisomériques avec des acides organiques optiquement actifs tels que les acides camphorsulfoniques, les acides mandéliques éventuellement substitués ou d'autres acides optiquement actifs.

Si l'aminométhyltétraline (III) contient un deuxième centre chiral, les diastéréoisomères et les quatre isomères purs peuvent être isolés comme décrit ci-dessus. Ainsi, ils peuvent être utilisés pour la préparation de tous les isomères des composés (I),

Selon un autre mode opératoire convenable pour préparer les composés (III), on utilise, comme composés de départ, les acides carboxyliques correspondants de formule (X) dans laquelle Z est un groupe OH

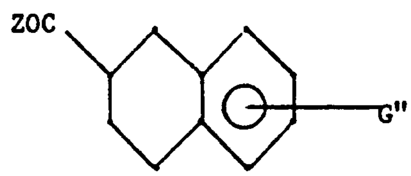

Ces produits sont transformés en amides correspondants ((X): Z = $NH_2$) dont le groupe amido est ensuite transformé en groupe aminométhyle.

Les acides ci-dessus de formule (X) où Z est -OH, peuvent être préparés à partir des 1-tétralones correspondantes (V) selon une méthode générale qui est illustrée dans le Schéma II suivant

Schéma II

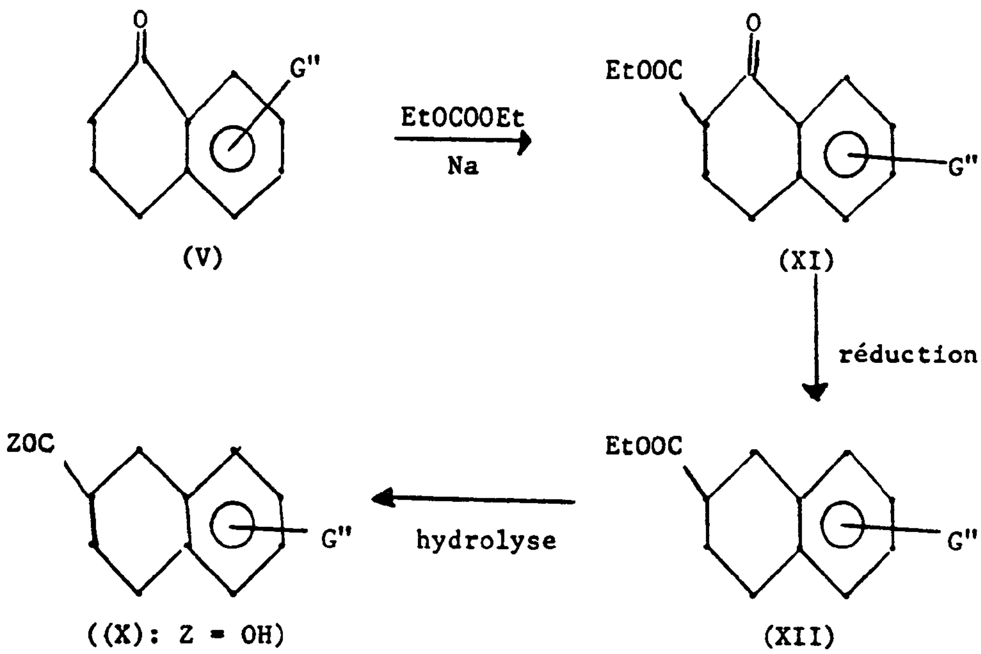


(V) (XI) (XII) ((X): Z = OH)

et qui prévoit:

(i) une réaction de Claisen pour l'introduction d'un groupe éthoxycarbonyle en position 2 de la 1-tétralone (V), par réaction avec le diéthylcarbonate en présence de sodium,

(ii) la réduction du groupe 1-oxo dans le composé (XI) par voie catalytique, avec $H_2$ en présence de Pd/C, ou chimique, avec le système triéthylsilane/acide trifluoroacétique (Tétrahedron, 1967, 23, 2235) ou triéthylsilane/$BF_3.Et_2O$ (J. Org. Chem., 1985, 50, 3619) ou, encore, avec le triéthylsilane/acide trifluorométhanesulfonique (Synthesis, 1986, 779), et

(iii) l'hydrolyse de l'ester (XII) en milieu basique.

La conversion des acides en les amides correspondants de formule (X), dans laquelle Z est un groupe $NH_2$, est effectuée selon les méthodes convertionnelles qui prevoient l'addition nucléophile de l'ammoniac sur le carbone polarisé positivement d'un dérivé fonctionnel de l'acide.

Comme dérivé fonctionnel on peut utiliser le chlorure, l'anhydride, les anhydrides mixtes, les esters actifs ou l'acide libre opportunement activé, par exemple, avec le DCCI ou le BOP.

L'étape de réduction du groupe amido est effectuée par exemple par action d'un hydrure tel que l'hydrure de lithium et d'aluminium ou du diborane, notamment du réactif borane-méthylsulfure. La réaction de réduction est conduite dans un solvant organique, aprotique, tel que les ethers cycliques ou linéaires, notamment dans le dioxanne ou le tétrahydrofuranne.

Les composés ainsi obtenus de formule (III) dans laquelle G = G" et représente l'hydrogène, un atome de chloro, un groupe hydroxy ou un groupe méthoxy, peuvent être convertis dans les autres composés de formule (III) comme décrit plus haut.

En partant de l'acide de formule (X) où Z = OH, sous forme optiquement active, on obtient le composé de formule (III) ayant la même configuration absolue à l'atome de carbone asymétrique.

Les acides optiquement actifs de formule (X) peuvent être obtenus à partir du mélange racémique correspondant par formation de sels diastéréoisomériques avec des amines optiquement actives telles que la d-alpha-méthylbenzylamine, la 1-alpha-méthylbenzylamine, la d-menthylamine et la 1-menthylamine et précipitation desdits sels dans un solvant approprié.

Les acides et les amides de formule (X), sous forme optiquement pure, permettent une préparation aisée des produits (I) optiquement purs. Les composés de formule (X) dans laquelle Z et G" sont tels que définis plus haut, sous réserve que lorsque Z est -OH, G" soit différent de l'hydrogène, sont des produits nouveaux qui représentent un autre objet de la présente invention.

Les composés de formule (I) et leurs sels possèdent des propriétés pharmacologiques intéressantes car ils se sont montrés très actifs comme modulateurs de la motricité intestinale.

En particulier, leur aptitude à réduire la motricité spontanée du côlon a été observée dans des essais pharmacologiques normalisés in vitro et a été confirmée chez l'animal in vivo.

Dans les essais in vitro on a évalué la capacité des phényléthanolaminométhyltétralines de l'invention à réduire, selon la concentration, la réponse contractile, dans des conditions normalisées particulières, des bandes de côlon proximal de rat isolés.

On sacrifie des rats mâles, non à jeun, pesant de 250 à 300 g. La partie proximale du côlon, consistant en un segment de 2 à 3 cm environ, est prélevée et suspendue dans une cuve à organes de 20 ml contenant une solution de Krebs-Ringer ayant la composition nM suivante: NaCl 118,4; KCl 4,7; $CaCl_2$ 2,45; $MgSO_4$ 1,16; $NaH_2$ $PO_4$ 3,7; Glucose 5,6; $NaHCO_3$ 30,9. La solution est aérée par un mélange d'oxygène (95%) et de $CO_2$ (5%) et sa température maintenue constante à 37°C. Les segments de côlon, soumis à une traction de 1 g environ, se contractent spontanément. Les composés à l'étude sont ajoutés après stabilisation de la préparation (2h).

On détermine la $CE_{50}$ qui représente la concentration du composé qui produit une réduction du 50% de la contraction du tissu atteinte dans l'absence de composés à essayer.

Dans ce test les composés de la présente invention ont montré une activité très élevée, caractérisée, pour les produits les plus actifs, par des $CE_{50}$ de l'ordre de 1 à 50 nM.

Les composés de formule I ont montré aussi une spécificité surprenante pour le côlon. Des essais in vitro, effectués selon la même méthode générale mais sur l'utérus isolé de rat, ont montré qu'on obtient un effect significatif sur la contractilité spontanée de l'utérus à des doses beaucoup plus élevées que les doses actives sur le côlon.

Par rapport aux composés décrits dans le brevet européen 211 721, les composés de formule I selon la présente invention se sont révélés plus puissants et plus sélectifs.

Par exemple, le composé de l'Exemple 4 (chlorhydrate de N-[(7-méthoxy-1,2,3,4-tétrahydronapht-2-yl)méthyl]-2-hydroxy-2-(3-chlorophényl) éthanamine) est caractérisé par une $CE_{50}$ sur le côlon de 43 nM et une $CE_{50}$ sur l'utérus de 2453 nM qui correspondent à un rapport de selectivité d'environ 57, alors que le composé décrit dans l'Exemple 7 du brevet européen 211 721 (chlorhydrate de N-(7-éthoxy-1,2,3,4-tétrahydronapht-2-yl)-2-hydroxy-2-(3-chlorophényl)éthanamine) est caractérisé par une $CE_{50}$ sur le côlon de 194 nM et une $CE_{50}$ sur l'utérus de 350 nM (rapport de sélectivité inférieur à 2).

Egalement, le composé de l'exemple 10, sous forme de chlorhydrate, est caractérisé par une $CE_{50}$ sur le côlon de 7 nM et une $CE_{50}$ sur l'utérus de 50 (rapport de selectivité environ 7), tandis que le composé décrit dans l'Exemple 8 du brevet européen 211 721, qui diffère du premier pour le groupe $-CH_2-$ entre le noyau tétralinique et le groupe -NH-, est caractérisé par une $CE_{50}$ de 110 nM tant sur le côlon que sur l'utérus (rapport de sélectivité = 1).

Dans les essais in vivo, on a évalué la motricité intestinale chez le rat anesthésié selon la technique décrite dans EP 255 415. Les composés de la présente invention, ont montré une très bonne activité à des doses très faibles.

Les phényléthanolaminométhyltétralines de formule (I) et leurs sels pharmaceutiquement acceptables possèdent une toxicité très faible, compatible avec l'utilisation de ces produits comme médicaments.

Ainsi, la présente invention, selon un autre de ses aspects, concerne des compositions pharmaceutiques utiles notamment pour le traitement des troubles intestinaux renfermant, en tant que principe actif, un ou plusieurs composés de formule (I) ci-dessus ainsi que leurs sels pharmaceutiquement acceptables.

Dans les compositions pharmaceutiques de la présente invention pour l'administration orale, sublinguale, sous-cutanée, intramusculaire, intraveineuse, transdermique ou rectale, les principes actifs ci-dessus peuvent être administrés sous formes unitaires d'administration, en mélange avec des supports pharmaceutiques classiques, aux mammifères pour le traitement des troubles de la motricité intestinale. Des formes unitaires d'administration appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire ou intraveineuse et les formes d'administration rectale.

Afin d'obtenir l'effet thérapeutique désiré, la dose de principe actif peut varier entre 0,01 et 100 mg par kg de poids du corps et par jour.

Chaque dose unitaire peut contenir de 0,1 à 500 mg de principe actif en combinaison avec un support pharmaceutique. Cette dose unitaire peut être administrée 1 à 4 fois par jour.

Lorsqu'on prépare une composition solide sous forme de comprimés, on mélange le principe actif principal avec un véhicule pharmaceutique tel que la gélatine, l'amidon, le lactose, le stéarate de magnésium, le talc, la gomme arabique ou analogues. On peut enrober les comprimés de saccharose ou d'autres matières appropriées ou encore on peut les traiter de telle sorte qu'ils aient une activité prolongée ou retardée et qu'ils libèrent d'une façon continue une quantité prédéterminée de principe actif.

On obtient une préparation en gélules en mélangeant le principe actif avec un diluant et en versant le mélange obtenu dans des gélules molles ou dures.

Une préparation sous forme de sirop ou d'élixir ou pour l'administration sous forme de gouttes peut contenir le principe actif conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir l'ingrédient actif en mélange avec des agents de dispersion ou des agents mouillants, ou des agents de mise en suspension, comme la polyvinylpyrrolidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs.

Le principe actif principal de formule (I) peut être administré sous forme de base libre ou de sel pharmaceutiquement acceptable tel quel ou sous forme de complexe avec par exemple une cyclodextrine ou bien en association ou en co-administration avec d'autres principes actifs, par exemple avec des tranquillisants.

Les composés de formule (I) et leurs sels sont aussi actifs dans le contrôle de la pression intraoculaire elevée, i.e. dans la régulation, la diminution et la modulation de la tension intraoculaire élevée. Ils peuvent donc être utilisés pour le traitement de l'hypertension oculaire et du glaucome, une affection oculaire qui conduit à la destruction des fibres du nerf optique et peut entraîner la perte de la vision, qui est caractérisée entre autres symptômes par une augmentation de la pression intraoculaire.

L'effet d'abbaissement de la pression intraoculaire des composés de formule (I) ainsi que de leurs sels peut être déterminée chez l'animal, par exemple chez le lapin, dans un test qui prévoit l'administration orale de quantités importantes d'eau, tel que décrit par exemple dans Arch. Ophthal., 1969, 82, 381-384 ou dans J. Ocul. Pharmacol., 1985, 1(2), 161-168, ou l'injection rapide par voie intraveineuse d'une solution de glucose, tel que décrit par exemple dans Boll. Ocul., 1979, 58(7-8), 359-366.

Selon un autre de ses aspects, la présente invention concerne égalément une composition pharmaceutique ophtalmique pour une administration topique sur l'oeil, comprenant une phényléthanolaminométhyltétraline de formule (I) ou un de ses sels pharmaceutiquement acceptables.

Les formulations ophtalmiques selon la présente invention, sous forme de solutions, suspensions ou pommades, peuvent contenir de 0,00001 à 1% en poids d'un composé de formule (I) ou d'un de ses sels pharmaceutiquement acceptables, plus particulièrement de 0,0001 à 0,2%.

Chaque unité de dosage (goutte) comprend une quantité de phényléthanolaminométhyltétraline comprise entre 10 ng et 1 mg, préférentiellement entre 100 ng et 0,2 mg.

L'administration de ces formulations peut être effectuée par application dans l'oeil de 1-2 gouttes 1 à 3 fois par jour de façon à assurer une posologie journalière de 10 ng à 1 mg, de préférence de 100 ng à 0,2 mg de principe actif.

Pour préparer des formulations convenables, les phényléthanolaminométhyltétralines de l'invention peuvent être mélangées avec un véhicule adapté pour une administration ophtalmique topique.

Comme véhicules pharmaceutiques acceptables pour une administration ophtalmique, on peut citer l'eau, un mélange d'eau et de solvants miscibles à l'eau comme les alcanols inférieurs, les huiles végétales, les huiles minérales et comprenant de 0,5 à 5 % en poids d'hydroxyéthylcellulose, d'oléate d'éthyle, de carboxyméthylcellulose, de polyvinylpyrrolidone et d'autres polymères solubles dans l'eau, non toxiques et compatibles avec une utilisation ophtalmique, par exemple des dérivés de la cellulose tels que le méthylcellulose, un dérivé d'un métal alcalin de carboxyméthylcellulose, l'hydroxyméthylcellulose, l'hydroxyéthylcellulose, l'hydroxypropylméthylcellulose, des acrylates tels que des sels d'acides polyacryliques, des éthylacrylates, des polyacrylamides, des produits naturels tels que la gélatine, les alginates, les pectines, le tragacanth, le karaya, le chondrus, l'agar, l'acacia, des dérivés d'amidon comme l'acétate d'amidon, les éthers d'hydroxyéthylamidon, l'hydroxypropylamidon, ainsi que d'autres dérivés synthétiques comme le polyvinylalcool, la polyvinylpyrrolidone, le polyvinylméthyléther, le polyéthylèneoxyde, le carbopol neutre ou le xanthane ou des mélanges de ces polymères. La préparation pharmaceutique peut aussi contenir des substances auxiliaires non toxiques comme des émulsifiants, des conservateurs, des agents de mouillage, des agents texturant et d'autres comme par exemple les polyéthylènes glycols 200, 300, 400, 600, les carbowax 1000, 1500, 4000, 6000, 10000, des produits antibactériens, comme des ammonium quaternaires, des sels phénylmercuriques connus pour avoir des propriétés stérilisantes à froid sans être agressifs, le timérosal, le propylparaben, l'alcool benzylique, le phényl éthanol, des agents tampons comme un chlorure de métal alcalin, des tampons borate, acétate ou gluconate,

des antioxydants comme le métabisulfite de sodium, l'hydroxyanisol butylé, l'hydroxytoluène butylé ou des agents semblables et d'autres agents classiquement utilisés comme le monolaurate de sorbitan, l'oléate de triéthanolamine, le monopalmitate de polyéthylène sorbitan, un sel de métal alcalin du dioctyl sulfosuccinate, le monothioglycérol, l'acide éthylènediamine tétracétique ou autres.

De plus, des excipients ophtalmiques acceptables peuvent être utilisés comme le tampon phosphate, l'acide borique isotonique, un chlorure alcalin isotonique, ou la trométhamine par exemple.

La préparation pharmaceutique peut également être une suspension dans laquelle les particules solubles sont des polymères solubles dans l'eau ou insolubles. Une telle suspension peut contenir des microformes telles que des microparticules ou des nanoparticules.

Les compositions selon l'invention peuvent contenir des agents thérapeutiques additionnels. Ainsi des antibiotiques, des anesthésiques, des antiinflammatoires stéroidiens ou des corticostéroides pour le traitement du glaucome, dont un effet secondaire est l'augmentation de la pression intraoculaire, ou d'autres agents abaissant la pression intraoculaire peuvent être présents.

Les exemples suivants illustrent l'invention sans toutefois la limiter. Les solvants indiqués entre parenthèse après le point de fusion sont les solvants de cristallisation. Le symbole du pouvoir rotatoire est indiqué par convention [alpha], mais on doit l'entendre comme $[alpha]_D^{20}$.

Préparation des composés de départ de formule (III)

(A) chlorhydrate de 2-aminométhyl-5-méthoxy-1,2,3,4-tétrahydronaphtalène.

On prépare le produit 2-cyano-5-méthoxy-1,2,3,4-tétrahydro-1-naphtol selon la méthode décrite en littérature pour le 2-cyano-6-méthoxy-1,2,3,4-tétrahydro-1-naphtol (Synthesis, 1981, pp. 449-451), méthode qui est schématisée dans les étapes (i) à (iv) suivantes, et on transforme ce produit intermédiaire en chlorhydrate de 2-aminométhyl-5-méthoxy-1,2,3,4-tétrahydronaphtalène comme décrit en détail dans les étapes (v) et (vi).

(i) 2-formyl-5-méthoxy-3,4-dihydronaphtalen-1(2H)-one. On ajoute une solution de formiate d'éthyle (20 ml, 0,37 moles) dans du benzène anhydre (100 ml) à de l'éthoxyde de sodium préparé à partir de sodium (8,34 g, 0,35 moles) et de l'éthanol absolu, dans du benzène anhydre (100 ml). On refroidit le mélange réactionnel à environ 0°C et on y ajoute lentement et sous agitation du 5-méthoxy-3,4-dihydronaphtalen-1(2H)-one (25 g, 0,14 moles) dans du benzène anhydre (100 ml). En traitant le mélange réactionnel selon la méthode décrite dans J. Am. Chem. Soc. 1947, 69, 2942, on recueille le produit indiqué dans le titre (24,8 g); p.f. 68-70°C.

(ii) 6-méthoxy-4,5-dihydronapht[2,1-d]isoxazole. On chauffe au reflux un mélange de produit obtenu dans l'étape (i) (23,8 g, 0,11 moles) et de chlorhydrate d'hydroxylamine (8,2 g, 0,12 moles) dans du méthanol (300 ml) pendant dix minutes et on évapore ensuite sous vide. On ajoute de l'eau et on extrait par de l'éther éthylique en obtenant 19 g de produit; p.f. 84-86°C.

(iii) 2-cyano-5-méthoxy-3,4-dihydronaphtalen-1(2H)-one. On traite le produit obtenu dans l'étape précédente (19 g, 0,094 moles), à environ 0°C, avec du méthoxyde de sodium préparé à partir de sodium (4,7 g, 0,188 moles) et de méthanol anhydre (250 ml) pendant une heure. On évapore sous vide, on ajoute de l'eau et on extrait par de l'acétate d'éthyle. On obtient le produit indiqué dans le titre (16,7 g); p.f. 120-122°C.

(iv) 2-cyano-5-méthoxy-1,2,3,4-tétrahydro-1-napthol. On réduit le produit obtenu dans l'étape (iii) (16,2 g, 0,080 moles) avec du borohydrure de sodium (3,1 g, 0,082 moles) dans du méthanol absolu (500 ml). On évapore sous vide, on ajoute une mélange eau/glace, on acidifie avec du HCl concentré et on extrait par de l'acétate d'éthyle. Après évaporation du solvent, on obtient le 2-cyano-5-méthoxy-1,2,3,4-tétrahydro-1-naphtol (16,2 g); p.f. 96-98°C.

(v) 2-cyano-5-méthoxy-3,4-dihydronaphtalène. On chauffe à 120°C (température externe), pendant 3 heures, un mélange du produit obtenu dans l'étape (iv) (16,2 g, 0,079 moles) et du $POCl_3$ (30 ml, 0,32 moles) dans de la pyridine (200 ml). On refroidit le mélange et on l'acidifie en y ajoutant goutte à goutte de l'acide chlorhydrique 2N. On traite la solution avec de l'acétate d'éthyle, on sépare la phase organique et on la lave avec une solution saturée de bicarbonate de sodium et de l'eau. On sèche (sulfate de sodium), on filtre et on évapore à sec en obtenant le produit indiqué dans le titre (9,8 g); p.f. 47-49°C (éther isopropylique).

(vi) chlorhydrate de 2-aminométhyl-5-méthoxy-1,2,3,4-tétrahydronaphtalène.

On ajoute goutte à goutte une solution du produit de l'étape (v) (9,2 g, 0,05 moles) dans du tétrahydrofuranne anhydre (150 ml) dans un mélange d'hydrure de lithium et d'aluminium (3,8 g, 0,1 moles) et tétrahydrofuranne anhydre (50 ml) sous atmosphère d'azote. On chauffe le mélange réactionnel

au reflux pendant 4 heures, on le refroidit et on y ajoute très lentement de l'eau (40 ml). On l'extrait par de l'acétate d'éthyle (2 x 300 ml), on sèche la phase organique et on l'évapore. On purifie le résidu par flash chromatographie en utilisant un mélange méthanol: ammoniac 97:3 comme éluant. On prépare le chlorhydrate par traitement de la base libre ainsi obtenue avec de l'isopropanol saturé d'acide chlorhydrique. On obtient 9 g du produit indiqué dans le titre; p.f. 231-232°C (éthanol).

(B) chlorhydrate de 2-aminométhyl-6-méthoxy-1,2,3,4-tétrahydronaphtalène.

Le produit est obtenu selon la méthode opératoire décrite dans la Préparation (A) ci-dessus mais à partir du 6-méthoxy-3,4-dihydronaphtalen-1-one au lieu du 5-méthoxy-3,4-dihydronaphtalen-1-one; p.f. 222-224°C (éthanol).

(C) chlorhydrate de 2-aminométhyl-8-méthoxy-1,2,3,4-tétrahydronaphtalène.

On fait tomber goutte à goutte pendant 10 minutes du cyanure de triméthylsilyle (6,9 g, 9,3 ml, 0,07 moles) dans un mélange de 8-méthoxy-3,4-dihydronaphtalèn-2(1H)-one (10,9 g, 0,06 moles) préparé comme décrit par la littérature (J. Chem. Soc., 1958, 409), d'acétonitrile anhydre (60 ml), et d'une quantité catalytique de iodure de zinc sous atmosphère d'azote. On chauffe à la température externe de 80°C pendant 3 heures. On refroidit le mélange réactionnel, on y ajoute lentement de l'acide chlorhydrique 1N (20 ml) et on agite à la température ambiante pendant 2 heures. On concentre le mélange sous vide, on reprend le résidu ainsi obtenu par de l'acétate d'éthyle, on lave la solution à l'eau, on la sèche sur $Na_2SO_4$ et, après filtration, on l'évapore à sec. On triture le produit obtenu avec de l'éther de pétrole, on le filtre et on le dissout dans de la pyridine (100 ml). On ajoute goutte à goutte du $POCl_3$ (20 ml) pendant 10 minutes et on chauffe le mélange reactionnel à 120°C (température externe) pendant 3 heures. On verse le mélange sur de la glace, on rend acide le mélange avec de l'acide chlorhydrique concentré et on l'extrait par de l'éther éthylique. On lave la phase organique à l'eau, on la sèche et on l'évapore. Par cristallisation du résidu dans de l'éther isopropylique, on obtient le 2-cyano-8-méthoxy-3,4-dihydronaphtalène (7,2 g); p.f. 66-68°C.
On hydrogène le produit ainsi obtenu à la température et à la pression ambiante dans de l'éthanol à 95% (100 ml) en utilisant du palladium sur charbon à 5% comme catalyseur.
Lorsque la quantité théorique d'hydrogène a été consommée, on filtre le mélange réactionnel, on concentre le filtrat sous pression reduite, on triture le résidu avec de l'éther de pétrole et on le recueille par filtration, en obtenant le 2-cyano-8-méthoxy-1,2,3,4-tétrahydronaphtalène (7 g); p.f. 66-68°C.
On dissout ledit produit dans du tétrahydrofuranne anhydre (30 ml), on ajoute la solution ainsi obtenue à une suspension d'hydrure de lithium et d'aluminium (1,5 g, 0,04 moles) dans du tétrahydrofuranne anhydre (20 ml) et on chauffe au reflux pendant 4 heures. On réfroidit jusqu'à la température ambiante et on traite le mélange avec de l'eau et, ensuite, avec de l'acétate d'éthyle. On sépare la phase organique, on la traite avec de l'acide chlorhydrique dilué, on sépare les eaux acides et on les rend basiques par de l'ammoniaque. On extrait par de l'acétate d'éthyle, on lave la phase organique à l'eau, on la sèche et l'évapore à sec. On reprend le résidu avec de l'isopropanol et on précipite le chlorhydrate de 2-aminométhyl-8-méthoxy-1,2,3,4-tétrahydronaphtalène (2,49 g) par traitement avec de l'isopropanol saturé par l'acide chlorhydrique. P.f. 210-212°C (isopropanol).

(D) bromhydrate de 2-aminométhyl-8-hydroxy-1,2,3,4-tétrahydronaphtalène.

On chauffe au reflux pendant 4 heures un mélange de produit de la Préparation (C) ci-dessus (3 g, 0,013 moles) et d'une solution aqueuse à 48% d'acide bromhydrique (50 ml). On concentre sous vide, et on reprend le résidu avec de l'éthanol absolu (3 x 50 ml) en évaporant chaque fois le solvant. On triture le résidu avec de l'acétone, on le filtre et on le lave avec de l'acétone et, ensuite, avec de l'éther éthylique. On obtient ainsi le produit indiqué dans le titre (2,8 g). P.f. 233-235°C (isopropanol).

(E) bromhydrate de 2-aminométhyl-5-hydroxy-1,2,3,4-tétrahydronaphtalène.

En suivant la procédure de la Préparation précédente, mais en utilisant comme produit de départ le composé de la préparation (B), on obtient le composé indiqué ci-dessus, P.f. 212-214°C (éthanol).

(F) chlorhydrate de 2(S)-minométhyl-7-méthoxy-1,2,3,4-tétrahydronaphtalène.

(i) acide 7-méthoxy-1,2,3,4-tétrahydronaphtalène-2(S)-carboxylique.

On ajoute de la (R)-(+)-alpha-méthylbenzylamine (25,8 ml, 0,2 mole) à une solution du mélange racémique de l'acide 7-méthoxy-1,2,3,4-tétrahydronaphtalène-2-carboxylique (41 g, 0,2 mole) dans de l'acétone (800 ml) et après environ 2 heures à température ambiante on sépare le sel (45,2 g) par filtration. On le recristallise dans de l'acétone douze fois en obtenant un produit caractérisé par un [alpha] constant de -20,5° (chloroforme, c = 1,4 %). On reprend le sel ainsi obtenu dans de l'eau (30 ml), on rend la solution acide avec de l'acide chlorhydrique concentré, et on extrait la solution acide par de l'éther éthylique. On sèche la phase organique, on l'évapore à sec et on recristallise l'acide ainsi obtenu dans du benzène (20 ml) en obtenant l'acide 7-méthoxy-1,2,3,4-tétrahydronaphtalène-2(S)-carboxylique (0,9 g); p.f. 133-135°C. [alpha] = -45,1°(chloroforme, c = 1,4 %).

Pour l'attribution de sa configuration absolue, on convert le produit ainsi obtenu dans le 2-amino-7-méthoxy-1,2,3,4-tétrahydronaphtalène correspondant selon la réaction de Curtius.

Le 2-amino-7-méthoxy-1,2,3,4-tétrahydronaphtalène ainsi obtenu a un [alpha] correspondant à l'isomère 2(S)- qui est décrit dans la demande de brevet européen 303545. Comme l'ordre de priorité des groupes liés au carbone asymétrique du 2-amino-7-méthoxy-1,2,3,4-tétrahydronaphtalène est identique au celle de l'acide 7-méthoxy-1,2,3,4-tétrahydronaphtalène-2-carboxylique et que la réaction de Curtius est stéréoconservatrice, on peut correctement attribuer la configuration absolue (S) à l'acide 7-méthoxy-1,2,3,4-tétrahydronaphtalène-2-carboxylique obtenu comme décrit ci-dessus.

(ii) 7-méthoxy-1,2,3,4-tétrahydro-2(S)-naphtalène carboxamide. A une solution de l'acide obtenu dans l'étape (i) (10,8 g, 0,052 mole) dans de l'acétone (200 ml), refroidie à -10°C, on ajoute une solution de triéthylamine (10,2 ml, 0,072 mole) dans de l'acétone (50 ml) pendant 15 minutes et une solution de chloroformiate d'éthyle (7,9 ml, 0,080 mole) dans de l'acetone (80 ml). Après 1,5 heure à -10°C on ajoute goutte à goutte une solution concentrée d'ammoniaque (16,6 ml, 0,133 mole) et on laisse le mélange réactionnel à -10°C pendant 1 heure et à la température ambiante pendant 3 heures. On évapore l'acétone, on reprend le résidu par de l'acétate d'éthyle (500 ml) et on lave la solution successivement avec de l'eau, une solution de bicarbonate de sodium, de l'acide chlorhydrique 6N, et de l'eau, puis on la sèche et l'évapore. On triture le résidu dans de l'éther isopropylique, on filtre et on obtient l'amide indiqué ci-dessus (9,5 g);

p.f. 159-161°C (acétate d'éthyle). [alpha] = -52,2° (chloroforme, c = 1,4 %). Excès énantiomérique: 96,5 %.

(iii) chlorhydrate de 2(S)-aminométhyl-7-méthoxy-1,2,3,4-tétrahydronaphtalène.

Sous atmosphère d'azote, on chauffe au reflux une solution du composé de l'étape (ii) (9,5 g, 0,046 mole) dans du tétrahydrofuranne anhydre (167 ml) et on y ajoute goutte à goutte une solution de borane-méthylsulfure 10M (14,2 ml, 0,142 mole) dans du tétrahydrofuranne anhydre (60 ml). On chauffe le mélange au reflux pendant 4 heures, on le refroidi à 0-5°C, on y ajoute lentement du méthanol (95 ml), on chauffe la solution au reflux pendant 1 heure et on l'évapore à sec. On reprend le résidu par une solution d'hydroxyde de sodium 1N, on extrait dans de l'acétate d'éthyle, on lave la phase organique à l'eau, on la sèche et on l'évapore. On purifie le résidu par flash chromatographie en éluant avec un mélange méthanol/ammoniac 98/2. On dissout le produit ainsi obtenu dans de l'isopropanol (30 ml) et on ajoute de l'isopropanol saturé d'acide chlorhydrique. Par filtration, on sépare le précipité (5,3 g); p.f. 228-230°C; [alpha] = -80,4° (méthanol, c = 1,4 %).

L'acide 7-méthoxy-1,2,3,4-tétrahydronaphtalène-2-carboxylique de départ, qui est un produit connu, a été préparé de la façon suivante:

On ajoute pendant une heure une solution de 7-méthoxy-3,4-dihydronaphtalèn-1(2H)-one (66,4 g, 0.376 mole) dans du tétrahydrofuranne anhydre (350 ml) dans un mélange de diéthylcarbonate distillé (116 ml, 0,957 mole), hydrure de sodium à 80% (39,7 g, 1,32 mole) et tétrahydrofuranne anhydre (350 ml) chauffé à 60°C. Or chauffe au reflux pendant 4 heures le mélange réactionnel ainsi obtenu, on le refroidit et on y ajoute goutte à goutte de l'acide acétique jusqu'à pH acide, et de l'eau jusqu'à dissolution du précipité. On extrait à l'éther éthylique, on lave à l'eau et à une solution de carbonate de sodium. On sèche et on évapore à sec en obtenant un produit huileux qui est purifié par distillation sous pression réduite. On obtient ainsi 90 g de l'ester éthylique de l'acide 7-méthoxy-1-oxo-1,2,3,4-tétrahydronaphtalèn-2-carboxylique. P.e. 160-165°C/0,4 mmHg.

On dissout le produit ainsi obtenu dans un mélange d'acide acétique glacial (600 ml) et une solution d'acide perchlorique à 70% (4 ml) et on l'hydrogène à température et pression ambiante en utilisant du palladium sur charbon à 10% en tant que catalysateur. Après 3 heures, on filtre sur célite, on verse dans l'eau (4500 ml) et on extrait à l'acétate d'éthyle. On lave la phase organique à l'eau et à une solution saturée de bicarbonate de sodium, on sèche sur sulfate de sodium, on filtre et on évapore à sec en obtenant un produit huileux qui est distillé à 0,3 mmHg et 130°C. On obtient ainsi 65,8 g de l'ester éthylique de l'acide 7-méthoxy-1,2,3,4-tétrahydronaphtalen-2-carboxylique.

Un mélange de l'ester ainsi obtenu (159,5 g, 0,68 mole) et de l'hydroxyde de sodium (29,9 g, 0,75 mole) dans de l'eau (600 ml) et de l'éthanol à 95% (600 ml) est chauffé au reflxu pendant 2 heures et demi. On évapore l'éthanol, on rend la solution acide par addition d'acide chlorhydrique concentré et on extrait à l'acétate d'éthyle. On sèche l'extrait organique sur $Na_2SO_4$, on le filtre et on l'évapore à sec en obtenant l'acide 7-méthoxy-1,2,3,4-tétrahydronaphtalen-2-carboxylique qui est donc cristallisé de l'éther isopropylique. P.f. 125-127°C.

En alternative, le composé (F) peut être aussi obtenu à partir du produit racémique selon la méthode suivante:

(i') On ajoute une solution d'acide L(+) mandélique (11,93 g, 0,078 mole) dans du méthanol (100 ml) à une solution de 2-aminométhyl-7-méthoxy-1,2,3,4-tétrahydronaphtalène (15 g, 0,078 mole)

dans du méthanol (100 ml). On sépare le précipité des eaux mères par filtration et on recristallise le sel sept fois dans du méthanol en obtenant un produit caractérisé par [alpha]=- 31,4° (c = 1,4 %, MeOH).

(ii') On reprend le sel ainsi obtenu dans une solution d'HCl 0,1N, on extrait à l'acétate d'éthyle, on rend basique la solution aqueuse avec une solution de $Na_2CO_3$ et on l'extrait par de l'acétate d'éthyle qui est ensuite séché et évaporé à sec. On dissout le résidu ainsi obtenu dans de l'isopropanol et on y ajoute de l'isopropanol saturé d'acide chlorhydrique. Par filtration on sépare le composé (F). P.f. 228-230°C; [alpha] = -79,0° (c = 1,4 %, MeOH).

(G) chlorhydrate de 2(R)-aminométhyl-7-méthoxy-1,2,3,4-tétrahydronaphtalène.

(i) acide 7-méthoxy-1,2,3,4-tétrahydro-2(R)-naphtalènecarboxylique.

On réunit les eaux mères de la précipitation du sel, et de la 1ère et de la 2ème recristallisation décrites dans la préparation (F)(i), et on les évapore. On ajoute au résidu de l'acide chlorhydrique, on extrait la solution par de l'éther éthylique et on évapore la phase organique à sec, en obtenant l'acide 7-méthoxy-1,2,3,4-tétrahydro-2-naphtalènecarboxylique (26 g, 0,126 mole). On dissout cet acide dans de l'acétone (250 ml) et on ajoute à la solution ainsi obtenue de la (S)-(-)-alpha-méthylbenzylamine (16,3 ml, 0,126 mole). Après 2 heures à la température ambiante, on filtre et on recueille le sel précipité (33,5 g). On recristallise ce sel dans l'acétone dix fois, puis on le reprend avec de l'eau (30 ml), on rend la solution acide en y ajoutant de l'acide chlorhydrique concentré et on extrait la solution acide par de l'éther éthylique. On sèche la phase organique, on la filtre, on l'évapore à sec et l'on obtient l'acide 7-méthoxy-1,2,3,4-tétrahydro-2-(R)-naphtalène- carboxylique (1 g). P.f. 133-135°C (benzène); [alpha] = + 44,6° (chloroforme, c = 1,4%).

Selon la méthode décrite dans la préparation (F)(i) on transforme l'acide ci-dessus dans le chlorhydrate de 2-amino-7-méthoxy-1,2,3,4-tétrahydronaphtalène. On obtient un produit caractérisé par un [alpha] = + 66,6° (méthanol, c = 0,5 %) qui correspond à l'/alpha/ du 2(R)-amino-7-méthoxy-1,2,3,4-tétrahydronaphtalène (Molecular Pharmacology, 1982, 22, 281).

La configuration absolue du produit est ainsi confirmée.

(ii) 7-méthoxy-1,2,3,4-tétrahydro-2(R)-naphtalènecarboxamide.

En utilisant la procédure décrite dans la Préparation (F)(ii), mais en partant de l'acide 7-méthoxy-1,2,3,4-tétrahydro-2(R)-naphtalène- carboxylique (10,8 g, 0,052 mole) on obtient le 7-méthoxy-1,2,3,4-tétrahydro-2(R)-napthalènecarboxamide (9,5 g). P.f. 157-159°C (acétate d'éthyle); [alpha] = +52,7° (chloroforme, c = 1,4%). Excès énantiomérique: 94%.

(iii) chlorhydrate de 2(R)-aminométhyl-7-méthoxy-1,2,3,4-tétrahydronaphtaléne.

En suivant le mode opératoire décrit dans la Péparation (F)(iii), mais en partant du 7-méthoxy-1,2,3,4-tétrahydro-2(R)-naphtalène-carboxamide (9 g, 0,044 mole) on obtient le composé (G)(5,5 g); p.f. 229-231°C (isopropanol). [alpha] = +83,6° (méthanol, c = 1,4 %)

En alternative le composé (G) peut être obtenu à partir des eaux mères de la précipitation du sel et de la 1ère et de la 2ème rescristallisations décrites dans la préparation alternative (F)(i') selon la méthode suivante:

On évapore la solution méthanolique à sec, on reprend le résidu par une solution d'acide chlorhydrique 1N et on lave à l'acétate d'éthyle. On rend la solution aqueuse basique avec une solution de NaOH 1N et on l'extrait à l'acétate d'éthyle. On sèche la phase organique et on l'évapore à sec. On dissout le résidu ainsi obtenu dans du méthanol et on y ajoute la quantité équimoléculaire d'acide D(-)mandélique. On cristallise le précipité dans du méthanol sept fois en obtenant un produit caractérisé par [alpha] = + 31,8° (c = 1,4%, MeOH).

On dissout le sel ainsi obtenu dans une solution 0,1N d'acide chlorhydrique et on extrait à l'acétate d'éthyle. On rend basique la solution aqueuse avec une solution aqueuse de $Na_2CO_3$ et on extrait à l'acétate d'éthyle. On sèche la phase organique et on l'évapore à sec, on dissout le résidu dans de l'isopropanol et on y ajoute de l'isopropanol saturé d'acide chlorhydrique. On obtient par filtration le composé (G). P.f. 228-230°C, [alpha] = + 83,1° (c = 1,4 %, MeOH).

(H) 2(R)-aminométhyl-7-hydroxy-1,2,3,4-tétrahydronaphtalène.

On chauffe au reflux pendant 5 heures une solution du produit de la préparation (G) (5 g, 0,022 mole) dans de l'acide bromhydrique aqueux à 48% (100 ml) et, ensuite, on évapore à sec. On reprend le résidu dans de l'ammoniaque concentrée (30 ml), on extrait la solution par de l'acétate d'éthyle (4 x 200 ml), on sèche les extraits organiques combinés, on les filtre et on les évapore à sec. Par recristallisation du résidu dans de l'isopropanol (80 ml) on obtient le produit indiqué dans le titre (2,4 g). P.f. 192-194°C; [alpha] = + 116,78° (méthanol, c = 1%).

(I) 2(S)-aminométhyl-7-hydroxy-1,2,3,4-tétrahydronaphtalène.

Suivant les modes opératoires de la préparation (H), mais en utilisant comme produit de départ le

composé de la préparation (F) (5 g, 0,022 mole), on obtient le composé ci-dessus (2,9 g); p.f. 191-193°C (isopropanol); [alpha] = - 106,5° (méthanol, c = 1%).

(J) 2-aminométhyl-6-hydroxy-1,2,3,4-tétrahydronaphtalène.

En suivant les modes opératoires de la préparation (H), mais en utilisant comme produit de départ le composé de la préparation (B), on obtient le composé indiqué dans le titre. P.f. 181-183°C (isopropanol).

(K) chlorhydrate du [(2-aminométhyl-1,2,3,4-tétrahydro-7-naphtyl)oxy]acétate d'éthyle.

(i) 7-hydroxy-2-(N-tert-butoxycarbonyl)aminométhyl-1,2,3,4-tétrahydronaphtalène.

On obtient le 2-aminométhyl-7-méthoxy-1,2,3,4-tétrahydronaphtalène selon la méthode décrite dans la préparation (A), mais à partir de la 7-méthoxy-3,4-dihydronaphtalen-1-one et on le traite avec de l'acide bromhydrique aqueux selon la méthode décrite dans la préparation (H). On prépare une suspension de ce 2-aminométhyl-7-hydroxy-1,2,3,4-tétrahydronaphtalène (6 g, 0,034 mole) ainsi obtenu dans du diméthylformamide (89 ml) et de la triéthylamine (4,7 ml, 0,034 mole). On agite cette suspension à la température ambiante pendant 10 minutes et on y ajoute du di-tert-butyl-dicarbonate à 90% (8,2 g, 0,034 mole). On agite le mélange réactionnel à la température ambiante pendant 3 heures, on le verse dans de l'eau (environ 400 ml) et on extrait la solution aqueuse ainsi obtenue par de l'acétate d'éthyle. On lave la solution organique à l'eau, on la sèche sur du sulfate de sodium, on la filtre et on l'évapore. On obtient une huile qui est purifiée par flash chromatographie en utilisant un mélange acétate d'éthyle:cyclohexane 2:8.

Par traitement sous vide du produit huileux ainsi obtenu, on obtient une poudre vitreuse.

IR (KBr): 3364 (b): O-H, CON-H; 1690: OC=ONH $cm^{-1}$.

(ii) [(2-(N-tert-butoxycarbonyl)aminométhyl-1,2,3,4-tétrahydro-7-naphtyl)oxy]acétate d'éthyle.

On agite à la température ambiante pendant 30 minutes un mélange de produit obtenu ci-dessus (3,4 g, 0,009 mole), de carbonate de potassium en poudre (4 g, 0,09 mole) et d'acétone (100 ml) et, ensuite, on ajoute du bromoacétate d'éthyle (4,56 g, 3 ml, 0,027 mole). On chauffe le mélange réactionnel à reflux pendant 5 heures, on le filtre et on le concentre sous vide. On dissout le résidu dans de l'éther éthylique, on lave la solution obtenue à l'eau, on la sèche sur du sulfate de sodium, on la filtre et on l'évapore à sec sous pression réduite. On triture le produit avec de l'éther isopropylique et on le filtre. On obtient ainsi le[(2-(N-tert-butoxycarbonyl)aminométhyl-1,2,3,4-tétrahydro-7-naphtyl)oxy]acétate d'éthyle (p.f. 94-97°C).

(iii) chlorhydrate du [(2-aminométhyl-1,2,3,4,-tétrahydro-7-naphtyl)oxy]acétate d'éthyle.

On refroidit à environ 0°C un mélange du produit obtenu dans l'étape ii) (2,1 g, 0,0058 mole) et de l'éthanol absolu (15 ml) et on y ajoute une solution éthanolique 7,2 N de HCl gazeux (5 ml). Lorsque l'addition est terminée, on chauffe le mélange à environ 50°C pendant 30 minutes, on le concentre sous vide, on triture le résidu avec de l'acétone et on le filtre. On obtient ainsi le composé indiqué dans le titre (1,2 g); p.f. 136°-138°C (isopropanol).

(L) Chlorhydrate du[(2-aminométhyl-1,2,3,4-tétrahydro-7-naphtyl)oxy]butanoate d'éthyle.

On agite à température ambiante pendant 30 minutes un mélange du composé obtenu dans la préparation (K) (i) (3,8 g, 0,013 mole), de carbonate de potassium en poudre (4 g) et d'acétone (100 ml) et, ensuite, on ajoute du 4-bromobutanoate d'éthyle (11,5 g, 0,06 mole). On chauffe le mélange réactionnel au reflux pendant 10 heures, on le filtre, on l'évapore à sec et on reprend le résidu avec de l'éthanol absolu (15 ml) et avec une solution 6N de HCl gazeux dans l'éthanol absolu (25 ml). On chauffe ce mélange à 90°C extérnes pendant 4 heures, puis on concentre sous vide. On triture le résidu dans de l'acétone et on le filtre en obtenant le produit indiqué dans le titre (2,7 g); p.f. 146- 148°C.

(M) 2-aminométhyl-7-hydroxy-1,2,3,4-tétrahydronaphtalène.

Suivant le mode opératoire de la Préparation (H), mais à partir du racémate de 2-aminométhyl-7-méthoxy-1,2,3,4-tétrahydronaphtalène (préparé comme décrit dans la demande de brevet EP-213080), on obtient le 2-aminométhyl-7-hydroxy-1,2,3,4-tétra-hydronaphtalène; p.f. 187-189°C (isopropanol).

(N) Chlorhydrate de 2-aminométhyl-1,2,3,4-tétrahydronaphtalène.

Suivant le mode opératoire de la Préparation (C), mais à partir de la 3,4-dihydronaphtalen-2(1H)-one, on obtient le composé indiqué dans le titre; p.f. 228-230°C (éthanol).

(O) Chlorhydrate de (+) 2-aminométhyl-6-méthoxy-1,2,3,4-tétra-hydronaphtalène (chlorhydrate de (2R) ou (2S)-aminométhyl-6-méthoxy-1,2,3,4-tétrahydronaphtalène).

(i) acide (+) 6-méthoxy-1,2,3,4-tétrahydronaphtalen-2-carboxylique (acide (2R) ou (2S) 6-méthoxy-1,2,3,4-tétrahydronaphtalen-2-carboxylique).

On ajoute une solution de (R)-(+)-alpha-méthylbenzylamine (88,3 g, 93 ml, 0,72 mole) dans de l'acétone (500 ml) à une solution du mélange racémique de l'acide 6-méthoxy-1,2,3,4-tétrahydro-naphtalen-2-carboxylique (150 g, 0,727 mole) dans de l'acétone (250 ml). On laisse à température ambiante pendant une nuit et après on sépare le sel (152 g) par filtration. On le cristallise dans de l'acétone onze

fois en obtenant un produit (6,3 g) caractérisé par /alpha/ = + 47,7° (c = 1,4%, $CHCl_3$).

On reprend le sel ainsi obtenu dans une solution 0,1 N de NaOH et on lave la solution aqueuse à l'éther éthylique (3 x 30 ml) avant de la traiter par un charbon décolorant. On filtre et on rend la solution acide avec de l'acide chlorhydrique concentré. On sépare l'acide ainsi obtenu par filtration, on le lave à l'eau et à l'éther éthylique, et on le sèche à l'étuve. On obtient 3,3 g d'acide optiquement actif. P.f. 129-130°C. [alpha] = + 47,9° (c = 1,4 %, $CHCl_3$).

(ii) (+) 6-méthoxy-1,2,3,4-tétrahydronaphtalen-2-carboxamide ((2R) ou (2S) 6-méthoxy-1,2,3,4-tétrahydronaphtalen-2-carboxamide.

A une solution de l'acide obtenu dans l'étape (i) (3 g, 0,014 mole) dans de l'acétone (50 ml), refroidie à -10°C, on ajoute une solution de triéthylamine (2,7 ml, 0,019 mole) et de chloroformiate d'éthyle (2 ml, 0,021 mole) dans de l'acétone (40 ml). Après 1,5 heures à -10°C, on ajoute goutte à goutte une solution concentrée d'ammoniaque (4,5 ml, 0,036 mole) et on laisse le mélange réactionnel à -10°C pendant 1 heure et à la température ambiante pendant une nuit. On concentre sous vide, on y ajoute de l'acétate d'éthyle (150 ml) et on lave la solution successivement avec de l'eau, une solution saturée de bicarbonate de sodium, de l'acide chlorhydrique 6N, et de l'eau, puis on la sèche et l'évapore. On triture le résidu dans de l'éther isopropylique, on filtre et on obtient l'amide indiqué ci-dessus (1,7 g); p.f. 136-138°C, [alpha] = + 40,2° (chloroforme, c = 1,4 %).

(iii) chlorhydrate de (+) 2-aminométhyl-6-méthoxy-1,2,3,4-tétrahydronaphtalène (chlorhydrate de (2R) ou (2S)-amino-méthyl-6-méthoxy-1,2,3,4-tétrahydronaphtalène).

Sous atmosphère d'azote, on chauffe au reflux une solution du composé de l'étape (ii) (1,6 g, 0,0077 mole) dans du tétrahydrofuranne anhydre (20 ml) et on y ajoute goutte à goutte une solution de borane-méthylsulfure 10M (2,3 ml, 0,023 mole) et du tétrahydrofuranne anhydre (5 ml). On chauffe le mélange au reflux pendant 4 heures, on y ajoute lentement du méthanol (5 ml), on chauffe la solution au reflux pendant 1 heure, on y ajoute un solution 1N d'acide chlorhydrique (10 ml) et on chauffe au reflux pendant 1 heure. On concentre à pression reduite, on rend basique par addition d'ammoniaque, on extrait dans de l'acétate d'éthyle, on lave la phase organique à l'eau, on la sèche et l'évapore.

On purifie le résidu par chromatographie-flash en éluant avec un mélange méthanol/ammoniac 98/2. On dissout le produit ainsi obtenu dans de l'isopropanol (30 ml), on filtre et on ajoute de l'isopropanol saturé d'acide chlorhydrique. Par filtration, on sépare le précipité (1,1 g); p.f. 245-255°C, [alpha] = + 70,7° (méthanol, c = 1,4 %).

En alternative, le composé (O) peut être aussi obtenu à partir du produit racémique selon la méthode suivante:

(i') On ajoute une solution d'acide L(+) mandélique (11,93 g, 0,078 moles) dans du méthanol (100 ml) à une solution de 2-aminométhyl-6-méthoxy-1,2,3,4-tétrahydronaphtalène (15 g, 0,078 mole) dans du méthanol (100 ml). On sépare le précipité des eaux mères par filtration et on cristallise le sel sept fois dans du méthanol en obtenant un produit caractérisé par [alpha] = + 92,7° (c = 1,4%, MeOH).

(ii') On reprend le sel ainsi obtenu dans une solution d'HCl 0,1N, on extrait par de l'acétate d'éthyle, on rend basique la solution aqueuse avec une solution de $Na_2CO_3$ et on l'extrait par de l'acétate d'éthyle qui est ensuite évaporé à sec. On dissout le résidu ainsi obtenu dans de l'isopropanol et on y ajoute de l'isopropanol saturé d'acide chlorhydrique. Par filtration on sépare le composé (O). [alpha] = +76.7 (c = 1,4 %, MeOH).

(P) Bromhydrate de (+) 2-aminométhyl-6-hydroxy-1,2,3,4-tétrahydronaphtalène (bromhydrate de (2R) ou (2S)-aminométhyl-6-hydroxy-1,2,3,4-tétrahydronaphtalène).

On chauffe au reflux pendant 5 heures un mélange du produit de la préparation (O) ci-dessus (0,86 g, 0,0038 mole) et d'une solution aqueuse à 48% d'acide bromhydrique (15 ml). On évapore à sec, on reprend le résidu avec de l'éthanol absolu (3 x 15 ml) en évaporant chaque fois le solvant. On triture le résidu avec de l'acétone et on filtre en obtenant le produit indiqué dans le titre (0,82 g). P.f. 248-252°C [alpha] = + 61° (c = 1,4 %, MeOH).

(Q) Chlorhydrate de (-) 2-aminométhyl-6-méthoxy-1,2,3,4-tétrahydronaphtalène (chlorhydrate de (2S) ou (2R)-aminométhyl-6-méthoxy-1,2,3,4-tétrahydronaphtalène).

(i) acide (-) 6-méthoxy-1,2,3,4-tétrahydronaphtalen-2-carboxylique (acide (2S) ou (2R) 6-méthoxy-1,2,3,4-tétrahydronaphtalen-2-carboxylique).

On réunit les eaux mères de la précipitation du sel et de la 1ère, 2ème et 3ème cristallisations décrites dans la préparation (O)(i), et on les évapore. On ajoute au résidu de l'acide chlorhydrique, on extrait la solution par de l'éther éthylique et on évapore la phase organique à sec, en obtenant l'acide 6-méthoxy-1,2,3,4-tétrahydro-2-naphtalènecarboxylique (126 g, 0,61 mole). On dissout cet acide dans de l'acétone (2000 ml) et on ajoute à la solution ainsi obtenue une solution de (S)-(-)-alpha-méthylbenzylamine (80,6 ml, 0,61 mole) dans de l'acétone (500 ml), On filtre et on recueille le sel précipité (116

g). On recristallise ce sel dans de l'acétone dix fois en obtenant 5,6 g d'un produit ayant [alpha] = -46,7° (c = 1,4 %, $CHCl_3$).

On reprend le résidu avec une solution 0,1 N d'hydroxyde de sodium et on lave à l'éther éthylique (3 x 30 ml). On rend la solution acide en y ajoutant de l'acide chlorhydrique concentré et on sépare l'acide ainsi obtenu par filtration.

On le lave à l'eau, et à l'éther de pétrole et on le sèche à l'étuve, en obtenant 3,37 g d'acide optiquement actif. P.f. 129-130°C. [alpha] = -52,5° (c = 1,4 %, $CHCl_3$).

(ii) (-) 6-méthoxy-1,2,3,4-tétrahydro-2-naphtalène-carboxamide ((2S) ou (2R) 6-méthoxy-1,2,3,4-tétrahydro-2-naphtalènecarboxamide).

En utilisant la même procédure de la préparation (O)(ii), mais à partir du produit de l'étape (i) ci-dessus (3 g, 0,014 mole), on obtient 1,7 g de l'amide indiqué dans le titre. P.f. 138-140°C; [alpha] = -45,8° (c = 1,4 %, $CHCl_3$).

(iii) Chlorhydrate de (-) 2-aminométhyl-6-méthoxy-1,2,3,4-tétrahydronaphtalène (chlorhydrate de (2S)ou (2R)-aminométhyl-6-méthoxy-1,2,3,4-tétrahydronaphtalène).

En suivant le mode opératoire décrit dans la Préparation (O)(iii), mais à partir du (-) 6-méthoxy-1,2,3,4-tétrahydro-naphtalène-2-carboxamide (1,61 g, 0,0078 mole), on obtient le composé indiqué dans le titre (1 g). P.f. 258-260°C (déc.); [alpha] = -73,8° (c = 1,4 %, MeOH).

En alternative le composé (Q) peut être obtenu à partir des eaux mères de la précipitation du sel et de la 1ère et de la 2ème rescristallisation décrites dans la préparation alternative (O)(i'), selon la méthode suivante. On évapore la solution méthanolique à sec, on reprend le résidu par une solution d'acide chlorhydrique 1N et on lave par de l'acétate d'éthyle. On rend la solution aqueuse basique avec une solution de NaOH 1N et on l'extrait par de l'acétate d'éthyle. On sèche la phase organique et on l'évapore à sec. On dissout le résidu ainsi obtenu dans du méthanol et on y ajoute la quantité équimoléculaire d'acide- D(-)mandélique. On cristallise le précipité dans du méthanol sept fois en obtenant un produit caractérisé par [alpha] = - 90,5° (c = 1,4 %, MeOH).

On dissout le sel ainsi obtenu dans une solution 0,1N d'acide chlorhydrique et on lave par de l'acétate d'éthyle. On rend basique la solution aqueuse avec une solution aqueuse de Na2CO3 et on extrait par de l'acétate d'éthyle. On sèche la phase organique et on l'évapore à sec, on dissout le résidu dans de l'isopropanol et on y ajoute de l'isopropanol saturé d'acide chlorhydrique. On obtient par filtration le composé (Q). [alpha] = - 76,4° (c = 1,4 %, MeOH).

(R) Bromhydrate de (-) 2-aminométhyl-6-hydroxy-1,2,3,4-tétrahydronaphtalène (bromhydrate de (2S) ou (2R)-aminométhyl-6-hydroxy-1,2,3,4-tétrahydronaphtalène).

En suivant la procédure de la Préparation (P), mais à partir du composé de la Préparation (Q) (0,75 g, 0,0033 mole), on obtient le composé indiqué ci-dessus. P.f. 250-252°C; [alpha] = -64,2° (c = 1,4 %, MeOH).

(S) Chlorhydrate de [(2(R)-aminométhyl-1,2,3,4-tétrahydronapht-7-yl)oxy]acétate d'éthyle.

(i) 7-hydroxy-2(R)-(tertbutoxycarbonyl)aminométhyl-1,2,3,4-tétrahydronaphtalène.

On agite une suspension du produit de la Préparation (H) ci-dessus sous forme de base libre (4,6 g, 0,026 mole), dans du diméthyl formamide (60 ml) et de la triéthylamine (3,6 ml, 0,026 mole) pendant 15 minutes à température ambiante et on y ajoute du di-tertbutyl-dicarbonate à 90% (6,3 g, 0,026 mole).

Après 3 heures, sous agitation à température ambiante, on verse le mélange réactionnel dans l'eau (~ 300 ml) et on extrait par de l'acétate d'éthyle. La phase organique est ensuite lavée à l'eau, séchée et évaporée. Le résidu qu'on obtient est purifié par chromatographie flash en utilisant un mélange acétate d'éthyle: cyclohexane 2:8 comme éluant.

(ii) [(2R)2-tertbutoxycarbonylaminométhyl-1,2,3,4-tétrahydro-7-naphtyl)oxy]acétate d'éthyle.

On agite à température ambiante pendant 1 heure un mélange du produit obtenu à l'étape (i) ci-dessus (3,6 g, 0,013 mole) et de carbonate de potassium en poudre (4,4 g, 0,03 mole) dans de l'acétone (100 ml) et on y ajoute du bromoacétate d'éthyle (5,1 g, 0,03 mole). Le mélange réactionnel ainsi obtenu est chauffé au reflux pendant 5 heures, filtré et concentré sous pression réduite. On dissout le résidu dans de l'éther éthylique, on lave la solution organique à l'eau, on la sèche et on évapore le solvant sous pression réduite. On lave le résidu à une petite quantité d'éther isopropylique en obtenant le [((2R)2-tertbutylaminométhyl-1,2,3,4-tétrahydro-7-naphtyl)oxy]acétate d'éthyle (2,3 g).

(iii) Chlorhydrate de [((2R)2-aminométhyl-1,2,3,4-tétrahydro-7-naphtyl)oxy]acétate d'éthyle.

On ajoute une solution éthanolique 7,2N de HCl gazeux (5 ml) à une solution du produit obtenu dans l'étape (ii) ci-dessus (2,3 g, 0,0063 mole) dans de l'éthanol absolu (15 ml) et après on chauffe le mélange à 50°C pendant 30 minutes. On concentre sous pression réduite et on lave le résidu avec une petite quantité d'acétone en obtenant le composé indiqué dans le titre (1,4 g).

(T) Chlorhydrate de [(2(S)-aminométhyl-1,2,3,4-tétrahydronapht-7-yl)oxy]acétate d'éthyle.

(i) 7-hydroxy-2(S)-(tertbutoxycarbonyl)aminométhyl-1,2,3,4-tétrahydronaphtalène.

Suivant le mode opératoire de la Préparation (S)(i) ci-dessus, mais à partir du composé de la Préparation (I) (2,1 g, 0,012 mole) on obtient le produit indiqué ci-dessus (2,2 g).

(ii) [2(S)2-tertbutoxycarbonylaminométhyl-1,2,3,4-tétra-hydro-7-naphtyl)oxy]acétate d'éthyle.

En suivant la procédure de la Préparation (S)(ii) mais à partir du composé obtenu dans l'étape (i) ci-dessus (2,2 g, 0,008 mole) on obtient le produit indiqué ci-dessus (1,4 g).

(iii) Chlorhydrate de [((2S)2-aminométhyl-1,2,3,4-tétrahydro-7-naphtyl)oxy]acétate d'éthyle.

En suivant la procédure de la Préparation (S)(iii) mais à partir du composé obtenu dans l'étape (ii) ci-dessus on obtient le produit indiqué dans le titre (0,8 g).

(U) Chlorhydrate de [((2S)2-aminométhyl-1,2,3,4-tétrahydro-6-naphtyl)oxy]acétate d'éthyle et

(V) Chlorhydrate de [((2R)2-aminométhyl-1,2,3,4-tétrahydro-6-naphtyl)oxy]acétate d'éthyle.

En suivant la procédure de la Préparation (S), étapes (i), (ii) et (iii), mais à partir des composés des Préparations (P) et (R) on obtient les produits indiqués ci dessus avec des rendements de 15 à 20%.

Dans le cas de dérivés 6-substitués, l'attribution de la configuration (R) à l'entantiomère dextrogyre, et de la configuration (S) à l'enantiomère lévogyre, bien que vraisemblable, n'a pas été confirmée. En effet, au contraire de la série de dérivés 7-substitués, où on a pu aisément attribuer la configuration absolue au composé de départ de base (voir la Préparation (F)(i)) par comparaison avec des composés connus, dans la série des dérivés 6-substitués on n'a pas pu utiliser la même méthode car les produits optiquement actifs qui,par analogie, devraient être utilisés en tant que produits de référence, ne sont pas décrits en littérature.

EXEMPLE 1

Chlorhydrate de N-[(2(R)-7-hydroxy-1,2,3,4-tétrahydronapht-2-yl)-méthyl]-(2R)-2-hydroxy-2-(3-chlorophényl)éthanamine.

(i) N-[(2(R)-7-hydroxy-1,2,3,4-tétrahydronapht-2-yl)méthyl]-(R)-3-chloromandélamide.

On agite pendant 5 heures à température ambiante une suspension du produit obtenu dans la préparation (H) ci-dessus (2,2 g, 0,013 moles), d'acide (R)-3-chloromandélique (2,3 g, 0,013 moles), d'hexafluorophosphate de benzotriazolyl-N-oxytris-(diméthyl-amino)phosphonium (BOP - 5,2 g, 0,013 moles) dans du chlorure de méthylène anhydre (100 ml) et de triéthylamine (1,8 ml, 1,3 g, 0,013 moles). On ajoute de la saumure (50 ml) et on agite le mélange pendant 30 minutes. On sépare la phase organique et on la lave successivement avec de l'acide chlorhydrique 2N (2 x 30 ml), de l'eau, une solution aqueuse saturée de bicarbonate de sodium, et de l'eau, puis on la sèche et l'évapore à sec. On purifie le produit obtenu par flash chromatographie en utilisant un mélange acétate d'éthyle: cyclohexane 1:1 comme éluant. On sèche le produit huileux ainsi obtenu sous pression réduite à 40°C pendant 2 jours et on obtient l'amide indiqué ci-dessus (3,1 g) sous forme de poudre vitreuse; [alpha] = + 31,6° (méthanol, c = 1%).

(ii) Chlorhydrate de N-[(2(R)-7-hydroxy-1,2,3,4-tétrahydronapht-2-yl)méthyl]-(2R)-2-hydroxy-2-(3-chlorophényl)éthanamine.

On chauffe au reflux une solution du produit obtenu dans l'étape (i) ci-dessus (2,7 g, 0,008 moles) dans du tétrahydrofuranne anhydre (50 ml) sous atmosphère d'azote et on y ajoute goutte à goutte une solution de borane-méthylsulfure 10 M (2,4 ml, 0,024 moles) dans du tétrahydrofuranne anhydre (20 ml). On chauffe le mélange réactionnel ainsi obtenu au reflux pendant 4 heures, on le refroidit et on y ajoute du méthanol (20 ml) goutte à goutte. On chauffe le mélange au reflux pendant 30 minutes, on l'évapore à sec et on purifie le produit ainsi obtenu sous forme de base libre par flash chromatographie en utilisant du méthanol comme éluant. On dissout la base ainsi obtenu dans de l'acétone (40 ml) et on rend la solution acide par de l'isopropanol saturé d'acide chlorhydrique. On obtient le composé indiqué dans le titre (1 g) qui est séché sous pression réduite à 40°C pendant 2 jours. P.f. = 145-148°C; [alpha] = + 34° (méthanol, c = 1%).

EXEMPLE 2

N-[(2(S)-7-hydroxy-1,2,3,4-tétrahydronapht-2-yl)méthyl]-(2R)-2-hydroxy-2-(3-chlorophényl)éthanamine.

(i) N-[(2(S)-7-hydroxy-1,2,3,4-tétrahydronapht-2-yl)méthyl]-(R)-3-chloromandélamide.

Suivant le mode opératoire de l'Exemple 1(i), mais en partant d'un mélange du produit de la Préparation (I) (3,0 g, 0,017 moles), d'acide (R)-3-chloromandélique (3,2 g, 0,017 moles), de BOP (6,8 g, 0,017 moles) et de triéthylamine (2,4 ml, 0,017 moles) dans du chlorure de méthylène anhydre (120 ml), on obtient l'amide (4 g) sous forme de poudre vitreuse. /alpha/ = -80,6° (méthanol, c = 1%).

(ii) N-[(2(S)-7-hydroxy-1,2,3,4-tétrahydronapht-2-yl)-méthy]-(2 R)-2-hydroxy-2-(3-chlorophényl)éthanamine.

Suivant le mode opératoire de l'Exemple 1(ii), mais en partant de l'amide ci-dessus (3,6 g, 0,010 moles), on obtient le produit voulu sous forme de base libre qui est purifiée par flash chromatographie en utilisant un mélange méthanol: acétate d'éthyle 60:40 comme éluant et, ensuite, par cristallisation dans le méthanol. On obtient 1,9 g; p.f. 159-161°C. [alpha] = -77,2° (méthanol, c = 0,5 %).

EXEMPLE 3

Chlorhydrate de N-[(8-hydroxy-1,2,3,4-tétrahydronapht-2-yl)-méthyl]-2-hydroxy-2-(3-chlorophényl)éthanamine.

(i) N-[(8-hydroxy-1,2,3,4-tétrahydronapht-2-yl)méthyl]-3-chloromandélamide.

On agite pendant 5 heures à température ambiante un mélange du produit obtenu dans la Préparation (D) (2,2 g, 0,0085 mole), d'acide 3-chloromandélique (1,6 g, 0,0085 moles), de BOP (3,4 g, 0,0085 moles) et de triéthylamine (2,4 ml, 1,72 g, 0,017 moles) dans du chlorure de méthylène (50 ml). On dilue ledit mélange avec de l'acétate d'éthyle et, après, on le lave successivement à l'eau, à l'acide chlorhydrique dilué, à une solution aqueuse saturée de bicarbonate de sodium et à l'eau, puis on le sèche et évapore à sec. On obtient l'amide ci-dessus (1,6 g) ayant un spectre d'absorption IR qui correspond à la structure attribuée.

IR (KBr): 3342 (d): O-H, CON-H; 1641: HNC=O $cm^{-1}$.

(ii) Chlorhydrate de N-[(8-hydroxy-1,2,3,4-tétrahydronapht-2-yl)-méthyl]-2-hydroxy-2-(3-chlorophényl)éthanamine.

On chauffe au reflux une solution du produit de l'étape (i) ci-dessus (1,6 g, 0,0046 moles) dans du tétrahydrofuranne anhydre (30 ml) sous atmosphère d'azote et on y ajoute lentement un mélange d'une solution 10 M de borane-méthylsulfure (1,4 ml, 0,0014 moles) et de tétrahydrofuranne anhydre (10 ml). On chauffe la solution ainsi obtenue au reflux pendant 4 heures, puis on ajoute très lentement du méthanol (10 ml). On concentre sous vide, or dissout le résidu dans de l'éther éthylique, on rend la solution ainsi obtenue acide par de l'isopropanol saturé d'acide chlorhydrique et l'on obtient, par filtration, 0,9 g du composé indiqué dans le titre; p.f. 175-178°C.

EXEMPLES 4 à 9

Suivant les modes opératoires des exemples 1 à 3 ci-dessus, mais en partant de l'acide 3-chloromandélique et du dérivé 1,2,3,4-tétrahydronaphtalénique correspondant, on obtient les composés de formule I suivants par l'intermédiaire des composés de formule IV indiqués entre parenthèses et caractérisés par les maxima d'absorption au infrarouge indiqués.

Exemple 4

Chlorhydrate de N-[(7-méthoxy-1,2,3,4-tétrahydronapht-2-yl)-méthyl]-2-hydroxy-2-(3-chlorophényl)éthanamine. P.f.: 170-173°C (isopropanol) (N-[(7-méthoxy-1,2,3,4-tétrahydronapht-2-yl)méthyl]-3-chloromandélamide).

IR (liquide sans solvant): 3390 (sh), 3324: O-H, CON-H; 1655: NHC=O $cm^{-1}$.

Exemple 5

Chlorhydrate de N-[(6-méthoxy-1,2,3,4-tétrahydronapht-2-yl)-méthyl]-2-hydroxy-2-(3-chlorophényl)éthanamine. P.f.: 205-208°C (éthanol absolu)

(N-[(6-méthoxy-1,2,3,4-tétrahydronapht-2-yl)-méthyl] -3-chloromandélamide).

IR (KBr): 3520 (sh), 3300 (sh), 3247: O-H, CON-H; 1627, 1650: NHC=O $cm^{-1}$.

Exemple 6

Chlorhydrate de N-[(6-hydroxy-1,2,3,4-tétrahydronapht-2-yl)-méthyl]-2-hydroxy-2-(3-chlorophényl)éthanamine. P.f.: 174-176°C (isopropanol) (N-[(6-hydroxy-1,2,3,4-tétrahydronapht-2-yl)méthyl]-3-chloromandélamide).

IR (KBr): 3349 (b): O-H, CON-H; 1659: NHC=O $cm^{-1}$.

EXEMPLE 7

Chlorhydrate de N-[(5-hydroxy-1,2,3,4-tétrahydronapht-2-yl)-méthyl](-2-hydroxy-2-(3-chlorophényl)éthanamine. P.f. 177-180°C (trituré dans l'acétone)

(N-[(5-hydroxy-1,2,3,4-tétrahydronapht-2-yl)méthyl]-3-chloromandélamide).

IR (KBr): 3365 (b): O-H, CON-H; 1659: NHC-O $cm^{-1}$.

EXEMPLE 8

Chlorhydrate de N-[(8-méthoxy-1,2,3,4-tétrahydronapht-2-yl)-méthyl]-2-hydroxy-2-(3-chlorophényl)éthanamine, P.f. 186-188°C (isopropanol) (N-[(8-méthoxy-1,2,3,4-tétrahydronapht-2-yl)méthyl]-3-chloromandélamide).

IR (KBr): 3319 (d): O-H, CON-H; 1658: NHC=O $cm^{-1}$.

EXEMPLE 9

Chlorhydrate de N-[(5-méthoxy-1,2,3,4-tétrahydronapht-2-yl)-méthyl]-2-hydroxy-2-(3-chlorophényl)éthanamine. P.f. 216-218°C (éthanol à 95%).

(N-[(5-méthoxy-1,2,3,4- tétrahydronapht-2-yl)méthyl]-3-chloromandélamide, p.f. 92-95° C).

EXEMPLE 10

Oxalate de N-[(7-hydroxy-1,2,3,4-tétrahydronapht-2-yl)-méthyl]-2-hydroxy-2-(3-chlorophényl)éthanamine.

On prépare ce composé selon la procédure de l'exemple 1(i) et (ii), mais à partir du composé de la préparation (M) et en traitant le composé obtenu, sous forme de base libre avec, une solution d'acide oxalique dans l'acétone. P.f. 218-220°C (trituré dans l'acétone).

EXEMPLE 11

Chlorhydrate de [[2-[N-[2-(3-chlorophényl)-2-hydroxy]éthyl]-aminométhyl-1,2,3,4-tétrahydronapht-7-yl]oxy]acétate d'éthyle.

On chauffe à 80°C, pendant 8 heures et sous agitation, un mélange de produit de la préparation (K) sous forme de base libre (2,8 g, 0,010 moles), d'oxyde de 3-chlorostyrène (2,6 g, 0,015 moles) et de diméthylsulfoxyde anhydre (15 ml). On verse le mélange réactionnel dans de l'eau, on extrait la solution avec de l'acétate d'éthyle, on lave la phase organique à l'eau, on la sèche sur du sulfate de sodium, on la filtre et on l'évapore à sec. On dissout le résidu dans de l'isopropanol chaud (40 ml) et on y ajoute de l'isoproparol saturé d'acide chlorhydrique. Par filtration, on recueille le produit indiqué dans le titre (1,4 g) P.f. 157-161°C.

EXEMPLE 12

Chlorhydrate de 4-[[2-[N-[2-(3-chlorophényl)-2-hydroxy]-éthyl]-aminométhyl-1,2,3,4-tétrahydronapht-7-yl]oxy]butanoate d'éthyle.

En opérant selon le mode opératoire de l'Exemple 11, mais en utilisant le produit obtenu dans la préparation (L) on obtient le composé indiqué dans le titre; p.f. 138-145°C (isopropanol).

EXEMPLE 13

Chlorhydrate de N-[(7-méthoxy-1,2,3,4-tétrahydronapht-2-yl)-méthyl]-2-hydroxy-2-(3,4-dichlorophényl)éthanamine.

(i) N-[(7-méthoxy-1,2,3,4-tétrahydronapht-2-yl)méthyl]-3,4-dichloro-mandélamide.

Suivant le mode opératoire de l'Exemple 1(i), mais en partant de l'acide 3,4-dichloromandélique (3,1 g, 0,014 moles) et de la 2-aminométhyl-7-méthoxytétraline (2,67 g, 0,014 moles) on obtient le N-[(7-méthoxy-1,2,3,4-tétrahydronapht-2-yl)-méthyl]-3,4-dichloromandélamide (4,5 g) sous forme de produit huileux caractérisé par les maxima d'absorption infrarouge suivants: 3380 (b): O-H, CON-H; et 1641: NHC=O $cm^{-1}$.

(ii) Chlorhydrate de N-[(7-méthoxy-1,2,3,4-tétrahydronapht-2-yl)-méthyl]-2-hydroxy-2-(3,4-dichlorophényl)éthanamine.

On chauffe au reflux une solution du produit obtenu dans l'étape (i) ci-dessus (4,5 g, 0,0114 moles) dans du tétrahydrofuranne (75 ml) sous atmosphère d'azote et on y ajoute lentement un mélange d'une solution 10 M de borane-méthylsulfure (3,5 ml, 0,035 moles) et de tétrahydrofuranne (10 ml). On chauffe la solution ainsi obtenue au reflux pendant 4 heures, puis on ajoute très lentement du méthanol (25 ml). On concentre sous pression réduite, on dissout le résidu dans de l'isopropanol et on précipite le produit indiqué dans le titre par addition d'acide chlorhydrique dans l'isopropanol. On cristallise le produit ainsi obtenu dans l'éthanol et on obtient 1,87 g, p.f. 194-198°C.

EXEMPLE 14

Chlorhydrate de [[2-[[N-(2-phényl)2-hydroxy]éthyl]-aminométhyl-1,2,3,4-tétrahydronapht-7-yl]oxy]acétate d'éthyle.

Suivant le mode opératoire de l'Exemple 11, mais en remplaçant l'oxyde de 3-chlorostyrène par l'oxyde de styrène, on obtient le composé indiqué dans le titre; p.f. 163-170°C (isopropanol).

EXEMPLE 15

Chlorhydrate de N-[(7-méthoxy-1,2,3,4-tétrahydronapht-2-yl)- méthyl] 2-hydroxy-2-phényléthanamine.

On chauffe pendant 10 heures à 80°C (température externe) un mélange d'oxyde de styrène (1 g, 0,0083 moles), de 2-aminométhyl-7-méthoxy-1,2,3,4-tétrahydronaphtalène (1,5 g, 0,0078 moles) dans du diméthylsulfoxyde (20 ml). On verse le mélange réactionnel dans de l'eau et on extrait par de l'acétate d'éthyle. On lave la phase organique à l'eau, on la sèche et on évapore à sec. On obtient un résidu huileux et on le soumet à une flash chromatographie en éluant par un mélange chlorure de méthylène/méthanol 95:5. On évapore les fractions réunies, on dissout le résidu ainsi obtenu dans de l'éther éthylique et on y ajoute de l'isopropanol saturé d'acide chlorhydrique. On filtre le précipité et on obtient 0,5 g; p.f. 187-192°C.

EXEMPLE 16

Chlorhydrate de N-[(7-hydroxy-1,2,3,4-tétrahydronapht-2-yl)-méthyl]2-hydroxy-2-phényléthanamine.

Suivant le mode opératoire de l'Exemple 15, mais en remplaçant le 2-aminométhyl-7-méthoxy-1,2,3,4-tétrahydro-naphtalène par le composé de la Préparation (M), on obtient un produit qui lavé avec une petite quantité d'acétone donne le composé du titre. P.f. 155-158°C.

EXEMPLE 17

Chlorhydrate de N-[(1,2,3,4-tétrahydronapht-2-yl)méthyl]-2-hydroxy-2-(3-chlorophényl)éthanamine.

On chauffe à 80°C (température externe),pendant 8 heures, sous agitation un mélange de 2-aminométhyltétraline base (1,4 g, 0,0087 moles) obtenue par neutralisation de son chlorhydrate (Préparation (N)) par de l'hydroxyde de sodium et extraction par de l'acétate d'éthyle, et de l'oxyde de 3-chlorostyrène (2 g, 0,013 moles) dans du diméthylsulfoxyde (15 ml). On verse le mélange réactionnel dans de l'eau (environ 100 ml), on extrait par de l'éther éthylique. On lave la phase organique à l'eau, on la sèche et évapore à sec. On reprend le résidu dans de l'éther de pétrole et on le filtre. On dissout le produit ainsi obtenu (1,5 g) dans de l'acétone (50 ml) en chauffant faiblement et on y ajoute de l'isopropanol saturé d'acide chlorhydrique. On filtre le précipité et on obtient 1,5 g; p.f. 232-235°C.

EXEMPLE 18

Acide [[2-[N-[2-(3-chlorophényl)-2-hydroxy]éthyl]aminométhyl-1,2,3,4-tétrahydronapht-7-yl]oxy]acétique.

On chauffe à 50°C pendant 5 heures un mélange du produit de l'Exemple 11 sous forme de base libre (2,0 g, 0,0047 moles) de l'hydroxyde de potassium (0,3 g, 0,0057 moles) de l'éthanol à 95% (30 ml) et de l'eau (30 ml). On évapore l'éthanol, on y ajoute de l'eau (20 ml) et on extrait par de l'éther éthylique (2 x 50 ml). La phase organique est traitée avec du charbon, filtrée et acidifiée avec HCl 1N, jusqu'à pH 6,5. On filtre le précipité et on obtient le produit indiqué dans le titre. P.f. 213-217°C.

EXEMPLE 19

Chlorhydrate de N-[((2R)ou (2S)- 6-hydroxy-1,2,3,4-tétrahydronapht-2-yl)méthyl]-(2R)-2-hydroxy-2-(3-chlorophényl)éthanamine.

(i) N-/((2R) ou (2S) 6-hydroxy-1,2,3,4-tétrahydronapht-2-yl)méthyl]-(R)-3-chloromandélamide.

On agite à température ambiante pendant une nuit un mélange du composé de la Préparation (P) (0,6 g, 0,0023 mole), d'acide (R)-3-chloromandélique (0,4 g, 0,0023 mole), et de BOP (1 g, 0,0023 mole) dans du chlorure de méthyléne (20 ml) et de la triéthylamine (1,8 ml, 1,3 g, 0,013 mole). On y ajoute de l'acétate d'éthyle (60 ml) et on lave en séquence à une solution 2N d'acide chlorhydrique, à une solution saturée de bicarbonate de sodium et à l'eau. On sépare la phase organique, on la séche et on évapore à sec. On purifie le résidu par chromatographie en éluant avec un mélange acétate d'éthyle/cyclohexane 1/1 et en obtenant ainsi 0,7 g de l'amide indiqué ci-dessus. [alpha]= + 21,9° (c = 1%, MeOH).

(ii) Chlorhydrate de N-/((2R) ou (2S) 6-hydroxy-1,2,3,4-tétra hydrorapht-2-yl)méthyl] -(2R)-2-hydroxy-2-(3-chlorophényl)éthanamine.

On ajoute lentement une solution 10M de borane-méthyl sulfure (0,5 ml, 0,005 moles) dans du tétrahydrofuranne anhydre (5 ml) à une solution du composé obtenu à l'étape (i) ci-dessus (0,5 g, 0,0014 mole) dans du tétrahydrofuranne anhydre (20 ml) chauffé au reflux sous atmosphère d'azote. Le mélange réactionnel ainsi obtenu est chauffé au reflux pendant 4 heures, on y ajoute lentement du méthanol (5 ml) et après 30 minutes, on ajoute une solution d'acide chlorhydrique 1N (4ml). On chauffe le mélange au reflux pendant 30 minutes, on concentre sous vide, on rend le mélange basique par addition d'ammoniaque, et on l'extrait avec de l'acétate d'éthyle. On lave la phase organique à l'eau, on la séche et on l'évapore à sec. On dissout le residu dans de l'isopropanol chaud (10 ml), on acidifie par addition d'isopropanol saturé d'acide chlorhydrique et on récupère le produit ainsi obtenu par filtration (0,24 g). P.f. 175-77°C; [alpha] = + 18,3° (C = 1%, MeOH).

EXEMPLE 20

Chlorhydrate de N-[((2S) ou (2R) 6-hydroxy-1,2,3,4-tétrahydronapht-2-yl)méthyl]-(2R)-2-hydroxy-2-(3-chlorophényl)éthanamine.

(i) N-[((2S) ou (2R) 6-hydroxy-1,2,3,4-tétrahydronapht-2-yl)-méthy]-(R)-3-chloromandélamide.

En suivant la même procédure de l'Exemple 19 (i) mais à partir du composé de la Préparation (R) (0,5 g, 0,002 mole), de l'acide (R)-3-chloromandélique (0,4 g, 0,002 mole), et du BOP (0,88 g, 0,002 mole) dans du chlorure de méthylène (20 ml) et de la triéthylamine (0,6 ml, 0,4 g, 0,004 mole), on obtient 0,6 g de l'amide indiqué ci-dessus. [alpha] = -75,1° (c = 1%, MeOH).

(ii) Chlorhydrate de N-[((2S) ou (2R)6-hydroxy-1,2,3,4-tétrahydronapht-2-yl)méthyl]-(2R)-2-hydroxy-2-(3-chlorophényl)éthanamine.

En utilisant la procédure décrite dans l'Exemple 19 (ii) mais à partir du composé obtenu dans l'étape (i) ci-dessus (0,5 g, 0,0014 mole), on obtient 0,34 g du composé indiqué dans le titre. P.f. 217-219°C. [alpha] = -72,2°- (c = 1%, MeOH).

EXEMPLE 21

N-[(6-hydroxy-1,2,3,4-tétrahydronapht-2-yl)méthyl]-2-hydroxy-2-(4-chlorophényl)éthanamine.

(i) N-[(6-hydroxy-1,2,3,4-tétrahydronapht-2-yl)méthyl]-4-chloromandélamide.

On agite à température ambiante pendant une nuit un mélange du bromhydrate du composé de la Préparation (J) (3,77 g, 0,015 mole), de l'acide 4-chloromandélique (2,8 g, 0,015 mole), du BOP (6,63 g, 0,015 mole) et de la triéthylamine (3 g, 0,03 mole) dans du chlorure de méthylène (80 ml). On y ajoute de l'acétate d'éthyle, on lave en séquence avec de l'eau, une solution 2N d'acide chlorhydrique, une solution saturée de bicarbonate de sodium et de l'eau. On sèche et on concentre sous pression réduite en obtenant un produit huileux qui est purifié par chromatographie en éluant avec un mélange éthyl acétate/cyclohexane 1/1.

On obtient ainsi l'amide indiqué ci-dessus (3,47 g).

(ii) N-[(6-hydroxy-1,2,3,4-tétrahydronapht-2-yl)méthyl]-2-hydroxy-2-(4-chlorophényl)éthanamine.

On chauffe au reflux sous atmosphère d'azote une solution du composé obtenu à l'étape (i) ci-dessus (3,2 g, 0,0092 mole) dans du tétrahydrofuranne anhydre (65 ml) et on y ajoute une solution 10 M de borane-méthyl sulfure (2,8 ml, 0,028 mole) et du tétrahydrofuranne anhydre (10 ml). On chauffe au reflux pendant 4 heures, on y ajoute lentement du méthanol (30 ml) et on chauffe au reflux pendant une heure supplémentaire. On ajoute une solution 1N d'acide chlorhydrique (60 ml) et on chauffe au reflux pendant une heure. On concentre sous pression réduite, on reprend le résidu dans de l'acétate d'éthyle, on lave la solution organique à l'ammoniaque et après à l'eau, on sèche sur sulfate de sodium et on concentre sous pression réduite en obtenant un produit solide qui est lavé à l'éther isopropylique (2,3 g). P.f. 150-153°C.

EXEMPLE 22

Fumarate de N-[(7-hydroxy-1,2,3,4-tétrahydronapht-2-yl)-méthyl]-2-hydroxy-2-(2-chlorophényl)éthanamine.

(i) N-[(7-hydroxy-1,2,3,4-tétrahydronapht-2-yl)méthyl]-2-chloromandélamide.

En suivant la procédure de l'Exemple 19 (i) mais à partir du composé de la Préparation (M) (1,5 g, 0,0085 mole), de l'acide 2-chloromandélique (1,6 g, 0,0085 mole), du BOP (3,75 g, 0,0085 mole) et de la triéthylamine (0,86 g, 0,0085 mole) dans du chlorure de méthylène (55 ml), on obtient 2,2 g de l'amide indiquée ci-dessus.

(ii) Fumarate de N-[(7-hydroxy-1,2,3,4-tétrahydronapht-2-yl)-méthyl]-2-hydroxy-2-(2-chlorophényl)éthanamine.

En utilisant la procédure de l'Exemple 19 (ii) mais à partir du compose obtenu ci-dessus dans l'étape (i) (1,9 g, 0,0055 mole) et en utilisant de l'isopropanol contenant de l'acide fumarique au lieu d'isopropanol saturé d'acide chlorohydrique, on obtient 0,09 g du composé indiqué dans le titre. P.f. 215-217°C.

EXEMPLE 23

Hémifumarate de N-[(7-hydroxy-1,2,3,4-tétrahydronapht-2-yl)-méthyl]-2-hydroxy-2-(4-chlorophényl)éthanamine.

(i) N-[(7-hydroxy-1,2,3,4-tétrahydronapht-2-yl)méthyl]4-chloromandélamide.

En utilisant la procédure de l'Exemple 19 (i) mais en partant du composé de la Préparation (M) (2 g, 0,0113 mole), de l'acide 4-chloromandélique (2,1 g, 0,0113 mole) du BOP (5 g, 0,0113 mole) et de la triéthylamine (1,6 ml, 0,0113 mole) dans du chlorure de méthylène (60 ml) on obtient 2,2 g de l'amide indiqué ci-dessus.

(ii) Hémifumarate de N-[(7-hydroxy-1,2,3,4-tétrahydronapht-2-yl)-méthyl]-2-hydroxy-2-(4-chlorophenyl)éthanamine.

En suivant la procédure de l'Exemple 19 (ii) mais à partir de l'amide obtenu ci-dessus (2 g, 0,0058 mole) et en utilisant de l'isopropanol contenant de l'acide fumarique au lieu de l'isoproparol saturé d'acide chlorhydrique on obtient 0,18 g du composé indiqué dans le titre. P.f. 210-213°c.

EXEMPLE 24

Chlorohydrate de N-[(7-hydroxy-1,2,3,4-tétrahydronapht-2-yl)méthyl] -2-hydroxy-2-(3-éthoxyphényl)éthanamine.

(i) 3-méthoxystyrène oxyde

On agite pendant 17 heures un mélange de 3-méthoxybenzaldéhyde (13,4 g, 0,098 mole), une solution de NaOH (200 g) dans l'eau (200 ml), dodécyldiméthylsulfonium méthylsulfate (51 g, 0,15 mole) et toluène (150 ml). On y ajoute de la glace et on sépare la phase organique. On la lave à l'eau (3x50 ml), on la sèche sur sulfate de sodium, on filtre et on concentre sous pression réduite. On récupère du résidu le produit indiqué ci-dessus par distillation à 135-140°C et 30 mmHg.

(ii) Chlorhydrate de N-[(7-hydroxy-1,2,3,4-tétrahydronapht-2-yl)-méthyl]-2-hydroxy-2-(3-méthoxyphenyl)éthanamine.

On chauffe au reflux pendant une nuit un mélange du produit obtenu à l'étape (i) ci-dessus qui a un titre chromatographique de 71,5% (1,2 g, 0,0059 mole) et du composé de la Préparation (M) (1,4 g, 0,0079 mole) dans de l'éthanol absolu (60 ml). On concentre sous pression réduite et on purifie le produit huileux ainsi obtenu par chromatographie en éluant avec de l'acétate d'éthyle.

On dissout le produit ainsi obtenu dans l'éther éthylique et on y ajoute de l'isopropanol saturé d'acide chlorhydrique en obtenant 0,36 g du produit indiqué dans le titre.

EXEMPLE 25

Chlorhydrate de N-[(6-hydroxy-1,2,3,4-tétrahydronapht-2-yl)-méthyl]-2-hydroxy-2-(3-méthoxyphenyl)éthanamine.

En suivant la même procédure de l'Exemple 24 (ii) mais en remplaçant le composé de la Préparation (M) par le composé de la Préparation (J) (1,9 g) on obtient le composé indiqué dans le titre (0,62 g).

EXEMPLE 26

Chlorhydrate de N-[((2R) 7-méthoxy-1,2,3,4-tétrahydronapht-2-yl)-méthyl]-(2R)-2-hydroxy-2-(3-chlorophényl)éthanamine.

On chauffe à 60°C (température externe), pendant 48 heures, un mélange du chlorhydrate de 2(R)-aminométhyl-7-méthoxy-1,2,3,4-tétrahydronaphtalène (0,11 g, 0,48 mmole) (Préparation (G)), (R)-3-chlorostyrène oxyde (0,07 g, 0,46 mmole) et triéthylamine (0,13 ml, 0,96 mmole) dans du dyméthylsulfoxyde (5 ml). On verse le mélange réactionnel dans de l'eau et on extrait à l'acétate d'éthyle. On lave l'extrait organique à l'eau, on le sèche sur sulfate de sodium, on filtre et on évapore à sec. On soumet le résidu à une chromatographie flash en éluant par du chlorure de méthylene. On dissout le résidu qu'on obtient par évaporation des fractions réunies, dans de l'acétone et on y ajoute de l'isopropanol saturé d'acide chlorhydrique. On filtre le précipité et on obtient 0,02 g du produit indiqué dans le titre. P.f. 214-216°C, [alpha] = +25,1° (c = 1%, MeOH).

EXEMPLE 27

Chlorhydrate de N-[((2S)7-méthoxy-1,2,3,4-tétrahydronapht-2-yl)-méthyl]-(2R)-2-hydroxy-2-(3-chlorophényl)éthanamine.

En utilisant la procédure de l'Exemple 26 mais à partir du chlorhydrate de 2(S)-aminométhyl-7-méthoxy-1,2,3,4-tétrahydronaphtalène (0,11 g, 0,48 mmole), (R)-3-chlorostyrène oxyde (0,07 g, 0,46 mmole) et triéthylamine (0,13 ml, 0,96 mmole) dans du diméthylsulfoxyde (5 ml) on obtient 0,02 g du produit indiqué dans le titre. P.f. 189-191°C; /alpha/ = -70,7° (c = 1%, MeOH).

EXEMPLE 28

Chlorhydrate de N-[((2R) ou (2S) 6-méthoxy-1,2,3,4-tétrahydronapht-2-yl)méthyl]-(2R)-2-hydroxy-2-(3-chlorophényl)éthanamine.

En suivant la procédure d'ouverture de l'époxyde chiral décrite dans l'Exemple 26 mais à partir de 2(R) (ou 2(S))-aminométhyl-6-méthoxy-1,2,3,4-tétrahydronaphtalène (0,63 g, 0,0033 mole) obtenu par neutralisation de son chlorhydrate décrit dans la Préparation (O) et du (R)-3-chlorostyrène oxyde (0,6 g, 0,0039 mole) dans du diméthylsulfoxyde (10 ml) on obtient 0,5 g du produit indiqué dans le titre.

EXEMPLE 29

Chlorhydrate de N-[((2S) ou (2R) 6-méthoxy-1,2,3,4-tétrahydronapht-2-yl)-méthyl]-(2R)-2-hydroxy-2-(3-chlorophényl)- éthanamine.

En utilisant la même procédure de l'Exemple 26 mais à partir de 2(S) (ou 2(R))-aminométhyl-6-méthoxy-1,2,3,4-tétrahydronaphtalène (0,5 g, 0,0026 mole) obtenu par neutralisation de son chlorhydrate décrit dans la Préparation (Q) et du (R)-3-chlorostyrène oxyde (0,6 g, 0,0039 mole) dans du diméthylsulfoxyde (10 ml), on obtient 0,4 g du produit indiqué dans le titre.

EXEMPLE 30

Chlorhydrate de[[2(R)-[N-[2-(3-chlorophényl)-2(R)-hydroxy]éthyl] aminométhyl-1,2,3,4-tétrahydronapht-7-yl]oxy]acétate d'éthyle.

On chauffe à 80°C (température externe), pendant 10 heures et sous agitation, un mélange de [[2(R)-aminométhyl-1,2,3,4-tétrahydronapht-7-yl]oxy]-acétate d'éthyle (1 g, 0,0038 mole) obtenu par neutralisation de son chlorhydrate décrit dans la Préparation (S), de (R)-3-chlorostyrène oxyde (0,8 g, 0,0052 mole) dans du diméthylsulfoxyde anhydre (15 ml). On verse le mélange réactionnel dans l'eau, on'extrait la solution à l'acétate d'éthyle, on lave la solution organique à l'eau et après séchage sur $Na_2SO_4$ et filtration, on évapore à sec. On dissout le résidu dans de l'isopropanol à chaud, et on précipite le produit indiqué dans le titre par addition d'isopropanol saturé d'acide chlorhydrique (0,6 g).

EXEMPLE 31

Chlorhydrate du [[2(S)-[N-[2-(3-chlorophenyl)-2(R)-hydroxy]éthyl]-aminométhyl-1,2,3,4-tétrahydronapht-7-yl]oxy]acétate d'éthyle.

En suivant la procédure de l'Exemple 30 mais en remplaçant le [[2(R)-aminométhyl-1,2,3,4-tétrahydronapht-7-yl]oxy]acétate d'éthyle par l'énantiomère (S) (1 g, 0,0038 mole) obtenu par neutralisation du chlorhydrate décrit dans la Préparation (T), on obtient 0,5 g du composé indiqué dans le titre.

EXEMPLE 32

Chlorhydrate du [[2(R) (ou 2(S))-[N-[2-(3-chlorophényl)-2(R)-hydroxy]éthyl] aminométhyl-1,2,3,4-tétrahydronapht-6-yl]oxy]acétate d'éthyle.

En utilisant la procédure de l'Exemple 30, mais à partir d'un mélange de [[2(R)(ou 2(S))-aminométhyl-1,2,3,4-tétrahydronapht-6-yl]-oxy]acétate d'éthyle (2,2 g, 0,0083 mole) obtenu par neutralisaton du chlorhydrate décrit dans la Préparation (U), et de (R)-3-chlorostyrène oxyde (1,8 g, 0,012 mole) dans du diméthylsulfoxyde anhydre (20ml) on obtient 1,2 g du produit indiqué dans le titre.

EXEMPLE 33

Chlorhydrate du [[2(S) (ou 2(R))-[N-[2-(3-chlorophényl)-2(R)-hydroxy]éthyl]aminométhyl-1,2,3,4-tétrahydronapht-6-yl]oxy]acétate d'éthyle.

En suivant la procédure de l'Exemple 30, mais à partir d'un mélange de [[2(S) (ou 2(R))-aminométhyl-1,2,3,4-tétrahydronapht-6-yl]oxy]acétate d'éthyle (1,8 g, 0,0068 mole) obtenu par neutralisation du chlorhydrate correspondant de la Préparation (V), et de (R)-3-chlorostyrène oxyde (1,5 g, 0,0097 mole), dans du diméthylsulfoxyde anhydre (20 ml) on obtient 1,1 g du produit indiqué dans le titre.

EXEMPLE 34

On prépare des comprimés à base du composé de l'Exemple 6, ayant la composition suivante:

| | |
|---|---|
| Composé de l'Exemple 6 | 20 mg |
| cellulose microcristalline | 30 mg |
| amidon de mais séché | 30 mg |
| lactose | 100 mg |
| stéarate de magnésium | 5 mg |

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.

De la même façon on prépare des comprimés contenant 40 mg d'ingrédient actif.

EXEMPLE 35

En opérant comme décrit dans l'Exemple 34, mais en utilisant le composé de l'Exemple 7, on prépare des comprimés ayant la composition suivante:

| | |
|---|---|
| Composé de l'Exemple 7 | 50,0 mg |
| amidon de mais séché | 100,0 mg |
| lactose | 95,0 mg |
| talc | 4,5 mg |
| stéarate de magnésium | 0,5 mg |

EXEMPLE 36

10.000 gélules avec un teneur en substance active de 50 mg sont préparées à partir des constituants suivants: 500 g du composé de l'Exemple 4, 495 g de cellulose microcristalline, 5 g de gel de silice amorphe. Les constituants ci-dessus sont bien mélangés et introduits dans des gélules de gélatine dure de dimension 4.

EXEMPLE 37

On prépare une solution aqueuse stérile appropriée pour une utilisation parentérale en ampoules à base du composé de l'Exemple 6 ayant la composition suivante:

| | | |
|---|---|---|
| composé de l'Exemple 6 | | 30 mg |
| chlorure de sodium | | 5 mg |
| eau distillée | q.s.p. | 2 ml |

EXEMPLE 38

On prépare une solution ophtalmique en mélangeant les constituants suivants dans les conditions habituelles:

| | | |
|---|---|---|
| composé de l'Exemple 4 | | 1,0 mg |
| $NaH_2PO_4$ | | 10,4 mg |
| $Na_2HPO_4$ | | 2,4 mg |
| Chlorobutanol | | 5,0 mg |
| Hydroxypropylméthylcellulose | | 5,0 mg |
| NaOH 1N | q.s.p. | pH = 7,4 |
| eau distillée | q.s.p. | 1,0 ml |

## Revendications

**Revendications pour les Etats contractants suivants : DE, GB, FR, IT, NL, SE, CH, LI, BE, AT, LU, DK**

**1.** Une phényléthanolaminométhyltétraline de formule (I) suivante

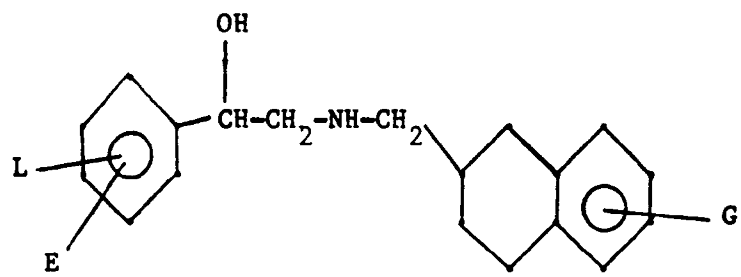

(I)

dans laquelle

- E représente l'hydrogène, un groupe ($C_1$-$C_4$)alkyle, un groupe ($C_1$-$C_4$) alcoxy, un groupe phényle, un groupe nitro, un atome d'halogène, ou un groupe trifluorométhyle,
- L représente l'hydrogène, un groupe ($C_1$-$C_4$)alkyle, phényle, ($C_1$-$C_4$)alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

ou un de ses sels.

**2.** Un composé selon la revendication 1 où G représente l'hydrogène, un groupe hydroxyle ou un groupe OG', où G' représente un groupe ($C_1$-$C_4$)alkyle substitué ou non.

**3.** Un composé selon la revendication 2 où G représente l'hydrogène, un groupe hydroxyle ou un groupe OG', où G' représente un groupe ($C_1$-$C_4$)alkyle non substitué ou substitué par un groupe carbo($C_1$-$C_4$)alkoxy ou carboxy.

**4.** Un composé selon l'une quelconque des revendications 1 à 3 ayant la configuration absolue R à l'atome de carbone chiral de la chaîne éthanolaminique.

**5.** Procédé pour la préparation d'un composé de formule (I) suivante

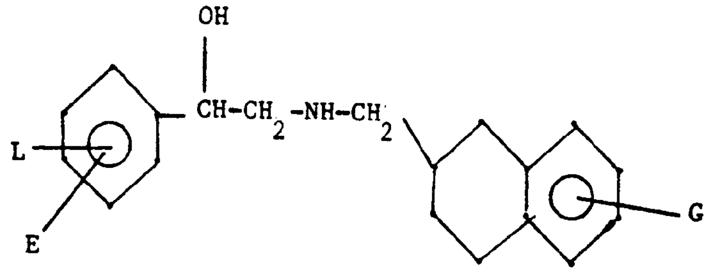

(I)

dans laquelle

- E représente l'hydrogène, un groupe $(C_1\text{-}C_4)$alkyle, un groupe $(C_1\text{-}C_4)$ alcoxy, un groupe phényle, un groupe nitro, un atome d'halogène, ou un groupe trifluorométhyle,
- L représente l'hydrogène, un groupe $(C_1\text{-}C_4)$alkyle, phényle, $(C_1\text{-}C_4)$alcoxy, un groupe nitro ou un atome d'halogène, ou
- E et L, ensemble, représentent un groupe -CH=CH-CH=CH- ou -CH2-CH2-CH2-CH2-, et
- G représente un atome d'hydrogène, un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle; ou un groupe $(C_2\text{-}C_4)$alcanoyle,

ou d'un de ses sels, caractérisé en ce qu'on fait réagir un composé de formule (II)

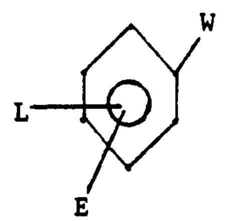

(II)

dans laquelle
- E et L ont les significations données ci-dessus et le radical -W représente un des groupes suivants (a) à (d)

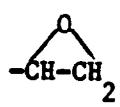 , 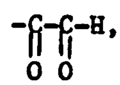 , 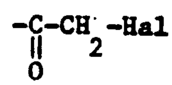 ou 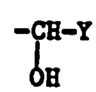

(a) (b) (c) (d)

où Hal représente le chlore, le brome ou l'iode et Y représente un groupe -COOH ou un dérivé fonctionnel de celui-ci,
avec un composé de formule (III)

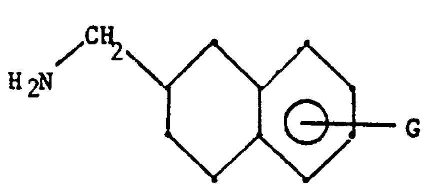 ,

(III)

dans laquelle G a la signification donnée ci-dessus, et, quand -W est autre que

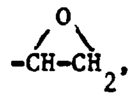

on traite le produit de la réaction avec un agent réducteur approprié et éventuellement on transforme le composé de formule (I) ainsi obtenu dans un de ses sels selon des méthodes par elles-même connues; ce procédé étant ultérieurement caractérisé en ce que

(i) lorsqu'on obtient un composé de formule (I) dans laquelle G est un groupe hydroxy, on peut convertir ce composé en les composés correspondants où G est un groupe OG', par réaction avec un agent d'alkylation ou d'acylation de formule G'-D dans laquelle G' est comme défini ci-dessus et D est un groupe facilement éliminable, après ou non protection temporaire du groupe amino,

(ii) lorsqu'on obtient un composé de formule (I) dans laquelle G est un groupe OG' où G' est un groupe alkyle substitué par un groupe ($C_1$-$C_4$)-alcoxycarbonyle, on peut convertir ce composé en le composé correspondant, où G' est un groupe alkyle substitué par carboxy, par hydrolyse basique,

(iii) lorsqu'on obtient un composé de formule (I) dans laquelle G est hydroxy on peut convertir ce composé en les composés correspondants, où G est un groupe de formule OG' dans laquelle G' est 1-méthyl-1-($C_1$-$C_4$)alcoxycarbonyl-éthyle ou 1-éthyl-1-($C_1$-$C_4$)alcoxycarbonyl-éthyle par traitement avec un composé de formule

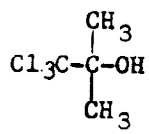

ou

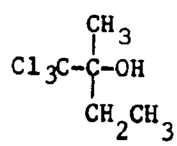

respectivement, en présence d'une base, suivi d'une réaction avec du chlorure de thionyle dans un ($C_1$-$C_4$)alcanol, et

(v) lorsqu'on obtient un composé de formule (I) sous forme d'un mélange d'isomères, on peut les séparer en les isomères purs ou en les couples d'énantiomères selon des méthodes conventionnelles.

**6.** Un composé de formule (IV) suivante

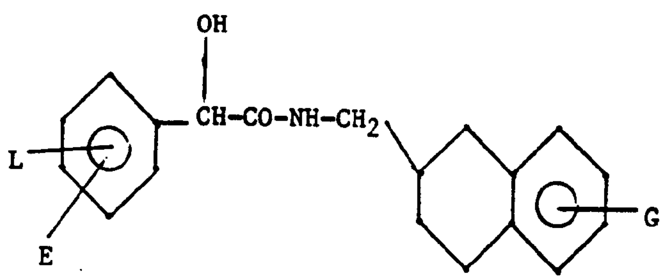

(IV)

dans laquelle

- E représente l'hydrogène, un groupe ($C_1$-$C_4$)alkyle, un groupe ($C_1$-$C_4$) alcoxy, un groupe phényle, un groupe nitro, un atome d'halogène, ou un groupe trifluorométhyle,
- L représente l'hydrogène, un groupe ($C_1$-$C_4$)alkyle, phényle, ($C_1$-$C_4$)alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe OG' où G' représente un groupe ($C_1$-$C_4$)alkyle non substitué ou subtitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

ou un de ses sels.

**7.** Composé selon la revendication 6 caractérisé en ce que G est un atome d'hydrogène, un groupe hydroxy ou un groupe OG', où G' est un groupe ($C_1$-$C_4$)alkyle non substitué ou ($C_3$-$C_7$)cycloalkyle.

**8.** Procédé pour la préparation d'un composé de formule (IV) suivante

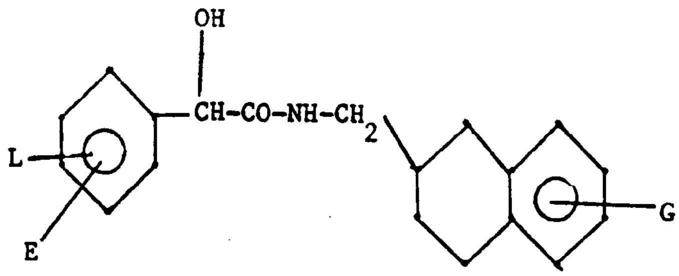

(IV)

dans laquelle

- E représente l'hydrogène, un groupe ($C_1$-$C_4$)alkyle, un groupe ($C_1$-$C_4$)alcoxy, un groupe phényle, un groupe nitro, un atome d'halogène, ou un groupe trifluorométhyle,
- L représente l'hydrogène, un groupe ($C_1$-$C_4$)alkyle, phényle ($C_1$-$C_4$)alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe OG'où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$) alcanoyle,

ou d'un de ses sels, caractérisé en ce qu'on fait réagir un composé de formule

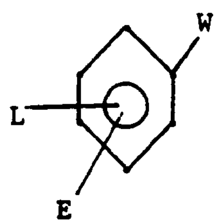

(II)

dans laquelle E et L ont les significations données ci-dessus et W représente un groupe

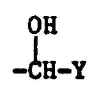

(d)

où Y représente un groupe -COOH ou un dérivé fonctionnel de celui-ci, avec un composé de formule (III)

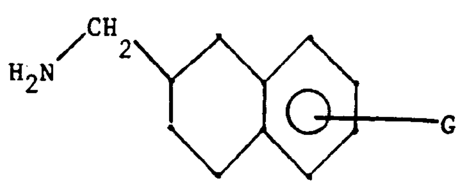

(III)

dans laquelle G a la signification donnée ci-dessus.

**9.** Un composé de formule (III)

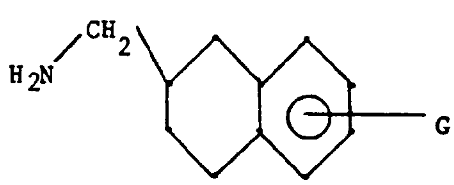

(III)

dans laquelle G est un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle; ou un groupe $(C_2\text{-}C_4)$alcanoyle, mais G est autre qu'un groupe $-OCH_3$ en position 7 ou 8, ou un de ses sels.

**10.** Un composé selon la revendication 9 caractérisé en ce que G est un groupe hydroxy ou un groupe OG' où G' représente un groupe $(C_1\text{-}C_4)$alkyle substitué par un groupe carboxy ou $(C_1\text{-}C_4)$alcoxycarbonyle.

**11.** Un composé de formule (III)

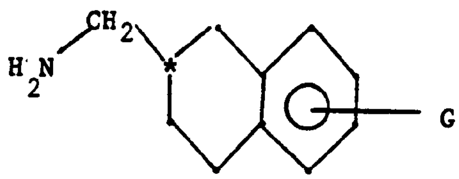

(III)

dans laquelle G est l'hydrogène, un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle; ou un groupe $(C_2\text{-}C_4)$alcanoyle, sous forme stéréoisomèrique pure, ou un de ses sels.

**12.** Procédé pour la préparation d'un composé de formule (III)

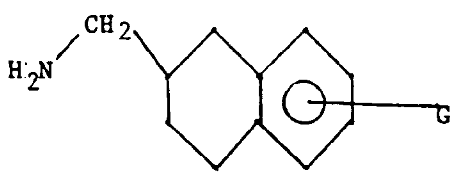

(III)

dans laquelle G est l'hydrogène, un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle, ou un groupe $(C_2\text{-}C_4)$alcanoyle, mais autre qu'un groupe $-OCH_3$ en position 7 ou 8, ou d'un de ses sels-, caractérisé en ce qu on réduit un composé de formule choisi entre

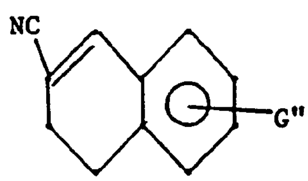

(VIII)

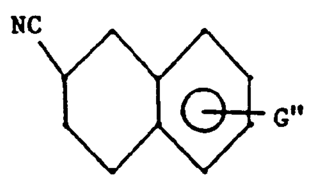

(VIII')

et

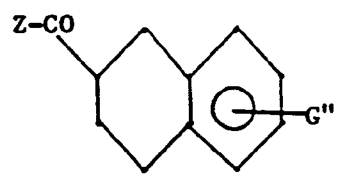

(X)

dans laquelle G" représente l'hydrogène, un atome de chlore, un groupe hydroxy ou un groupe méthoxy et Z est un groupe $-NH_2$, en obtenant un composé de formule (III) dans laquelle G = G", et on soumet le composé de formule (III) dans laquelle G" est l'hydroxyle aux procédures d'alkylation ou d'acylation conventionnelles avec ou sans protection préalable du groupe amino, ce procédé étant ultérieurement caractérisé en ce que lorsqu'on obtient un composé de formule (III) dans laquelle G = G" est un groupe méthoxy, on peut le convertir en le composé correspondant où G = G" est un groupe hydroxy par déméthylation avec de l'acide bromhydrique.

**13.** Un composé de formule

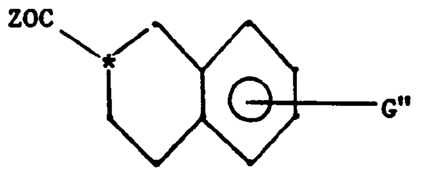

(X)

dans laquelle Z est un groupe -OH ou $-NH_2$ et G" représente l'hydrogène, un atome de chlore, un groupe hydroxy ou un groupe méthoxy, sous réserve que lorsque Z est un groupe -OH, G" soit différent de l'hydrogène, sous forme optiquement active, ou un de ses sels.

**14.** Composition pharmaceutique contenant, en tant que principe actif principal, un ou plusieurs composés selon la revendication 1.

**15.** Composition pharmaceutique selon la revendication 14 pour l'administration systémique sous forme d'unité de dosage, contenant de 0,1 à 500 mg de principe actif.

**16.** Composition pharmaceutique selon la revendication 14 ou 15 pour le traitement des troubles de la motricité intestinale.

**17.** Composition pharmaceutique selon la revendication 14, pour l'administration topique sur l'oeil.

**18.** Composition pharmaceutique selon la revendication 17 contenant de 10 ng à 1 mg pour chaque unité de dosage.

**19.** Composition pharmaceutique selon la revendication 17 ou 18 pour le traitement de l'hypertension oculaire et du glaucome.

**Revendications pour l'Etat contractant suivant : GR**

**1.** Une phényléthanolaminométhyltétraline de formule (I) suivante

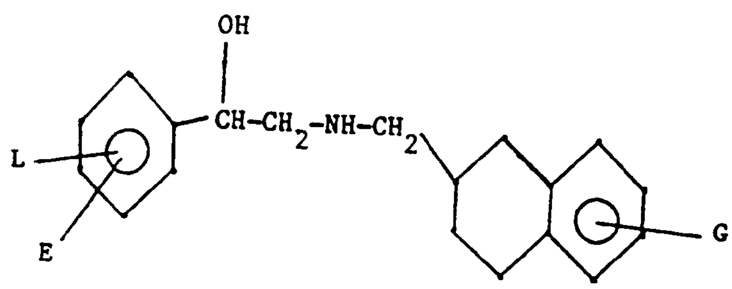

(I)

dans laquelle

- E représente l'hydrogène, un groupe $(C_1-C_4)$alkyle, un groupe $(C_1-C_4)$ alcoxy, un groupe phényle, un groupe nitro, un atome d'halogène, ou un groupe trifluorométhyle,
- L représente l'hydrogène, un groupe $(C_1-C_4)$alkyle, phényle, $(C_1-C_4)$alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe OG' où G' représente un groupe $(C_1-C_4)$alkyle non substitué ou substitué par un groupe hydroxy, $(C_1-C_4)$alcoxy, $(C_1-C_4)$alcoxycarbonyle, carboxy, ou $(C_3-C_7)$cycloalkyle; un groupe $(C_3-C_7)$cycloalkyle; ou un groupe $(C_2-C_4)$alcanoyle,

ou un de ses sels.

**2.** Un composé selon la revendication 1 où G représente l'hydrogène, un groupe hydroxyle ou un groupe OG', où G' représente un groupe $(C_1-C_4)$alkyle substitué ou non.

**3.** Un composé selon la revendication 2 où G représente l'hydrogène, un groupe hydroxyle ou un groupe OG', où G' représente un groupe $(C_1-C_4)$alkyle non substitué ou substitué par un groupe carbo$(C_1-C_4)$al-

koxy ou carboxy.

**4.** Un composé selon l'une quelconque des revendications 1 à 3 ayant la configuration absolue R à l'atome de carbone chiral de la chaîne éthanolaminique.

**5.** Procédé pour la préparation d'un composé de formule (I) suivante

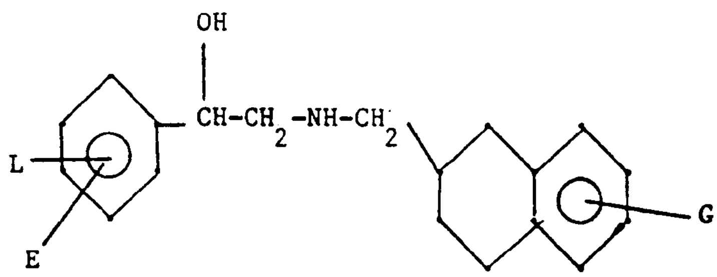

(I)

dans laquelle

- E représente l'hydrogène, un groupe ($C_1$-$C_4$)alkyle, un groupe ($C_1$-$C_4$) alcoxy, un groupe phényle, un groupe nitro, un atome d'halogène, ou un groupe trifluorométhyle,
- L représente l'hydrogène, un groupe ($C_1$-$C_4$)alkyle, phényle, ($C_1$-$C_4$)alcoxy, un groupe nitro ou un atome d'halogène, ou
- E et L, ensemble, représentent un groupe -CH=CH-CH=CH- ou -CH2-CH2-CH2-CH2-, et
- G représente un atome d'hydrogène, un atome de chlore, un groupe hydroxy ou un groupe OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

ou d'un de ses sels, caractérisé en ce qu'on fait réagir un composé de formule (II)

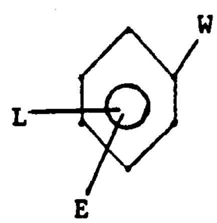

(II)

dans laquelle

- E et L ont les significations données ci-dessus et le radical -W représente un des groupes suivants (a) à (d)

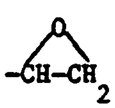 , 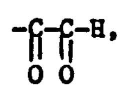 , 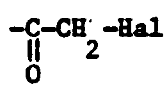 ou 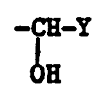

(a) (b) (c) (d)

où Hal représente le chlore, le brome ou l'iode et Y représente un groupe -COOH ou un dérivé fonctionnel de celui-ci,

avec un composé de formule (III)

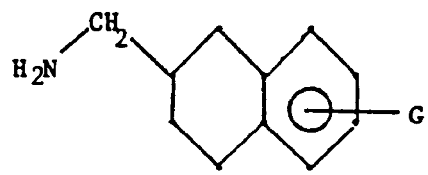 , (III)

dans laquelle G a la signification donnée ci-dessus, et, quand -W est autre que

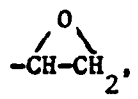

on traite le produit de la réaction avec un agent réducteur approprié et éventuellement on transforme le composé de formule (I) ainsi obtenu dans un de ses sels selon des méthodes par elles-même connues; ce procédé étant ultérieurement caractérisé en ce que

(i) lorsqu'on obtient un composé de formule (I) dans laquelle G est un groupe hydroxy, on peut convertir ce composé en les composés correspondants où G est un groupe OG', par réaction avec un agent d'alkylation ou d'acylation de formule G'-D dans laquelle G' est comme défini ci-dessus et D est un groupe facilement éliminable, après ou non protection temporaire du groupe amino,

(ii) lorsqu'on obtient un composé de formule (I) dans laquelle G est un groupe OG' où G' est un groupe alkyle substitué par un groupe ($C_1$-$C_4$)-alcoxycarbonyle, on peut convertir ce composé en le composé correspondant, où G' est un groupe alkyle substitué par carboxy, par hydrolyse basique,

(iii) lorsqu'on obtient un composé de formule (I) dans laquelle G est hydroxy on peut convertir ce composé en les composés correspondants, où G est un groupe de formule OG' dans laquelle G' est 1-méthyl-1-($C_1$-$C_4$)alcoxycarbonyl-éthyle ou 1-éthyl-1-($C_1$-$C_4$)alcoxycarbonyl-éthyle par traitement avec un composé de formule

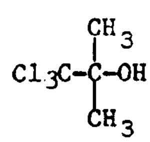 ou 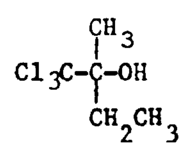

respectivement, en présence d'une base, suivi d'une réaction avec du chlorure de thionyle dans un ($C_1$-$C_4$)alcanol, et

(v) lorsqu'on obtient un composé de formule (I) sous forme d'un mélange d'isomères, on peut les séparer en les isomères purs ou en les couples d'énantiomères selon des méthodes conventionnelles.

**6.** Un composé de formule (IV) suivante

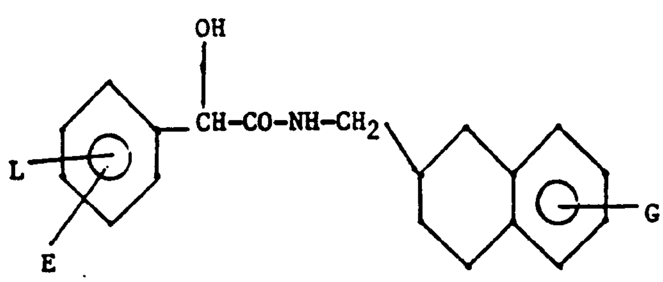 (IV)

dans laquelle

- E représente l'hydrogène, un groupe ($C_1$-$C_4$)alkyle, un groupe ($C_1$-$C_4$) alcoxy, un groupe phényle, un groupe nitro, un atome d'halogène, ou un groupe trifluorométhyle,
- L représente l'hydrogène, un groupe ($C_1$-$C_4$)alkyle, phényle, ($C_1$-$C_4$)alcoxy, un groupe nitro ou un atome d'halogène, ou
- E et L, ensemble, représentent un groupe $-CH=CH-CH=CH-$ ou $-CH_2-CH_2-CH_2-CH_2-$, et

- G représente un atome d'hydrogène, un atome de chlore, un groupe hydroxy ou un groupe OG' où G' représente un groupe ($C_1$-$C_4$)alkyle non substitué ou subtitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

ou un de ses sels.

**7.** Composé selon la revendication 6 caractérisé en ce que G est un atome d'hydrogène, un groupe hydroxy ou un groupe OG', où G' est un groupe ($C_1$-$C_4$)alkyle non substitué ou ($C_3$-$C_7$)cycloalkyle.

**8.** Procédé pour la préparation d'un composé de formule (IV) suivante

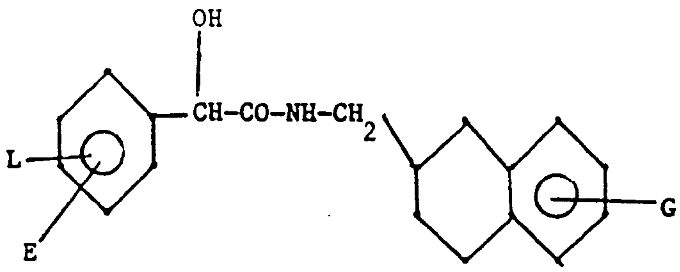

(IV)

dans laquelle

- E représente l'hydrogène, un groupe ($C_1$-$C_4$)alkyle, un groupe ($C_1$-$C_4$)alcoxy, un groupe phényle, un groupe nitro, un atome d'halogène, ou un groupe trifluorométhyle,
- L représente l'hydrogène, un groupe ($C_1$-$C_4$)alkyle, phényle ($C_1$-$C_4$)alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe OG'où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$) alcanoyle,

ou d'un de ses sels, caractérisé en ce qu'on fait réagir un composé de formule

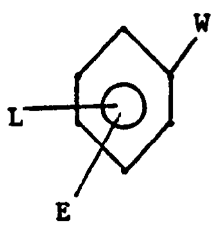

(II)

dans laquelle E et L ont les significations données ci-dessus et W représente un groupe

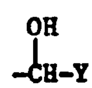

(d)

où Y représente un groupe -COOH ou un dérivé fonctionnel de celui-ci, avec un composé de formule (III)

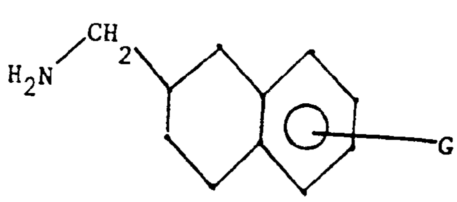

(III)

dans laquelle G a la signification donnée ci-dessus.

**9.** Un composé de formule (III)

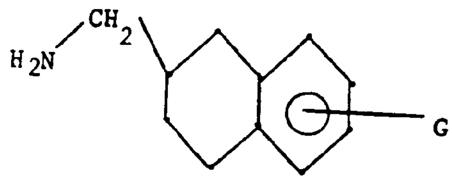

(III)

dans laquelle G est un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle; ou un groupe $(C_2\text{-}C_4)$alcanoyle, mais G est autre qu'un groupe $-OCH_3$ en position 7 ou 8, ou un de ses sels.

**10.** Un composé selon la revendication 9 caractérisé en ce que G est un groupe hydroxy ou un groupe OG' où G' représente un groupe $(C_1\text{-}C_4)$alkyle substitué par un groupe carboxy ou $(C_1\text{-}C_4)$alcoxycarbonyle.

**11.** Un composé de formule (III)

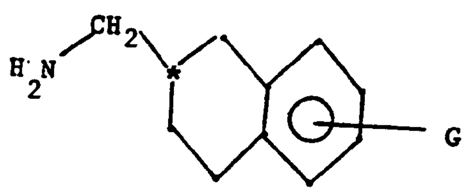

(III)

dans laquelle G est l'hydrogène, un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle; ou un groupe $(C_2\text{-}C_4)$alcanoyle, sous forme stéréoisomèrique pure, ou un de ses sels.

**12.** Procédé pour la préparation d'un composé de formule (III)

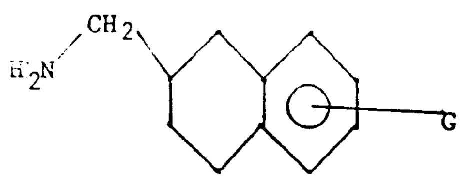

(III)

dans laquelle G est l'hydrogène, un atome de chlore, un groupe hydroxy ou un groupe OG' où G' représente un groupe $(C_1\text{-}C_4)$alkyle non substitué ou substitué par un groupe hydroxy, $(C_1\text{-}C_4)$alcoxy, $(C_1\text{-}C_4)$alcoxycarbonyle, carboxy, ou $(C_3\text{-}C_7)$cycloalkyle; un groupe $(C_3\text{-}C_7)$cycloalkyle; ou un groupe $(C_2\text{-}C_4)$alcanoyle, mais autre qu'un groupe $-OCH_3$ en position 7 ou 8, ou d'un de ses sels-, caractérisé en ce qu'on réduit un composé de formule choisi entre

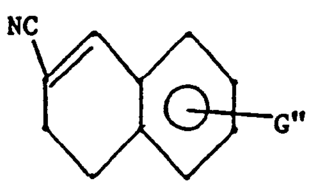

(VIII)

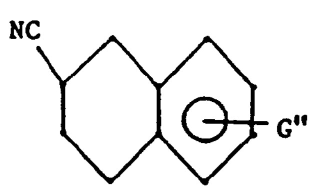

(VIII')

et

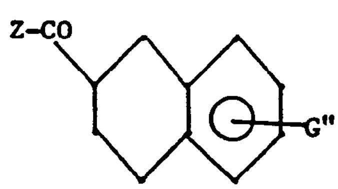

(X)

dans laquelle G" représente l'hydrogène, un atome de chlore, un groupe hydroxy ou un groupe méthoxy et Z est un groupe $-NH_2$, en obtenant un composé de formule (III) dans laquelle G = G", et on soumet le

composé de formule (III) dans laquelle G" est l'hydroxyle aux procédures d'alkylation ou d'acylation conventionnelles avec ou sans protection préalable du groupe amino, ce procédé étant ultérieurement caractérisé en ce que lorsqu'on obtient un composé de formule (III) dans laquelle G = G" est un groupe méthoxy, on peut le convertir en le composé correspondant où G = G" est un groupe hydroxy par déméthylation avec de l'acide bromhydrique.

13. Un composé de formule

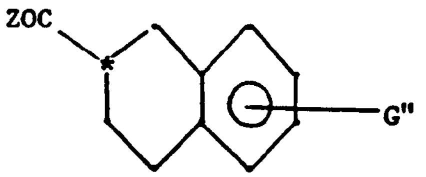

(X)

dans laquelle Z est un groupe -OH ou $-NH_2$ et G" représente l'hydrogène, un atome de chlore, un groupe hydroxy ou un groupe méthoxy, sous réserve que lorsque Z est un groupe -OH, G" soit différent de l'hydrogène, sous forme optiquement active, ou un de ses sels.

14. Composition pharmaceutique contenant, en tant que principe actif principal, un ou plusieurs composés selon la revendication 1.

15. Procédé pour la préparation d'une composition pharmaceutique, caractérisé en ce que l'on mélange, en tant que principe actif, un composé selon l'une quelconque des revendications 1 à 4 avec un véhicule pharmaceutiquement acceptable.

**Revendications pour l'Etat contractant suivant : ES**

1. Procédé pour la préparation d'un composé de formule (I) suivante

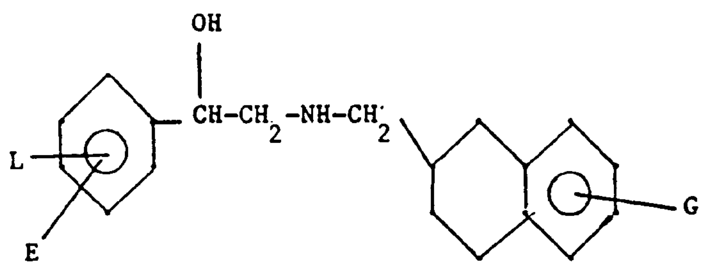

(I)

dans laquelle
- E représente l'hydrogène, un groupe ($C_1$-$C_4$)alkyle, un groupe ($C_1$-$C_4$)alcoxy, un groupe phényle, un groupe nitro, un atome d'halogène, ou un groupe trifluorométhyle,
- L représente l'hydrogène, un groupe ($C_1$-$C_4$)alkyle, phényle, ($C_1$-$C_4$)alcoxy, un groupe nitro ou un atome d'halogène, ou
- E et L, ensemble, représentent un groupe -CH=CH-CH=CH- ou -CH2-CH2-CH2-CH2-, et
- G représente un atome d'hydrogène, un atome de chlore, un groupe hydroxy ou un groupe OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

ou d'un de ses sels, caractérisé en ce qu'on fait réagir un composé de formule (II)

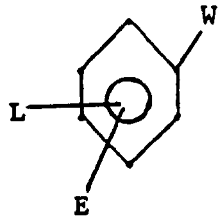

(II)

dans laquelle

- E et L ont les significations données ci-dessus et le radical -W représente un des groupes suivants (a) à (d)

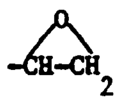 , 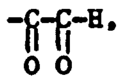 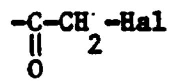 ou 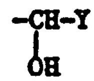

(a) (b) (c) (d)

où Hal représente le chlore, le brome ou l'iode et Y représente un groupe -COOH ou un dérivé fonctionnel de celui-ci,

avec un composé de formule (III)

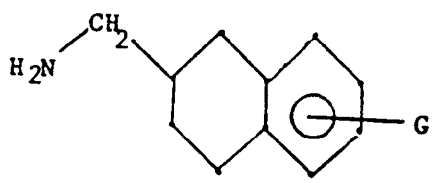

, (III)

dans laquelle G a!a signification donnée ci-dessus, et, quand -W est autre que

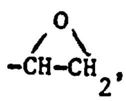

on traite le produit de la réaction avec un agent réducteur approprié et éventuellement on transforme le composé de formule (I) ainsi obtenu en l'un de ses sels selon des méthodes par elles-même connues; ce procédé étant ultérieurement caractérisé en ce que

(i) lorsqu'on obtient un composé de formule (I) dans laquelle G est un groupe hydroxy, on peut convertir ce composé en les composés correspondants où G est un groupe OG', par réaction avec un agent d'alkylation ou d'acylation de formule G'-D dans laquelle G' est comme défini ci-dessus et D est un groupe facilement éliminable, après ou non protection temporaire du groupe amino,

(ii) lorsqu'on obtient un composé de formule (I) dans laquelle G est un groupe OG' où G' est un groupe alkyle substitué par un groupe ($C_1$-$C_4$)-alcoxycarbonyle, on peut convertir ce composé en le composé correspondant, où G' est un groupe alkyle substitué par carboxy, par hydrolyse basique,

(iii) lorsqu'on obtient un composé de formule (I) dans laquelle G est hydroxy on peut convertir ce composé en les composés correspondants, où G est un groupe de formule OG' dans laquelle G' est 1-méthyl-1-($C_1$-$C_4$)alcoxycarbonyl-éthyle ou 1-éthyl-1-($C_1$-$C_4$)alcoxycarbonyl-éthyle par traitement avec un composé de formule

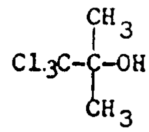 ou 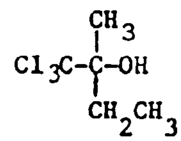

respectivement, en présence d'une base, suivi d'une réaction avec du chlorure de thionyle dans un ($C_1$-$C_4$)alcanol, et

(v) lorsqu'on obtient un composé de formule (I) sous forme d'un mélange d'isomères, on peut les séparer en les isomères purs ou en les couples d'énantiomères selon des méthodes conventionnelles.

2. Procédé selon la revendication 1 pour la préparation d'un composé de formule (I) dans laquelle G représente l'hydrogène, un groupe hydroxyle ou un groupe OG', où G' représente un groupe ($C_1$-$C_4$)alkyle substitué ou non.

3. Procédé selon la revendication 2 pour la préparation d'un composé de formule (I) dans laquelle G représente l'hydrogène, un groupe hydroxyle ou un groupe OG', où G' représente un groupe ($C_1$-$C_4$) alkyle non substitué ou substitué par un groupe carbo($C_1$-$C_4$) alkoxy ou carboxy.

4. Procédé selon l'une quelconque des revendications 1 à 3 pour la préparation d'un composé de formule (I) ayant la configuration absolue R à l'atome de carbone chiral de la chaîne éthanolaminique.

5. Procédé pour la préparation d'un composé de formule (IV)suivante

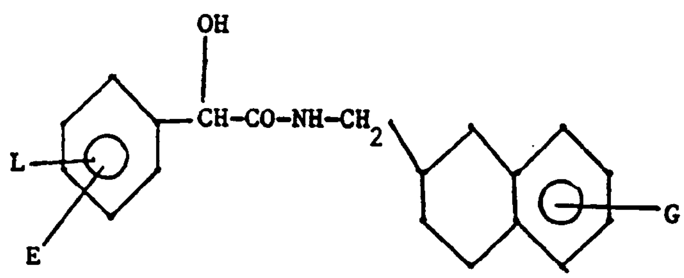

**(IV)**

dans laquelle

- E représente l'hydrogène, un groupe ($C_1$-$C_4$)alkyle, un groupe ($C_1$-$C_4$) alcoxy, un groupe phényle, un groupe nitro, un atome d'halogène, ou un groupe trifluorométhyle,
- L représente l'hydrogène, un groupe ($C_1$-$C_4$)alkyle, phényle, ($C_1$-$C_4$)alcoxy, un groupe nitro ou un atome d'halogène, ou
- E et L, ensemble, représentent un groupe -CH=CH-CH=CH- ou $-CH_2-CH_2-CH_2-CH_2-$, et
- G représente un atome d'hydrogène, un atome de chlore, un groupe hydroxy ou un groupe OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle,

ou d'un de ses sels, caractérisé en ce qu'on fait réagir un composé de formule (II)

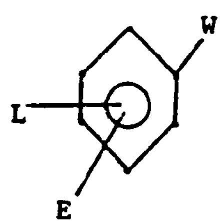

**(II)**

dans laquelle E et L ont les significations données ci-dessus et W représente un groupe

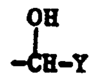 (d)

où Y représente un groupe -COOH ou un dérivé fonctionnel de celui-ci avec un composé de formule (III)

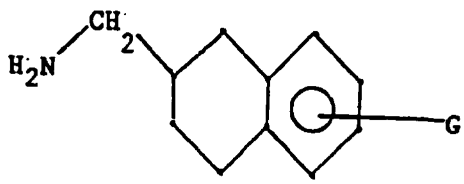 (III)

dans laquelle G a la signification donnée ci-dessus.

6. Procédé selon la revendication 5 pour la préparation d'un composé de formule (IV) dans laquelle G est un atome d'hydrogène un groupe hydroxy ou un groupe OG', où G' est un groupe ($C_1$-$C_4$) alkyle non substitué ou ($C_3$-$C_7$) cycloalkyle.

7. Procédé pour la préparation d'un composé de formule (III)

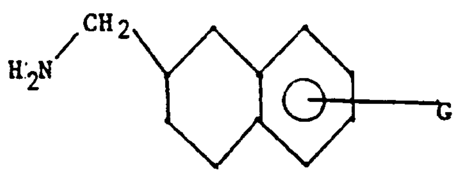 (III)

dans laquelle G est l'hydrogène, un atome de chlore, un groupe hydroxy ou un groupe OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle, mais autre qu'un groupe -$OCH_3$ en position 7 ou 8, ou d'un de ses sels-, caractérisé en ce qu'on réduit un composé de formule choisi entre

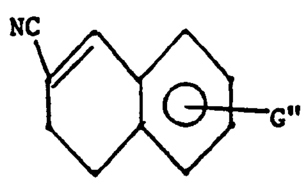
(VIII)

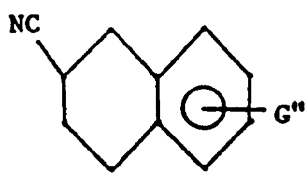
(VIII')

et

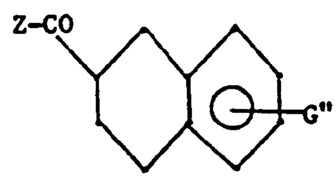
(X)

dans laquelle G" représente l'hydrogène, un atome de chlore, un groupe hydroxy ou un groupe méthoxy et Z est un groupe -$NH_2$, en obtenant un composé de formule (III) dans laquelle G = G", et on soumet le composé de formule (III) dans laquelle G" est l'hydroxyle aux procédures d'alkylation ou d'acylation conventionnelles avec ou sans protection préalable du groupe amino, ce procédé étant ultérieurement caractérisé en ce que lorsqu'on obtient un composé de formule (III) dans laquelle G = G" est un groupe méthoxy, on peut le convertir en le composé correspondant où G = G" est un groupe hydroxy par déméthylation avec de l'acide bromhydrique, le composé obtenu, lorsqu'il est sous forme d'un mélange d'isomères, pouvant être séparé en les isomères purs selon des méthodes conventionnelles.

8. Procédé selon la revendication 7 pour la préparation d'un composé de formule (III) dans laquelle G est un groupe hydroxy ou un groupe OG' où G' représente un groupe ($C_1$-$C_4$) alkyle substitué par un groupe carboxy ou ($C_1$-$C_4$) alcoxy-carbonyle.

9. Procédé selon la revendication 7 pour la préparation d'un composé de formule (III)

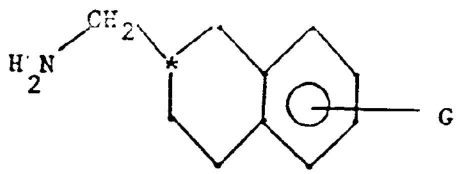

(III)

dans laquelle G est l'hydrogène, un atome de chlore, un groupe hydroxy ou un groupe OG' où G' représente un groupe ($C_1$-$C_4$)alkyle non substitué ou substitué par un groupe hydroxy, ($C_1$-$C_4$)alcoxy, ($C_1$-$C_4$)alcoxycarbonyle, carboxy, ou ($C_3$-$C_7$)cycloalkyle; un groupe ($C_3$-$C_7$)cycloalkyle; ou un groupe ($C_2$-$C_4$)alcanoyle, sous forme stéréoisomèrique pure, ou un de ses sels.

**10.** Procédé pour la préparation d'un composé de formule (X)

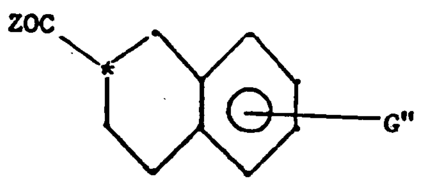

(X)

dans laquelle Z est un groupe -OH ou $-NH_2$ et G" représente l'hydrogène, un atome de chlore, un groupe hydroxy ou un groupe méthoxy, sous réserve que lorsque Z est un groupe -OH, G" soit différent de l'hydrogène, sous forme optiquement active, ou un de ses sels, caractérisé en ce qu'on traite un composé de formule (V)

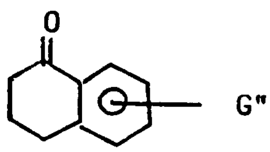

(V)

dans laquelle G" est tel que défini ci-dessus avec le diéthylcarbonate en présence de sodium afin d'obtenir un composé de formule (XI)

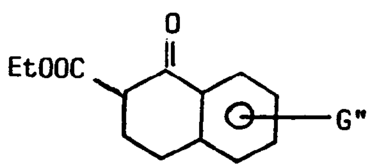

(XI)

dans laquelle G" est tel que défini ci-dessus, ce composé étant ensuite soumis à une réduction catalytique ou chimique, puis hydrolysé en milieu basique pour obtenir un composé de formule (X) ci-dessus, dans laquelle Z = OH et G" est tel que défini ci-dessus, ce composé étant éventuellement converti en amide correspondant par des méthodes conventionnelles pour obtenir un composé de formule (X) dans laquelle Z = $NH_2$, et éventuellement on transforme le composé de formule (X) ainsi obtenu en un de ses sels selon des méthodes connues.

**11.** Procédé pour la préparation d'une composition pharmaceutique, caractérisé en ce que l'on mélange, en tant que principe actif, un composé préparé selon le procédé de l'une quelconque des revendications 1 à 4 avec un véhicule pharmaceutiquement acceptable.

## Patentansprüche

### Patentansprüche für folgende Vertragsstaaten : DE, GB, FR, IT, NL, SE, CH, LI, BE, AT, LU, DK

1. Phenylethanolaminomethyltetralin der Formel (I)

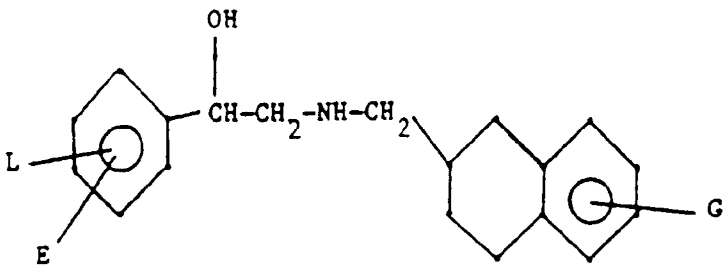

(I)

in der:
- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon.

2. Verbindung nach Anspruch 1, worin G Wasserstoff, eine Hydroxylgruppe oder eine OG'-Gruppe bedeutet, worin G' eine substituierte oder unsubstituierte ($C_1$-$C_4$)-Alkylgruppe bedeutet.

3. Verbindung nach Anspruch 2, worin G Wasserstoff, eine Hydroxylgruppe oder eine OG'-Gruppe bedeutet, worin G' eine ($C_1$-$C_4$)-Alkylgruppe bedeutet, die unsubstituiert oder durch eine Carbo-($C_1$-$C_4$)-alkoxy- oder Carboxygruppe substituiert ist.

4. Verbindung nach einem der Ansprüche 1 bis 3 mit der absoluten R-Konfiguration am chiralen Kohlenstoffatom der Ethanolaminkette.

5. Verfahren zur Herstellung der Verbindung der Formel (I)

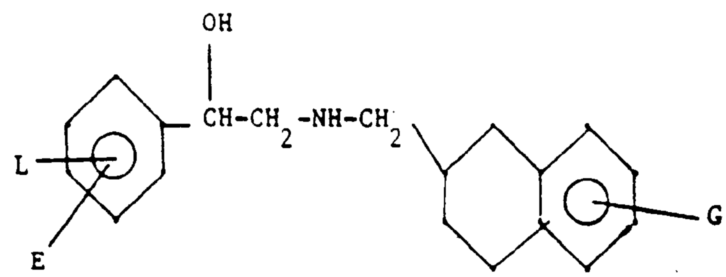

(I)

in der:
- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin

G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon,

dadurch gekennzeichnet, daß man eine Verbindung der Formel (II)

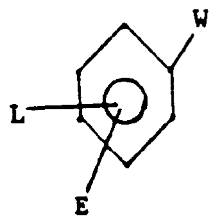

(II)

in der E und L die vorstehend angegebenen Bedeutungen besitzen und der Rest -W eine der folgenden Gruppen (a) bis (d) bedeutet

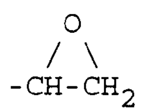 ,

(a)

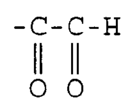 ,

(b)

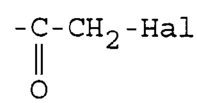

(c)

oder

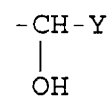

(d)

worin Hal Chlor, Brom oder Jod bedeutet und Y eine Gruppe - COOH oder ein funktionelles Derivat davon bedeutet, mit einer Verbindung der Formel (III)

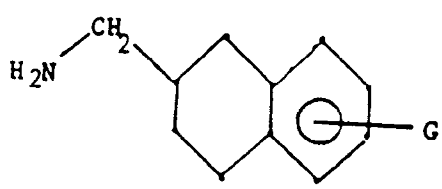

, (III)

in der G die vorstehend angegebene Bedeutung hat, umsetzt und, wenn -W eine von

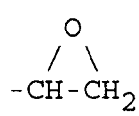 ,

abweichende Bedeutung hat, das Reaktionsprodukt mit einem geeigneten Reduktionsmittel umsetzt und ggf. die auf diese Weise erhaltene Verbindung der Formel (I) nach an sich bekannten Verfahren in ein Salz überführt;

wobei das Verfahren weiter dadurch gekennzeichnet ist, daß man,

(i) wenn man eine Verbindung der Formel (I), in der G eine Hydroxylgruppe bedeutet, erhält, diese Verbindung zu den entsprechenden Verbindungen, in denen G eine OG'-Gruppe bedeutet, umwandeln kann, indem man sie, ggf. nach einem vorübergehenden Schutz der Aminogruppe, mit einem Alkylierungs- oder Acylierungsmittel der Formel G'-D, in der G' die vorstehend definierte Bedeutung hat und D eine leicht entfernbare Gruppe bedeutet, umsetzt,

(ii) wenn man eine Verbindung der Formel (I), in der G eine OG'-Gruppe bedeutet, in der G' einen durch eine ($C_1$-$C_4$)-Alkoxycarbonylgruppe substituierte Alkylgruppe bedeutet, erhält, diese Verbindung durch basische Hydrolyse in die entsprechende Verbindung überführen kann, in der G' eine durch eine Carboxylgruppe substituierte Alkylgruppe bedeutet,

(iii) wenn man eine Verbindung der Formel (I), in der G eine Hydroxylgruppe bedeutet, erhält, diese Verbindung in die entsprechenden Verbindungen überführen kann, in denen G eine Gruppe der Formel OG' bedeutet, worin G' die Bedeutung 1-Methyl-1-($C_1$-$C_4$)-alkoxycarbonylethyl oder 1-Ethyl-1-($C_1$-

$C_4$)alkoxycarbonylethyl bedeutet, indem man eine Behandlung mit einer Verbindung der Formel

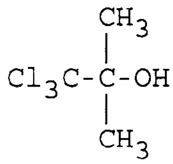

oder

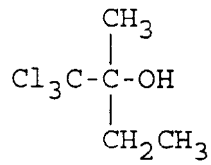

in Gegenwart einer Base durchführt, gefolgt von einer Umsetzung mit Thionylchlorid in einem ($C_1$-$C_4$)-Alkanol, und

(v) wenn man eine Verbindung der Formel (I) in Form eines Isomerengemisches erhält, diese Verbindungen nach herkömmlichen Verfahren in ihre reinen Isomeren oder in ihre Enantiomerenpaare auftrennen kann.

**6.** Verbindung der folgenden Formel (IV)

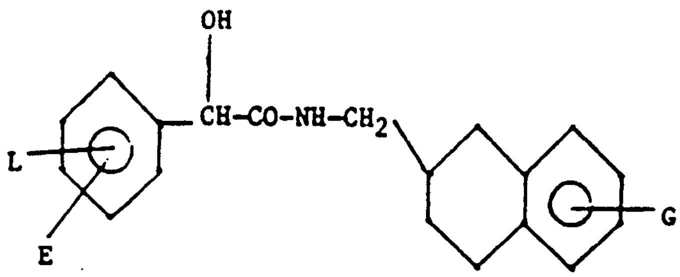

(IV)

in der:

- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon.

**7.** Verbindung nach Anspruch 6, dadurch gekennzeichnet, daß G ein Wasserstoffatom, eine Hydroxylgruppe oder eine OG'-Gruppe bedeutet, worin G' eine unsubstituierte ($C_1$-$C_4$)-Alkylgruppe oder ($C_3$-$C_7$)-Cyclolakylgruppe bedeutet.

**8.** Verfahren zur Herstellung einer Verbindung der Formel (IV)

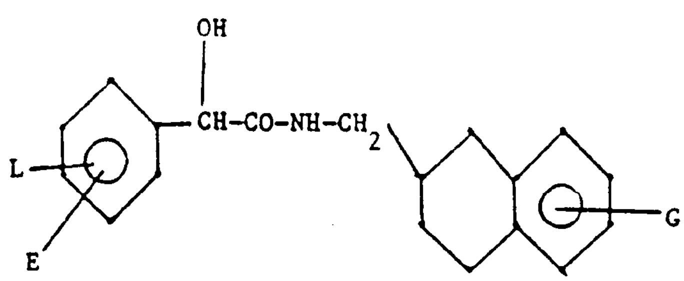

(IV)

in der:

- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,

- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon,

dadurch gekennzeichnet, daß man eine Verbindung der Formel

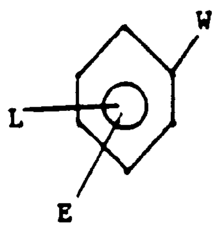

(II)

in der E und L die vorstehend angegebenen Bedeutungen haben und W eine Gruppe der Formel

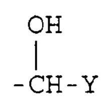

(d)

bedeutet, worin Y eine -COOH-Gruppe oder ein funktionelles Derivat davon bedeutet, mit einer Verbindung der Formel (III)

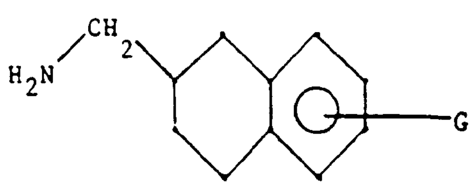

(III)

in der G die vorstehend angegebene Bedeutung hat, umsetzt.

**9.** Verbindung der Formel (III)

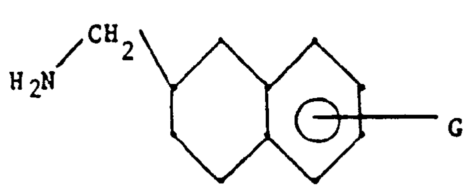

(III)

in der G ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, wobei G aber keine - $OCH_3$-Gruppe in der Stellung 7 oder 8 bedeutet, oder ein Salz davon.

**10.** Verbindung nach Anspruch 9, dadurch gekennzeichnet, daß G eine Hydroxylgruppe oder eine OG'-Gruppe bedeutet, worin G' eine durch eine Carboxylgruppe oder ($C_1$-$C_4$)-Alkoxycarbonylgruppe substituierte ($C_1$-$C_4$)-Alkylgruppe bedeutet.

**11.** Verbindung der Formel (III),

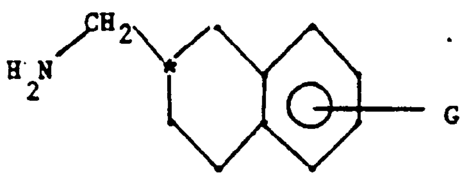

(III)

in der G Wasserstoff, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, in stereoisomerisch reiner Form, oder ein Salz davon.

**12.** Verfahren zur Herstellung einer Verbindung der Formel (III)

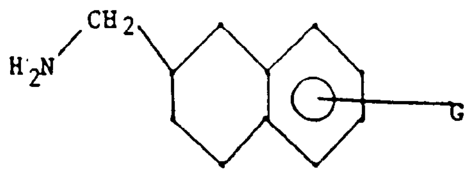

(III)

in der G Wasserstoff, ein Chloratom, eine Hydroxylgruppe oder eine OG'-Gruppe bedeutet, worin G' eine ($C_1$-$C_4$)-Alkylgruppe, die unsubstituiert oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, aber keine -$OCH_3$-Gruppe in 7- oder 8-Stellung bedeutet oder ein Salz davon,
dadurch gekennzeichnet, daß man eine unter folgenden Formeln ausgewählte Verbindung

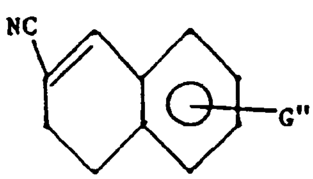

(VIII)

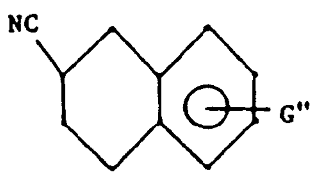

(VIII')

und

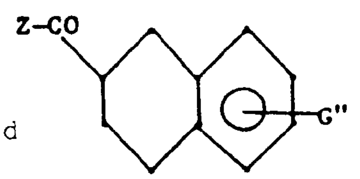

(X)

worin G" Wasserstoff, ein Chloratom, eine Hydroxylgruppe oder eine Methoxygruppe bedeutet und Z eine -$NH_2$-Gruppe bedeutet, reduziert, wodurch man eine Verbindung der Formel (III) erhält, in der G = G", und die Verbindung der Formel (III), in der G" eine Hydroxylgruppe bedeutet, üblichen Alkylierungs- oder Acylierungsverfahren, ggf. unter vorherigem Schutz der Aminogruppe, unterwirft, wobei das Verfahren ferner dadurch gekennzeichnet ist, daß man, wenn man eine Verbindung der Formel (III) erhält, in der G = G" eine Methoxygruppe bedeutet, diese Verbindung durch Demethylierung mit Bromwasserstoffsäure in die entsprechende Verbindung, in der G = G" eine Hydroxylgruppe bedeutet, überführen kann.

**13.** Verbindung der Formel

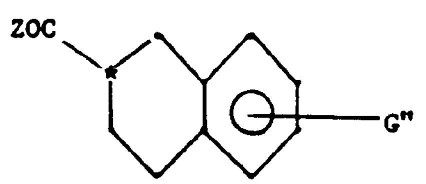

(X)

in der Z eine -OH- oder -$NH_2$-Gruppe bedeutet und G" ein Wasserstoff, ein Chloratom, eine Hydroxylgruppe oder eine Methoxygruppe bedeutet, mit der Maßgabe, daß, wenn Z eine - OH-Gruppe bedeutet, G" kein Wasserstoffatom bedeutet, in optisch aktiver Form oder ein Salz davon.

**14.** Pharmazeutische Zusammensetzung, enthaltend als hauptsächlichen Wirkstoff eine oder mehrere Verbindungen nach Anspruch 1.

**15.** Pharmazeutische Zusammensetzung nach Anspruch 14 zur systemischen Verabreichung in Form einer Dosierungseinheit, enthaltend 0,1 bis 500 mg Wirkstoff.

**16.** Pharmazeutishe Zusammensetzung nach Anspruch 14 oder 15 zur Behandlung von Störungen der intestinalen Motorik.

**17.** Pharmazeutische Zusammensetzung nach Anspruch 14, zur topischen Verabreichung auf das Auge.

**18.** Pharmazeutische Zusammensetzung nach Anspruch 17, enthaltend 10 ng bis 1 mg pro Dosierungseinheit.

**19.** Pharmazeutische Zusammensetzung nach Anspruch 17 oder 18 zur Behandlung von okularem Hochdruck und Glaukom.

**Patentansprüche für folgenden Vertragsstaat : GR**

**1.** Phenylethanolaminomethyltetralin der Formel (I)

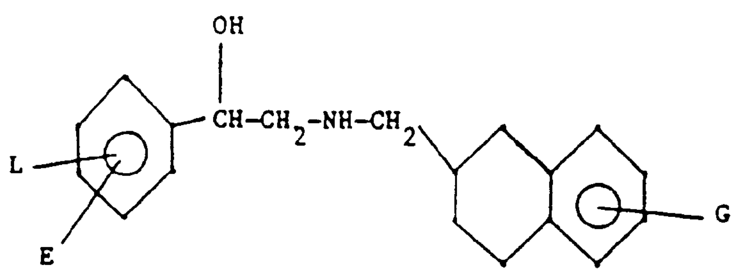

(I)

in der:

- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon.

**2.** Verbindung nach Anspruch 1, worin G Wasserstoff, eine Hydroxylgruppe oder eine OG'-Gruppe bedeutet, worin G' eine substituierte oder unsubstituierte ($C_1$-$C_4$)-Alkylgruppe bedeutet.

**3.** Verbindung nach Anspruch 2, worin G Wasserstoff, eine Hydroxylgruppe oder eine OG'-Gruppe bedeutet, worin G' eine ($C_1$-$C_4$)-Alkylgruppe bedeutet, die unsubstituiert oder durch eine Carbo-($C_1$-$C_4$)-alkoxy- oder Carboxygruppe substituiert ist.

**4.** Verbindung nach einem der Ansprüche 1 bis 3 mit der absoluten R-Konfiguration am chiralen Kohlenstoffatom der Ethanolaminkette.

**5.** Verfahren zur Herstellung der Verbindung der Formel (I)

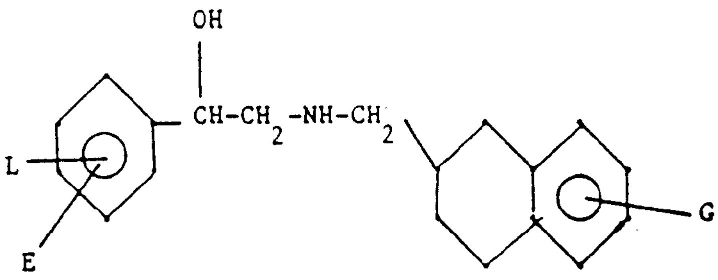

(I)

in der:

- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon,

dadurch gekennzeichnet, daß man eine Verbindung der Formel (II)

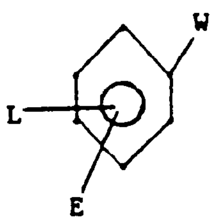

**(II)**

in der E und L die vorstehend angegebenen Bedeutungen besitzen und der Rest -W eine der folgenden Gruppen (a) bis (d) bedeutet

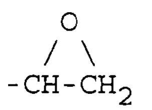 ,

(a)

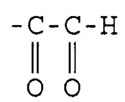 ,

(b)

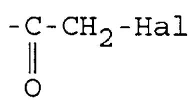

(c)

oder

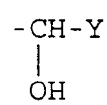

(d)

worin Hal Chlor, Brom oder Jod bedeutet und Y eine Gruppe -COOH oder ein funktionelles Derivat davon bedeutet, mit einer Verbindung der Formel (III)

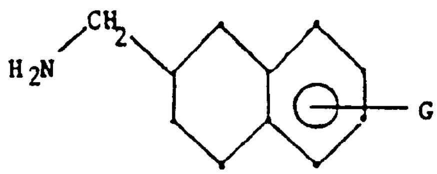 ,

(III)

in der G die vorstehend angegebene Bedeutung hat, umsetzt und, wenn -W eine von

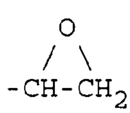 ,

abweichende Bedeutung hat, das Reaktionsprodukt mit einem geeigneten Reduktionsmittel umsetzt und ggf. die auf diese Weise erhaltene Verbindung der Formel (I) nach an sich bekannten Verfahren in ein Salz überführt;

wobei das Verfahren weiter dadurch gekennzeichnet ist, daß man,

(i) wenn man eine Verbindung der Formel (I), in der G eine Hydroxylgruppe bedeutet, erhält, diese Verbindung zu den entsprechenden Verbindungen, in denen G eine OG'-Gruppe bedeutet, umwandeln kann, indem man sie, ggf. nach einem vorübergehenden Schutz der Aminogruppe, mit einem Alkylierungs- oder Acylierungsmittel der Formel G'-D, in der G' die vorstehend definierte Bedeutung hat und D eine leicht entfernbare Gruppe bedeutet, umsetzt,

(ii) wenn man eine Verbindung der Formel (I), in der G eine OG'-Gruppe bedeutet, in der G' einen durch eine ($C_1$-$C_4$)-Alkoxycarbonylgruppe substituierte Alkylgruppe bedeutet, erhält, diese Verbindung durch basische Hydrolyse in die entsprechende Verbindung überführen kann, in der G' eine durch eine Carboxylgruppe substituierte Alkylgruppe bedeutet,

(iii) wenn man eine Verbindung der Formel (I), in der G eine Hydroxylgruppe bedeutet, erhält, diese Verbindung in die entsprechenden Verbindungen überführen kann, in denen G eine Gruppe der Formel OG' bedeutet, worin G' die Bedeutung 1-Methyl-1-($C_1$-$C_4$)-alkoxycarbonylethyl oder 1-Ethyl-1-($C_1$-$C_4$)alkoxycarbonylethyl bedeutet, indem man eine Behandlung mit einer Verbindung der Formel

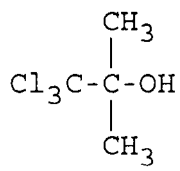

oder

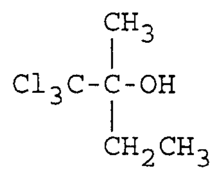

in Gegenwart einer Base durchführt, gefolgt von einer Umsetzung mit Thionylchlorid in einem ($C_1$-$C_4$)-Alkanol, und

(v) wenn man eine Verbindung der Formel (I) in Form eines Isomerengemisches erhält, diese Verbindungen nach herkömmlichen Verfahren in ihre reinen Isomeren oder in ihre Enantiomerenpaare auftrennen kann.

6. Verbindung der folgenden Formel (IV)

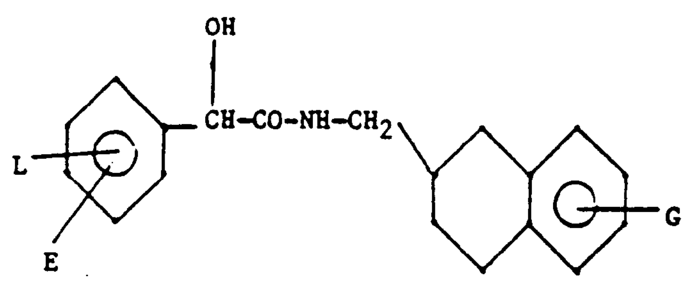

(IV)

in der:

- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon.

7. Verbindung nach Anspruch 6, dadurch gekennzeichnet, daß G ein Wasserstoffatom, eine Hydroxylgruppe oder eine OG'-Gruppe bedeutet, worin G' eine unsubstituierte ($C_1$-$C_4$)-Alkylgruppe oder ($C_3$-$C_7$)-Cyclo-

lakylgruppe bedeutet.

8. Verfahren zur Herstellung einer Verbindung der Formel (IV)

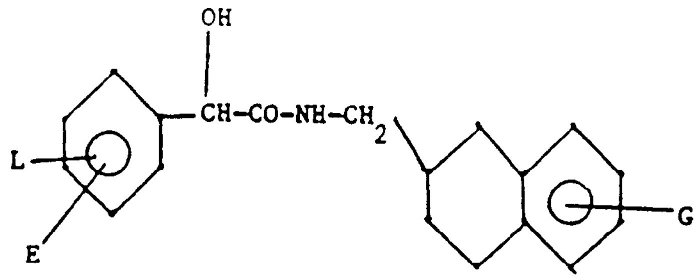

(IV)

in der:
- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder ein Salz davon,

dadurch gekennzeichnet, daß man eine Verbindung der Formel

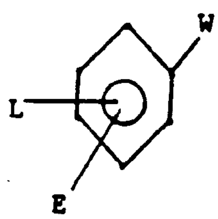

(II)

in der E und L die vorstehend angegebenen Bedeutungen haben und W eine Gruppe der Formel

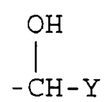

(d)

bedeutet, worin Y eine -COOH-Gruppe oder ein funktionelles Derivat davon bedeutet, mit einer Verbindung der Formel (III)

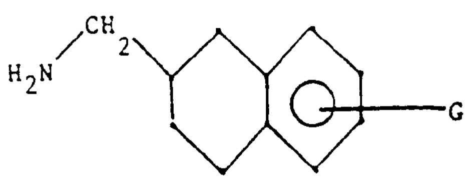

(III)

in der G die vorstehend angegebene Bedeutung hat, umsetzt.

9. Verbindung der Formel (III)

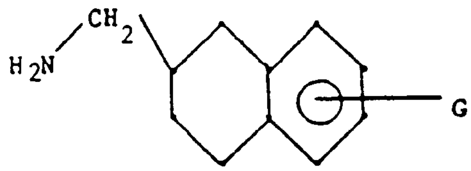

(III)

in der G ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, wobei G aber keine -$OCH_3$-Gruppe in der Stellung 7 oder 8 bedeutet, oder ein Salz davon.

**10.** Verbindung nach Anspruch 9, dadurch gekennzeichnet, daß G eine Hydroxylgruppe oder eine OG'-Gruppe bedeutet, worin G' eine durch eine Carboxylgruppe oder ($C_1$-$C_4$)-Alkoxycarbonylgruppe substituierte ($C_1$-$C_4$)-Alkylgruppe bedeutet.

**11.** Verbindung der Formel (III),

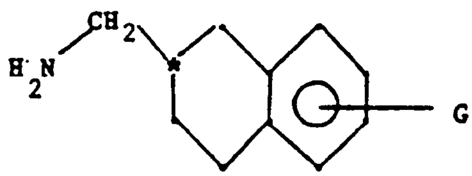

(III)

in der G Wasserstoff, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, in stereoisomerisch reiner Form, oder ein Salz davon.

**12.** Verfahren zur Herstellung einer Verbindung der Formel (III)

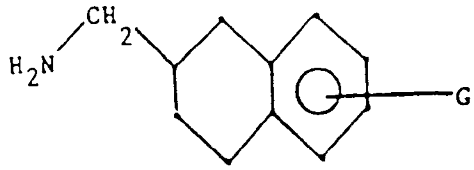

(III)

in der G Wasserstoff, ein Chloratom, eine Hydroxylgruppe oder eine OG'-Gruppe bedeutet, worin G' eine ($C_1$-$C_4$)-Alkylgruppe, die unsubstituiert oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, aber keine -$OCH_3$-Gruppe in 7- oder 8-Stellung bedeutet oder ein Salz davon,
dadurch gekennzeichnet, daß man eine unter folgenden Formeln ausgewählte Verbindung

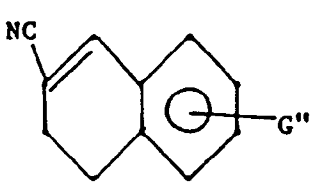

(VIII)

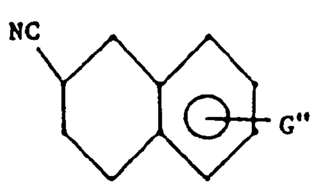

(VIII')

und

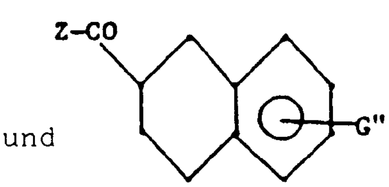

(X)

worin G" Wasserstoff, ein Chloratom, eine Hydroxylgruppe oder eine Methoxygruppe bedeutet und Z eine -$NH_2$-Gruppe bedeutet, reduziert, wodurch man eine Verbindung der Formel (III) erhält, in der G = G",

und die Verbindung der Formel (III), in der G" eine Hydroxylgruppe bedeutet, üblichen Alkylierungs- oder Acylierungsverfahren, ggf. unter vorherigem Schutz der Aminogruppe, unterwirft, wobei das Verfahren ferner dadurch gekennzeichnet ist, daß man, wenn man eine Verbindung der Formel (III) erhält, in der G = G" eine Methoxygruppe bedeutet, diese Verbindung durch Demethylierung mit Bromwasserstoffsäure in die entsprechende Verbindung, in der G = G" eine Hydroxylgruppe bedeutet, überführen kann.

**13.** Verbindung der Formel

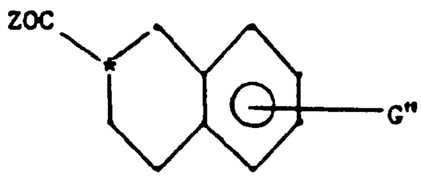

(X)

in der Z eine -OH- oder $-NH_2$-Gruppe bedeutet und G"Wasserstoff, ein Chloratom, eine Hydroxylgruppe oder eine Methoxygruppe bedeutet, mit der Maßgabe, daß, wenn Z eine -OH-Gruppe bedeutet, G" kein Wasserstoffatom bedeutet, in optisch aktiver Form oder ein Salz davon.

**14.** Pharmazeutische Zusammensetzung, enthaltend als hauptsächlichen Wirkstoff eine oder mehrere Verbindungen nach Anspruch 1.

**15.** Verfahren zur Herstellung einer pharmazeutischen Zusammensetzung, dadurch gekennzeichnet, daß man eine Verbindung nach einem der Ansprüche 1 bis 4 als Wirkstoff mit einem pharmazeutisch verträglichen Träger vermischt.

**Patentansprüche für folgenden Vertragsstaat : ES**

**1.** Verfahren zur Herstellung der Verbindung der Formel (I)

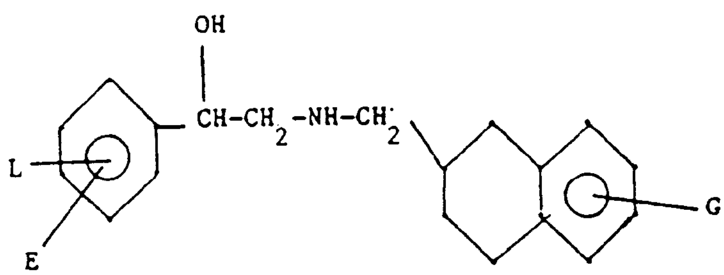

(I)

in der:

- E Wasserstoff, eine $(C_1-C_4)$-Alkylgruppe, eine $(C_1-C_4)$-Alkoxygruppe, eine Phenylgruppe, eine Nitrogruppe, ein Halogenatom oder eine Trifluormethylgruppe bedeutet,
- L Wasserstoff, eine $(C_1-C_4)$-Alkylgruppe, eine Phenylgruppe, eine $(C_1-C_4)$-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine $(C_1-C_4)$-Alkylgruppe, die unsubstituiert ist oder durch eine Hydroxyl-, $(C_1-C_4)$-Alkoxy-, $(C_1-C_4)$-Alkoxycarbonyl-, Carboxyl- oder $(C_3-C_7)$-Cycloalkylgruppe substituiert ist; eine $(C_3-C_7)$-Cycloalkylgruppe; oder eine $(C_2-C_4)$-Alkanoylgruppe bedeutet, oder ein Salz davon,

dadurch gekennzeichnet, daß man eine Verbindung der Formel (II)

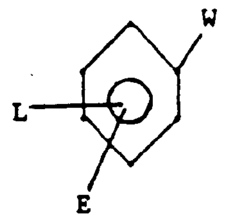

(II)

in der E und L die vorstehend angegebenen Bedeutungen besitzen und der Rest -W eine der folgenden Gruppen (a) bis (d) bedeutet

| (a) | (b) | (c) | | (d) |
|---|---|---|---|---|
| O<br>/ \\<br>$-CH-CH_2$ , | $-C-C-H$ ,<br>‖ ‖<br>O O | $-C-CH_2-Hal$<br>‖<br>O | oder | $-CH-Y$<br>\|<br>OH |

worin Hal Chlor, Brom oder Jod bedeutet und Y eine Gruppe -COOH oder ein funktionelles Derivat davon bedeutet, mit einer Verbindung der Formel (III)

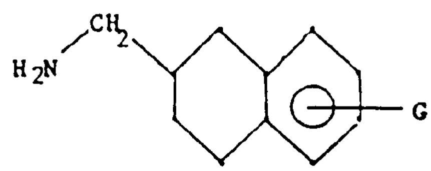

, (III)

in der G die vorstehend angegebene Bedeutung hat, umsetzt und, wenn -W eine von

O<br>/ \<br>$-CH-CH_2$ ,

abweichende Bedeutung hat, das Reaktionsprodukt mit einem geeigneten Reduktionsmittel umsetzt und ggf. die auf diese Weise erhaltene Verbindung der Formel (I) nach an sich bekannten Verfahren in ein Salz überführt;
wobei das Verfahren weiter dadurch gekennzeichnet ist, daß man,

(i) wenn man eine Verbindung der Formel (I), in der G eine Hydroxylgruppe bedeutet, erhält, diese Verbindung zu den entsprechenden Verbindungen, in denen G eine OG'-Gruppe bedeutet, umwandeln kann, indem man sie, ggf. nach einem vorübergehenden Schutz der Aminogruppe, mit einem Alkylierungs- oder Acylierungsmittel der Formel G'-D, in der G' die vorstehend definierte Bedeutung hat und D eine leicht entfernbare Gruppe bedeutet, umsetzt,

(ii) wenn man eine Verbindung der Formel (I), in der G eine OG'-Gruppe bedeutet, in der G' einen durch eine ($C_1$-$C_4$)-Alkoxycarbonylgruppe substituierte Alkylgruppe bedeutet, erhält, diese Verbindung durch basische Hydrolyse in die entsprechende Verbindung überführen kann, in der G' eine durch eine Carboxylgruppe substituierte Alkylgruppe bedeutet,

(iii) wenn man eine Verbindung der Formel (I), in der G eine Hydroxylgruppe bedeutet, erhält, diese Verbindung in die entsprechenden Verbindungen überführen kann, in denen G eine Gruppe der Formel OG' bedeutet, worin G' die Bedeutung 1-Methyl-1-($C_1$-$C_4$)-alkoxycarbonylethyl oder 1-Ethyl-1-($C_1$-$C_4$)alkoxycarbonylethyl bedeutet, indem man eine Behandlung mit einer Verbindung der Formel

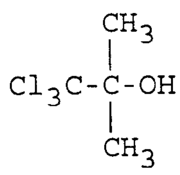

oder

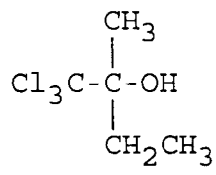

in Gegenwart einer Base durchführt, gefolgt von einer Umsetzung mit Thionylchlorid in einem ($C_1$-$C_4$)-Alkanol, und
(v) wenn man eine Verbindung der Formel (I) in Form eines Isomerengemisches erhält, diese Verbindungen nach herkömmlichen Verfahren in ihre reinen Isomeren oder in ihre Enantiomerenpaare auftrennen kann.

**2.** Verfahren nach Anspruch 1 zur Herstellung einer Verbindung der Formel (I), in der G Wasserstoff, eine Hydroxylgruppe oder eine OG'-Gruppe bedeutet, worin G' eine substituierte oder unsubstituierte ($C_1$-$C_4$)-Alkylgruppe bedeutet.

**3.** Verfahren nach Anspruch 2 zur Herstellung einer Verbindung der Formel (I), in der G Wasserstoff, eine Hydroxylgruppe oder eine OG'-Gruppe bedeutet, worin G' eine ($C_1$-$C_4$)-Alkylgruppe bedeutet, die unsubstituiert oder durch eine Carbo- ($C_1$-$C_4$)-alkoxy- oder Carboxygruppe substituiert ist.

**4.** Verbindung nach einem der Ansprüche 1 bis 3 zur Herstellung einer Verbindung der Formel (I) mit der absoluten R-Konfiguration am chiralen Kohlenstoffatom der Ethanolaminkette.

**5.** Verfahren zur Herstellung einer Verbindung der folgenden Formel (IV)

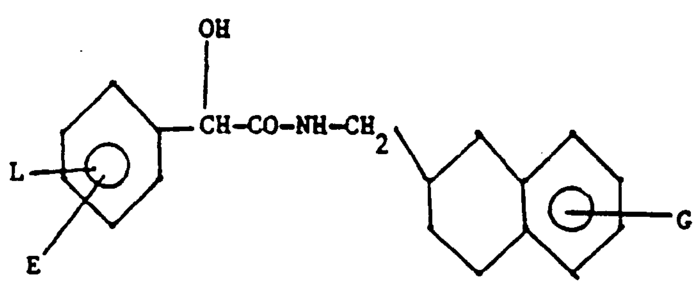

(IV)

in der:
- E Wasserstoff, eine ($C_1$-$C_4$)-Alkylgruppe, eine ($C_1$-$C_4$)-Alkoxygruppe, eine Phenylgruppe, eine Nitrogruppe, ein Halogenatom oder eine Trifluormethylgruppe bedeutet,
- L Wasserstoff, eine ($C_1$-$C_4$)-Alkylgruppe, eine Phenylgruppe, eine ($C_1$-$C_4$)-Alkoxygruppe, eine Nitrogruppe oder ein Halogenatom bedeutet oder
- E und L zusammen eine Gruppe der Formeln -CH=CH-CH=CH- oder $-CH_2-CH_2-CH_2-CH_2-$ bedeuten und
- G ein Wasserstoffatom, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, oder eines Salz davon,

dadurch gekennzeichnet, daß man eine Verbindung der Formel (II)

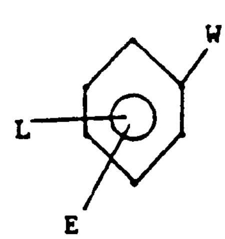

(II)

in der E und L die vorstehend angegebenen Bedeutungen haben und W eine Gruppe der Formel

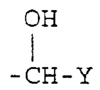 (d)

bedeutet, worin Y eine -COOH-Gruppe oder ein funktionelles Derivat davon bedeutet, mit einer Verbindung der Formel (III)

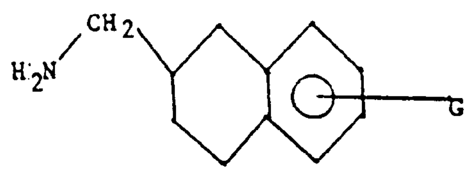 (III)

in der G die vorstehend angegebene Bedeutung hat, umsetzt.

6. Verfahren nach Anspruch 5 zur Herstellung einer Verbindung der Formel (IV), in der G ein Wasserstoffatom, eine Hydroxylgruppe oder eine OG'-Gruppe bedeutet, worin G' eine unsubstituierte ($C_1$-$C_4$)-Alkylgruppe oder ($C_3$-$C_7$)-Cyclolakylgruppe bedeutet.

7. Verfahren zur Herstellung einer Verbindung der Formel (III)

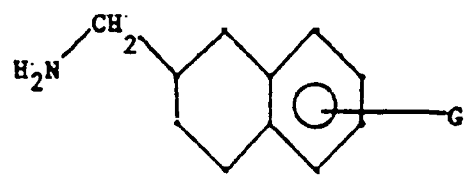 (III)

in der G Wasserstoff, ein Chloratom, eine Hydroxylgruppe oder eine OG'-Gruppe bedeutet, worin G' eine ($C_1$-$C_4$)-Alkylgruppe, die unsubstituiert oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, aber keine -$OCH_3$-Gruppe in 7- oder 8-Stellung bedeutet oder ein Salz davon,
dadurch gekennzeichnet, daß man eine unter folgenden Formeln ausgewählte Verbindung

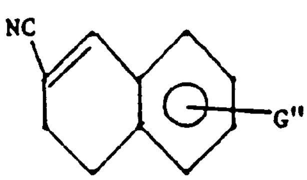 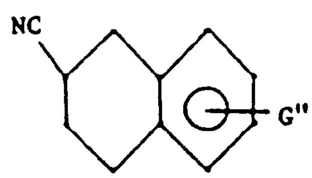 und 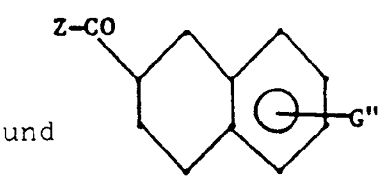

(VIII) (VIII') (X)

worin G" Wasserstoff, ein Chloratom, eine Hydroxylgruppe oder eine Methoxygruppe bedeutet und Z eine -$NH_2$-Gruppe bedeutet, reduziert, wodurch man eine Verbindung der Formel (III) erhält, in der G = G", und die Verbindung der Formel (III), in der G" eine Hydroxylgruppe bedeutet, üblichen Alkylierungs- oder Acylierungsverfahren, ggf. unter vorherigem Schutz der Aminogruppe, unterwirft, wobei das Verfahren ferner dadurch gekennzeichnet ist, daß man, wenn man eine Verbindung der Formel (III) erhält, in der G = G" eine Methoxygruppe bedeutet, diese Verbindung durch Demethylierung mit Bromwasserstoffsäure in die entsprechende Verbindung, in der G = G" eine Hydroxylgruppe bedeutet, überführen kann, wobei man die erhaltene Verbindung, sofern sie in Form eines Isomerengemisches vorliegt, nach herkömmlichen Methoden in ihre reinen Isomeren auftrennen kann.

8. Verfahren nach Anspruch 7 zur Herstellung einer Verbindung der Formel (III), in der G eine Hydroxylgruppe oder eine OG'-Gruppe bedeutet, worin G' eine ($C_1$-$C_4$)-Alkylgruppe bedeutet, die unsubstituiert oder durch eine Carboxygruppe oder eine ($C_1$-$C_4$)-Alkoxycarbonylgruppe substituiert ist.

9. Verfahren nach Anspruch 7 zur Herstellung einer Verbindung der Formel (III)

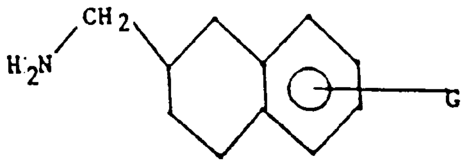

(III)

in der G Wasserstoff, ein Chloratom, eine Hydroxylgruppe oder eine OG'-Gruppe bedeutet, worin G' eine ($C_1$-$C_4$)-Alkylgruppe, die unsubstituiert ist oder durch eine Hydroxyl-, ($C_1$-$C_4$)-Alkoxy-, ($C_1$-$C_4$)-Alkoxycarbonyl-, Carboxyl- oder ($C_3$-$C_7$)-Cycloalkylgruppe substituiert ist; eine ($C_3$-$C_7$)-Cycloalkylgruppe; oder eine ($C_2$-$C_4$)-Alkanoylgruppe bedeutet, in stereoisomerisch reiner Form, oder eines Salzes davon.

10. Verfahren zur Herstellung einer Verbindung der Formel (X)

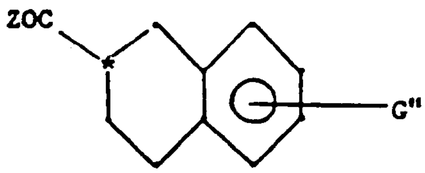

(X)

in der Z eine -OH- oder -$NH_2$-Gruppe bedeutet und G" Wasserstoff, ein Chloratom, eine Hydroxylgruppe oder eine Methoxygruppe bedeutet, mit der Maßgabe, daß, wenn Z eine -OH-Gruppe bedeutet, G" kein Wasserstoffatom bedeutet, in optisch aktiver Form oder ein Salz davon, dadurch gekennzeichnet, daß man eine Verbindung der Formel (V)

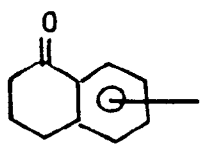

(V)

in der G" die vorstehend definierte Bedeutung hat, mit Diethylcarbonat in Gegenwart von Natrium zu einer Verbindung der Formel (XI) umsetzt

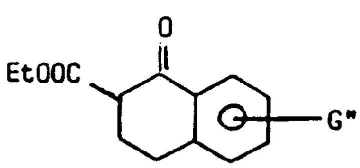

(XI)

in der G" die vorstehend definierte Bedeutung hat, wobei diese Verbindung anschließend einer katalytischen oder chemischen Reduktion unterworfen wird, anschließend in basischem Milieu hydrolysiert wird, wodurch man eine Verbindung der vorstehenden Formel X erhält, in der Z = OH und G" die vorstehend definierte Bedeutung hat, wobei diese Verbindung schließlich nach herkömmlichen Verfahren in das entsprechende Amid übergeführt wird, wodurch man eine Verbindung der Formel (X) erhält, in der Z = $NH_2$, und gegebenenfalls die auf diese Weise erhaltene Verbindung der Formel (X) nach bekannten Verfahren in eines ihrer Salze überführt.

11. Verfahren zur Herstellung einer pharmazeutischen Zusammensetzung, dadurch gekennzeichnet, daß man eine Verbindung nach einem der Ansprüche 1 bis 4 als Wirkstoff mit einem pharmazeutisch verträglichen Träger vermischt.

## Claims

### Claims for the following Contracting States : DE, GB, FR, IT, NL, SE, CH, LI, BE, AT, LU, DK

1. A phenylethanolaminomethyltetraline of following formula (I)

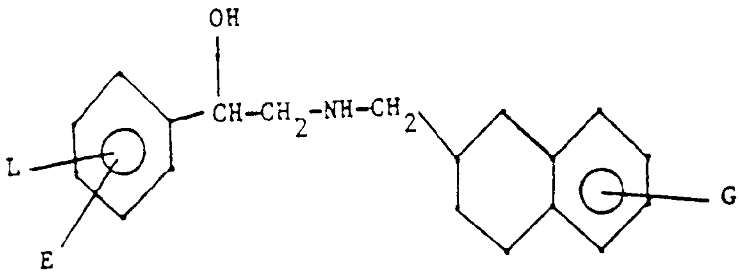

(I).

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2-$, and
- G represents a hydrogen atom, a chlorine atom, a hydroxy group or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group;

  a ($C_3$-$C_7$)cycloalkyl group; or a ($C_2$-$C_4$)alkanoyl group, or one of its salts.

2. A compound according to claim 1 wherein G represents hydrogen, a hydroxy group or a group OG' wherein G' represents an unsubstituted or a substituted ($C_1$-$C_4$) alkyl group.

3. A compound according to claim 2 wherein G represents hydrogen, a hydroxy group or a group OG' wherein G' represents a ($C_1$-$C_4$)alkyl group unsubstituted or substituted with a carbo($C_1$-$C_4$)alkoxy or carboxy group.

4. A compound according to any one of claims 1 to 3 wherein the chiral carbon atom in the ethanolamino chain has the absolute configuration (R).

5. A process for the preparation of a compound of following formula (I)

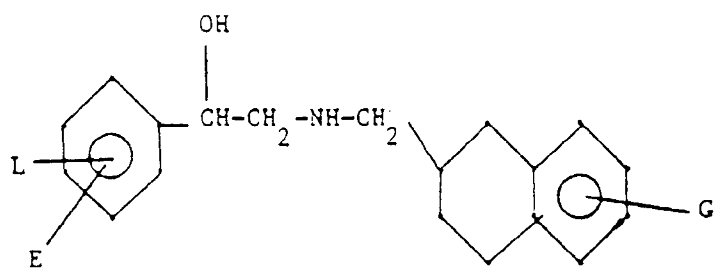

(I)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2$, and
- G represents a hydrogen atom, a chlorine atom, a hydroxy group or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group;

  a ($C_3$-$C_7$)cycloalkyl group; or a ($C_2$-$C_4$)alkanoyl group, or one of its salts, characterized in that it comprises reacting a compound of formula (II)

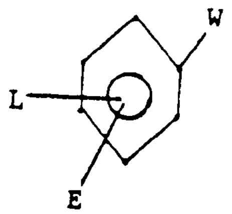

(II)

wherein

- E and L are as defined above and the radical -W represents one of the following groups (a) to (d)

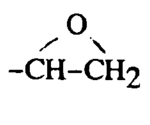 , 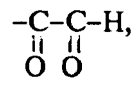 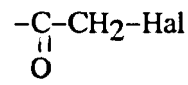 or 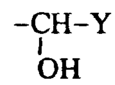

(a) (b) (c) (d)

wherein Hal represents chlorine, bromine, or iodine, and Y is a -COOH group or a functional derivative thereof,
with a compound of formula (III)

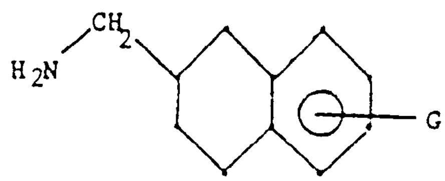

, (III)

wherein G is as defined above, and, when -W is different from

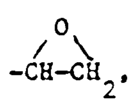

treating the reaction product with a suitable reducing agent and optionally converting the compound of formula (I) thus obtained in one of its salts according to per se known methods;
said process being further characterized in that

(i) when a compound of formula (I) is obtained wherein G is a hydroxy group, said compound may be converted into the corresponding compounds wherein G is an OG' group by reaction with alkylating or acylating agents of formula G'-D wherein G' is as defined above and D is an easily leaving group, after temporary protection, or not, of the amino group,

(ii) when a compound of formula (I) is obtained wherein G is an OG' group wherein G' is an alkyl group substituted with a ($C_1$-$C_4$)alkoxycarbonyl group, said compound may be converted into the corresponding compound wherein G' is an alkyl group substituted with carboxy, by basic hydrolysis.

(iii) when a compound of formula (I) is obtained wherein G is hydroxy, said compound may be converted into the corresponding compounds wherein G is a group of formula OG' wherein G' is
1-methyl-1-($C_1$-$C_4$)alkoxycarbonyl-ethyl or
1-ethyl-1-($C_1$-$C_4$)alkoxycarbonyl-ethyl by treatment with a compound of formula

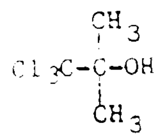

or

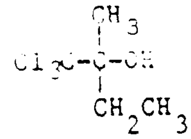

respectively, in the presence of a base, followed by reaction with thionyl chloride in a ($C_1$-$C_4$)alkanol, and

(iv) when a compound of formula (I) is obtained as a mixture of isomers, they may be separated into the pure isomers or the couples of enantiomers by conventional methods.

6. A compound of following formula (IV)

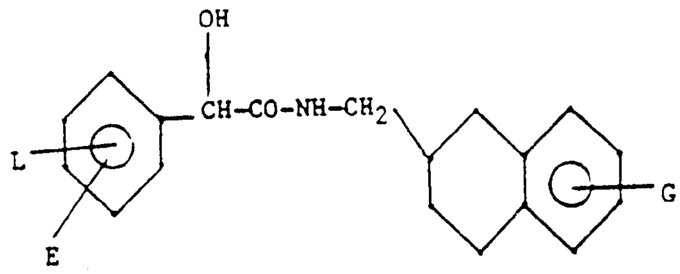

(IV)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2-$, and
- G represents a hydrogen atom, a chlorine atom, a hydroxy group or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group;
  a ($C_3$-$C_7$)cycloalkyl group; or a ($C_2$-$C_4$)alkanoyl group, or one of its salts.

7. A compound according to claim 6, characterized in that G is a hydrogen atom, a hydroxy group, or an OG' group wherein G' is an unsubstituted ($C_1$-$C_4$)alkyl group or ($C_3$-$C_7$)cycloalkyl.

8. A process for the preparation of a compound of following formula (IV)

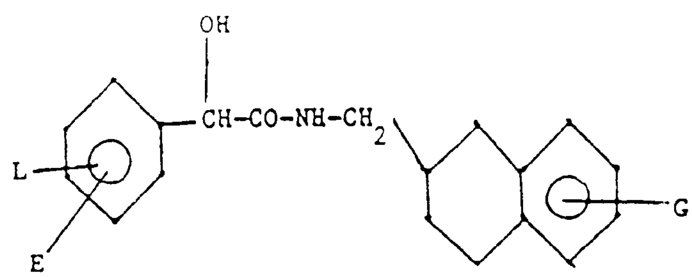

(IV)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2-$, and
- G represents a hydrogen atom, a chlorine atom, a hydroxy group or an OG' group wherein G' represents a ($C_1$-$C_4$)alkyl group either unsubstituted or substituted with a hydroxy, ($C_1$-$C_4$)alkoxy, ($C_1$-

$C_4$)alkoxycarbonyl, carboxy, or ($C_3$-$C_7$)cycloalkyl group;

a ($C_3$-$C_7$)cycloalkyl group; or a ($C_2$-$C_4$)alkanoyl group, or one of its salts, characterized in that it comprises reacting a compound of formula

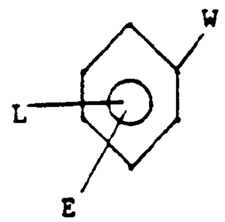

(II)

wherein E and L are as defined above and W represents a group

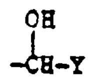

(d)

wherein Y is a -COOH group or a functional derivative thereof,
with a compound of formula (III)

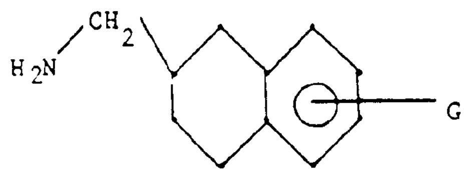

(III)

wherein G is as defined above.

**9.** A compound of formula (III)

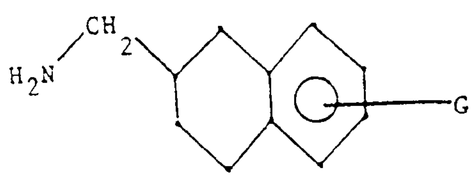

(III)

wherein G is a chlorine atom, a hydroxy group, or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy or ($C_3$-$C_7$)cycloalkyl group; a ($C_3$-$C_7$)cycloalkyl group; or ($C_2$-$C_4$)alkanoyl group, but G is different from an $-OCH_3$ group at the 7-position or 8-position, or one of its salts.

**10.** A compound according to claim 9, characterized in that G is a hydroxy group or an OG' group wherein G' represents a ($C_1$-$C_4$)alkyl group substituted with a carboxy or ($C_1$-$C_4$)alkoxycarbonyl group.

**11.** A compound of formula (III)

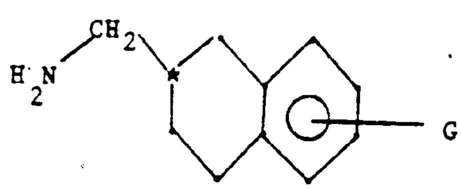

(III)

wherein G is hydrogen, a chlorine atom, a hydroxy group, or an OG' group wherein G' represents a ($C_1$-

$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group; a ($C_3$-$C_7$)cycloalkyl group; or a ($C_2$-$C_4$)alkanoyl group, in the form of pure stereoisomer, or one of its salts.

**12.** A process for the preparation of a compound of formula (III)

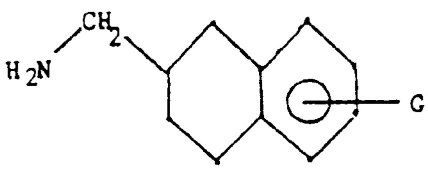

, (III)

wherein G is hydrogen, a chlorine atom, a hydroxy group, or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group; a ($C_3$-$C_7$)cycloalkyl group; or a ($C_2$-$C_4$)alkanoyl group, but different from an $-OCH_3$ group at the 7- or 8- position, or of one of its salts-, characterized in that it comprises reducing a compound of formula selected from

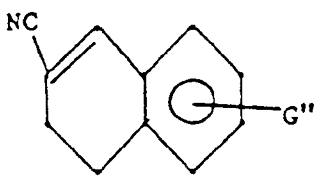

(VIII)

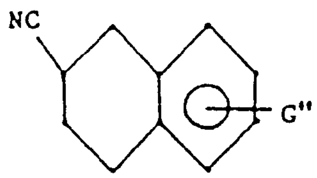

(VIII')

and

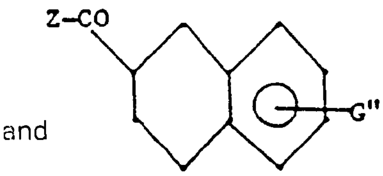

(X)

wherein G" represents hydrogen, a chlorine atom, a hydroxy group or a methoxy group and Z is an $-NH_2$ group, thus obtaining a compound of formula (III) wherein G = G", and submitting the compound of formula (III) wherein G" is hydroxy to conventional alkylation or acylation procedures with or without prior protection of the amino group, said process being further characterized in that when a compound of formula (III) is obtained wherein G = G" is a methoxy group, said compound may be converted into the corresponding compound wherein G = G" is a hydroxy group by demethylation with hydrobromic acid

**13.** A compound of formula

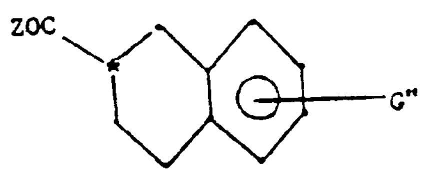

(X)

wherein Z is a group -OH or $-NH_2$ and G" represents hydrogen, a chlorine atom, a hydroxy group, or a methoxy group, provided that when Z is an -OH group, G" is different from hydrogen, in optically active form, or one of its salts.

**14.** A pharmaceutical composition containing one or more compounds according to claim 1 as the main active principle.

**15.** A pharmaceutical composition according to claim 14 for systemic administration in unit dosage form containing from 0.1 to 500 mg of active principle.

**16.** A pharmaceutical composition according to claim 14 or 15 for the treatment of intestinal motility troubles.

**17.** A pharmaceutical composition according to claim 14 for topical administration to the eye.

**18.** A pharmaceutical composition according to claim 17 containing from 10 ng to 1 mg of active principle per

unit dosage form.

19. A pharmaceutical composition according to claim 17 or 18 for the treatment of ocular hypertension and glaucoma.

**Claims for the following Contracting State : GR**

1. A phenylethanolaminomethyltetraline of following formula (I)

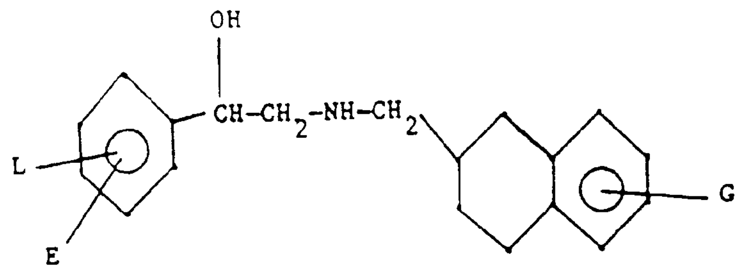

(I)

wherein
- E represents hydrogen, a $(C_1\text{-}C_4)$alkyl group, a $(C_1\text{-}C_4)$alkoxy group, a phenyl group, a nitro group, a halogen atom or a trifluoromethyl group,
- L represents hydrogen, a $(C_1\text{-}C_4)$alkyl group, phenyl, $(C_1\text{-}C_4)$ alkoxy, a nitro group, or a halogen atom, or
- E and L taken together represent a group -CH=CH-CH=CH- or $-CH_2\text{-}CH_2\text{-}CH_2\text{-}CH_2\text{-}$, and
- G represents a hydrogen atom, a chlorine atom, a hydroxy group or an OG' group wherein G' represents a $(C_1\text{-}C_4)$alkyl group either unsubstituted or substituted with a hydroxy, $(C_1\text{-}C_4)$alkoxy, $(C_1\text{-}C_4)$alkoxycarbonyl, carboxy, or $(C_3\text{-}C_7)$cycloalkyl group; a $(C_3\text{-}C_7)$cycloalkyl group; or a $(C_2\text{-}C_4)$alkanoyl group,

or one of its salts.

2. A compound according to claim 1 wherein G represents hydrogen, a hydroxy group or a group OG' wherein G' represents an unsubstituted or a substituted $(C_1\text{-}C_4)$ alkyl group.

3. A compound according to claim 2 wherein G represents hydrogen, a hydroxy group or a group OG' wherein G' represents a $(C_1\text{-}C_4)$alkyl group unsubstituted or substituted with a carbo$(C_1\text{-}C_4)$alkoxy or carboxy group.

4. A compound according to any one of claims 1 to 3 wherein the chiral carbon atom in the ethanolamino chain has the absolute configuration (R).

5. A process for the preparation of a compound of following formula (I)

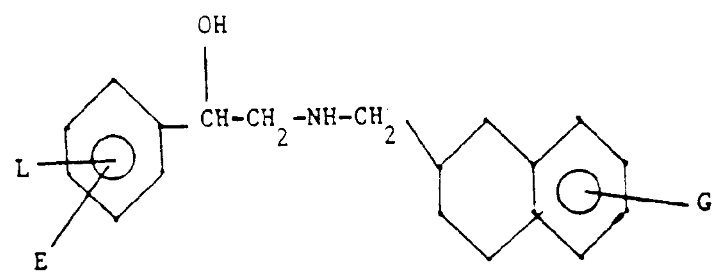

(I)

wherein
- E represents hydrogen, a $(C_1\text{-}C_4)$alkyl group, a $(C_1\text{-}C_4)$alkoxy group, a phenyl group, a nitro group, a halogen atom or a trifluoromethyl group,
- L represents hydrogen, a $(C_1\text{-}C_4)$alkyl group, phenyl, $(C_1\text{-}C_4)$ alkoxy, a nitro group, or a halogen atom, or

- E and L taken together represent a group -CH=CH-CH=CH- or $-CH_2-CH_2-CH_2-CH_2$, and
- G represents a hydrogen atom, a chlorine atom, a hydroxy group or an OG' group wherein G' represents a $(C_1-C_4)$alkyl group either unsubstituted or substituted with a hydroxy, $(C_1-C_4)$alkoxy, $(C_1-C_4)$alkoxycarbonyl, carboxy, or $(C_3-C_7)$cycloalkyl group;

a $(C_3-C_7)$cycloalkyl group; or a $(C_2-C_4)$alkanoyl group, or one of its salts, characterized in that it comprises reacting a compound of formula (II)

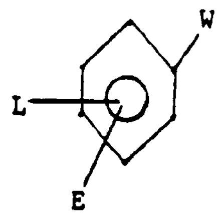

(II)

wherein

- E and L are as defined above and the radical -W represents one of the following groups (a) to (d)

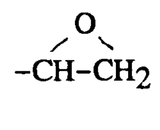 , 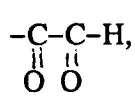 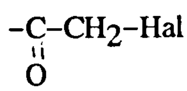 or 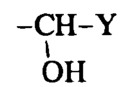

(a) (b) (c) (d)

wherein Hal represents chlorine, bromine, or iodine, and Y is a -COOH group or a functional derivative thereof,
with a compound of formula (III)

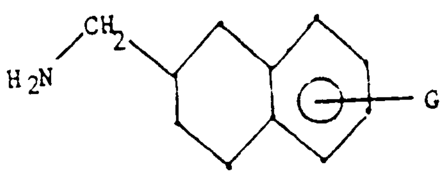

(III)

wherein G is as defined above, and, when -W is different from

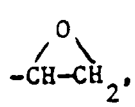

treating the reaction product with a suitable reducing agent and optionally converting the compound of formula (I) thus obtained in one of its salts according to per se known methods;
said process being further characterized in that

(i) when a compound of formula (I) is obtained wherein G is a hydroxy group, said compound may be converted into the corresponding compounds wherein G is an OG' group by reaction with alkylating or acylating agents of formula G'-D wherein G' is as defined above and D is an easily leaving group, after temporary protection, or not, of the amino group,

(ii) when a compound of formula (I) is obtained wherein G is an OG' group wherein G' is an alkyl group substituted with a $(C_1-C_4)$alkoxycarbonyl group, said compound may be converted into the corresponding compound wherein G' is an alkyl group substituted with carboxy, by basic hydrolysis.

(iii) when a compound of formula (I) is obtained wherein G is hydroxy, said compound may be converted into the corresponding compounds wherein G is a group of formula OG' wherein G' is

1-methyl-1-($C_1$-$C_4$)alkoxycarbonyl-ethyl or 1-ethyl-1-($C_1$-$C_4$)alkoxycarbonyl-ethyl by treatment with a compound of formula

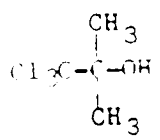

or

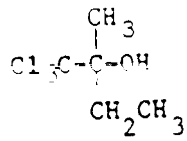

respectively, in the presence of a base, followed by reaction with thionyl chloride in a ($C_1$-$C_4$)alkanol, and

(iv) when a compound of formula (I) is obtained as a mixture of isomers, they may be separated into the pure isomers or the couples of enantiomers by conventional methods.

**6.** A compound of following formula (IV)

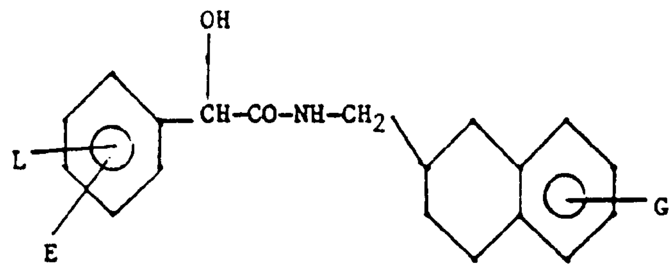

(IV)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2-$, and
- G represents a hydrogen atom, a chloro atom, a hydroxy group or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group; a ($C_3$-$C_7$)cycloalkyl group; or a ($C_2$-$C_4$)alkanoyl group,

or one of its salts.

**7.** A compound according to claim 6, characterized in that G is a hydrogen atom, a hydroxy group, or an OG' group wherein G' is an unsubstituted ($C_1$-$C_4$)alkyl group or ($C_3$-$C_7$)cycloalkyl.

**8.** A process for the preparation of a compound of following formula (IV)

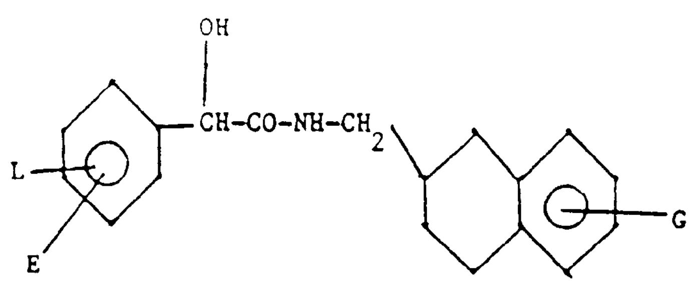

(IV)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom,

or

- E and L taken together represent a group -CH=CH-CH=CH- or $-CH_2-CH_2-CH_2-CH_2-$, and
- G represents a hydrogen atom, a chlorine atom, a hydroxy group or an OG' group wherein G' represents a $(C_1-C_4)$alkyl group either unsubstituted or substituted with a hydroxy, $(C_1-C_4)$alkoxy, $(C_1-C_4)$alkoxycarbonyl, carboxy, or $(C_3-C_7)$cycloalkyl group; a $(C_3-C_7)$cycloalkyl group; or a $(C_2-C_4)$alkanoyl group,

or one of its salts, characterized in that it comprises reacting a compound of formula

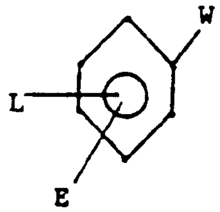

(II)

wherein E and L are as defined above and W represents a group

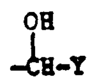

(d)

wherein Y is a -COOH group or a functional derivative thereof, with a compound of formula (III)

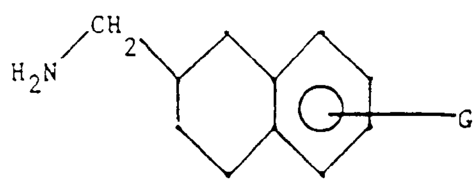

(III)

wherein G is as defined above.

9. A compound of formula (III)

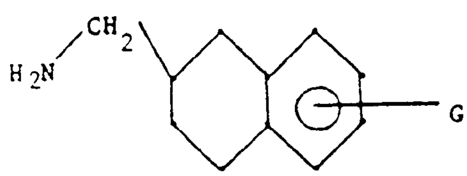

(III)

wherein G is a chlorine atom, a hydroxy group, or an OG' group wherein G' represents a $(C_1-C_4)$alkyl group either unsubstituted or substituted with a hydroxy, $(C_1-C_4)$alkoxy, $(C_1-C_4)$alkoxycarbonyl, carboxy or $(C_3-C_7)$cycloalkyl group; a $(C_3-C_7)$cycloalkyl group; or $(C_2-C_4)$alkanoyl group, but G is different from an $-OCH_3$ group at the 7-position or 8-position, or one of its salts.

10. A compound according to claim 9, characterized in that G is a hydroxy group or an OG' group wherein G' represents a $(C_1-C_4)$alkyl group substituted with a group carboxy or $(C_1-C_4)$alkoxycarbonyl.

11. A compound of formula (III)

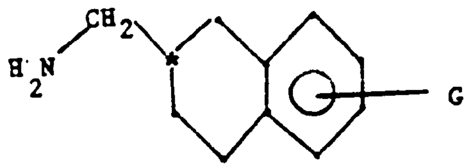

(III)

wherein G is hydrogen, a chlorine atom, a hydroxy group, or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group; a ($C_3$-$C_7$)cycloalkyl group; or a ($C_2$-$C_4$)alkanoyl group, in the form of pure stereoisomer, or one of its salts.

**12.** A process for the preparation of a compound of formula (III)

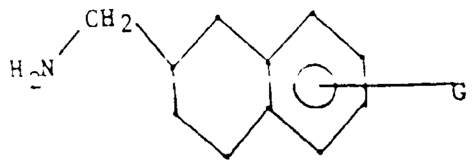

(III)

wherein G is hydrogen, a chlorine atom, a hydroxy group, or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group; a ($C_3$-$C_7$)cycloalkyl group; or a ($C_2$-$C_4$)alkanoyl group, but different from an $-OCH_3$ group

at the 7- or 8- position, or of one of its salts-, characterized in that it comprises reducing a compound of formula selected from

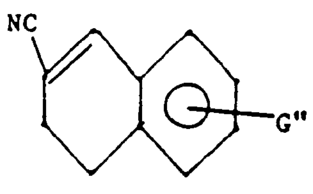

(VIII)

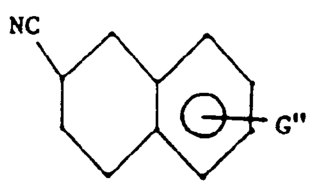

(VIII')

and

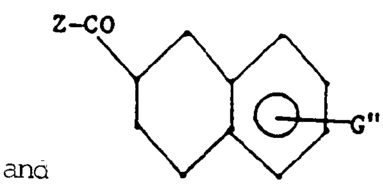

(X)

wherein G" represents hydrogen, a chlorine atom, a hydroxy group or a methoxy group and Z is an $-NH_2$ group, thus obtaining a compound of formula (III) wherein G = G", and submitting the compound of formula (III) wherein G" is hydroxy to conventional alkylation or acylation procedures with or without prior protection of the amino group, said process being further characterized in that when a compound of formula (III) is obtained wherein G = G" is a methoxy group, said compound may be converted into the corresponding compound wherein G = G" is a hydroxy group by demethylation with hydrobromic acid.

**13.** A compound of formula

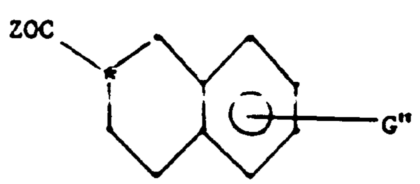

(X)

wherein Z is a group -OH or $-NH_2$ and G" represents hydrogen, a chlorine atom, a hydroxy group, or a methoxy group, provided that when Z is an -OH group, G" is different from hydrogen, in optically active form, or one of its salts.

**14.** A pharmaceutical composition containing one or more compounds according to claim 1 as the main active

principle.

15. A process for the preparation of a pharmaceutical composition, characterized in that it comprises mixing a compound according to any one of claims 1 to 4 with a pharmaceutically acceptable vehicle, as the active principle.

**Claims for the following Contracting State : ES**

1. A process for the preparation of a compound of following formula (I)

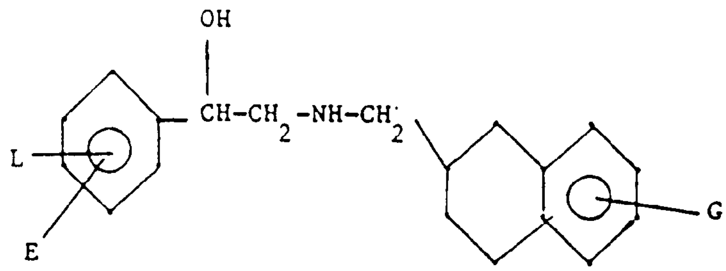

(I)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2-$, and
- G represents a hydrogen atom, a chlorine atom, a hydroxy group or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group;
  a ($C_3$-$C_7$)cycloalkyl group; or a ($C_2$-$C_4$)alkanoyl group,

or one of its salts, characterized in that it comprises reacting a compound of formula (II)

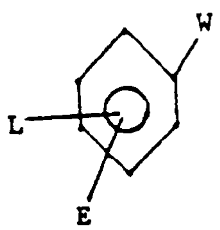

(II)

wherein

- E and L are as defined above and the radical -W represents one of the following groups (a) to (d)

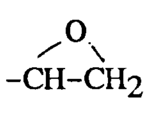 , 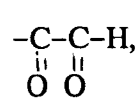 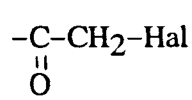 or 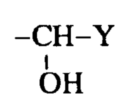

(a) (b) (c) (d)

wherein Hal represents chlorine, bromine, or iodine, and Y is a -COOH group or a functional derivative thereof, with a compound of formula (III)

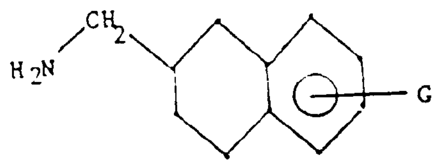

(III)

wherein G is as defined above, and when -W is different from

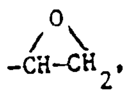

treating the reaction product with a suitable reducing agent and optionally converting the compound of formula (I) thus obtained in one of its salts according to per se known methods;
said process being further characterized in that

(i) when a compound of formula (I) is obtained wherein G is a hydroxy group, said compound may be converted into the corresponding compounds wherein G is an OG' group by reaction with alkylating or acylating agents of formula G'-D wherein G' is as defined above and D is an easily leaving group, after temporary protection, or not, of the amino group,
(ii) when a compound of formula (I) is obtained wherein G is an OG' group wherein G' is an alkyl group substituted with a ($C_1$-$C_4$)alkoxycarbonyl group, said compound may be converted into the corresponding compound wherein G' is an alkyl group substituted with carboxy, by basic hydrolysis.
(iii) when a compound of formula (I) is obtained wherein G is hydroxy, said compound may be converted into the corresponding compounds wherein G is a group of formula OG' wherein G' is
1-methyl-1-($C_1$-$C_4$)alkoxycarbonyl-ethyl or
1-ethyl-1-($C_1$-$C_4$)alkoxycarbonyl-ethyl by treatment with a compound of formula

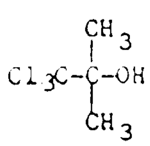

or

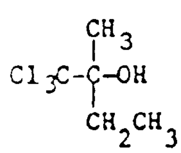

respectively, in the presence of a base, followed by reaction with thionyl chloride in a ($C_1$-$C_4$)alkanol, and
(iv) when a compound of formula (I) is obtained as a mixture of isomers, they may be separated into the pure isomers or the couples of enantiomers by conventional methods.

2. A process according to claim 1 for the preparation of a compouund of formula (I), wherein G represents hydrogen, a hydroxy group or a group OG', wherein G' represents an unsubstituted or a substituted ($C_1$-$C_4$) alkyl group.

3. A process according to claim 2 for the preparation of a compound of formula (I) wherein G represents hydrogen, a hydroxy group or a group OG' wherein G' represents a ($C_1$-$C_4$)alkyl group unsubstituted or substituted with a carbo($C_1$-$C_4$)alkoxy or carboxy group.

4. A process according to any one of claims 1 to 3 for the preparation of a compound of formula (I) wherein the chiral carbon atom in the ethanolamine chain has the absolute configuration (R).

5. A process for the preparation of a compound of following formula (IV)

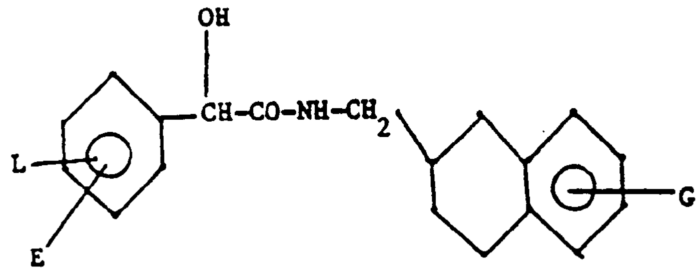

(IV)

wherein

- E represents hydrogen, a ($C_1$-$C_4$)alkyl group, a ($C_1$-$C_4$)alkoxy group, a phenyl group, a nitro group, a halogen atom or a trifluoromethyl group,
- L represents hydrogen, a ($C_1$-$C_4$)alkyl group, phenyl, ($C_1$-$C_4$) alkoxy, a nitro group, or a halogen atom, or
- E and L taken together represent a group -CH=CH-CH=CH- or $-CH_2-CH_2-CH_2-CH_2-$, and
- G represents a hydrogen atom, a chlorine atom, a hydroxy group or an OG' group wherein G' represents a ($C_1$-$C_4$)alkyl group either unsubstituted or substituted with a hydroxy, ($C_1$-$C_4$)alkoxy, ($C_1$-$C_4$)alkoxycarbonyl, carboxy, or ($C_3$-$C_7$)cycloalkyl group;

a ($C_3$-$C_7$)cycloalkyl group; or a ($C_2$-$C_4$)alkanoyl group, or one of its salts, characterized in that it comprises reacting a compound of formula

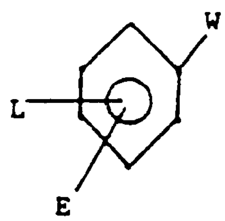

(II)

wherein E and L are as defined above and W represents a group

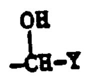

(d)

wherein Y is a -COOH group or a functional derivative thereof, with a compound of formula (III)

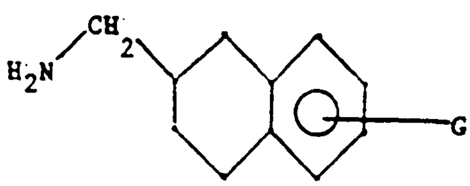

(III)

wherein G is as defined above.

6. A process according to claim 5 for the preparation of a compound of formula (IV) wherein G is a hydrogen atom, a hydroxy group, or an OG' group wherein G' is an unsubstituted ($C_1$-$C_4$) alkyl group or ($C_3$-$C_7$)cycloalkyl.

7. A process for the preparation of a compound of formula (III)

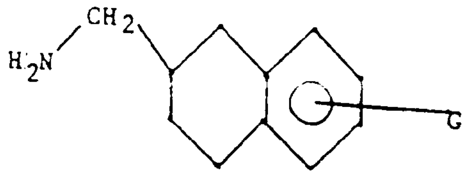

(III)

wherein G is hydrogen, a chlorine atom, a hydroxy group, or an OG' group wherein G' represents a $(C_1-C_4)$alkyl group either unsubstituted or substituted with a hydroxy, $(C_1-C_4)$alkoxy, $(C_1-C_4)$alkoxycarbonyl, carboxy, or $(C_3-C_7)$cycloalkyl group; a $(C_3-C_7)$cycloalkyl group; or a $(C_2-C_4)$alkanoyl group, but different from an $-OCH_3$ group
at the 7- or 8- position, or of one of its salts-, characterized in that it comprises reducing a compound of formula selected from

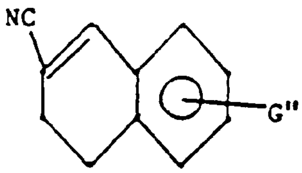

(VIII)

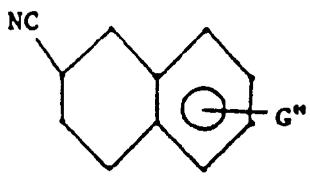

(VIII')

and

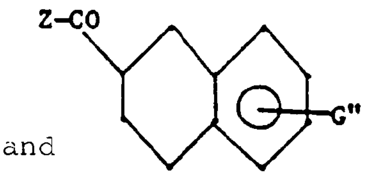

(X)

wherein G" represents hydrogen, a chlorine atom, a hydroxy group or a methoxy group and Z is an $-NH_2$ group, thus obtaining a compound of formula (III) wherein G = G", and submitting the compound of formula (III) wherein G" is hydroxy to conventional alkylation or acylation procedures with or without prior protection of the amino group, said process being further characterized in that when a compound of formula (III) is obtained wherein G = G" is a methoxy group, said compound may be converted into the corresponding compound wherein G = G" is a hydroxy group by demethylation with hydrobromic acid, it being possible for the compound obtained, when it is obtained as a mixture of isomers, to be separated into the pure isomers according to conventional methods.

8. A process according to claim 7 for the preparation of a compound of formula (III) wherein G is a hydroxy group or an OG' group wherein G' represents a $(C_1-C_4)$ alkyl group substituted with a group carboxy or $(C_1-C_4)$alkoxycarbonyl.

9. A process according to claim 7 for the preparation of a compound of formula (III)

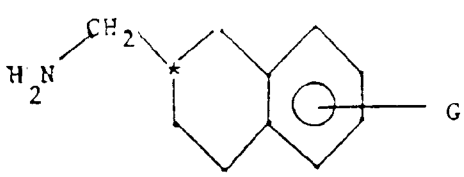

(III)

wherein G is hydrogen, achlorine atom, a hydroxy group, or an OG' group wherein G' represents a $(C_1-C_4)$alkyl group either unsubstituted or substituted with a hydroxy, $(C_1-C_4)$alkoxy, $(C_1-C_4)$alkoxycarbonyl, carboxy, or $(C_3-C_7)$cycloalkyl group; a $(C_3-C_7)$cycloalkyl group; or a $(C_2-C_4)$alkanoyl group, in the form of pure stereoisomer, or one of its salts.

10. A process for the preparation of a compound of formula (X)

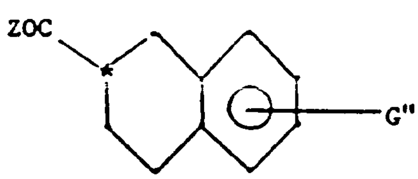

(X)

wherein Z is a group -OH or $-NH_2$ and G" represents hydrogen, a chlorine atom, a hydroxy group, or a methoxy group, provided that when Z is an -OH group, G" is different from hydrogen, in optically active form, or one of its salts, characterized in that it comprises treating a compound of formula (V)

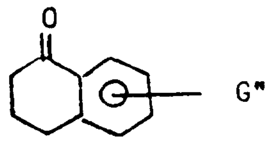

(V)

wherein G" is as defined above with diethylcarbonate in the presence of sodium, in order to obtain a compound of formula (XI)

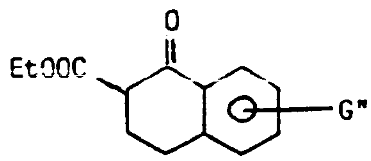

(XI)

wherein G" is as defined above, said compound being then submitted to a catalytic or chemical reduction, then hydrolyzed in a basic medium to obtain a compound of formula (X) above, wherein Z = OH and G" is as defined above, said compound being optionally converted into a corresponding amide by conventional methods to obtain a compound of formula (X) wherein Z = $NH_2$, and optionally the thus obtained compound of formula (X) is converted into one of its salts according to conventional methods.

**11.** A process for the preparation of a pharmaceutical composition, characterized in that it comprises mixing a compound prepared according to the process of any one of claims 1 to 4 with a pharmaceutically acceptable vehicle, as the active principle.